(19) Europäisches Patentamt
European Patent Office
Office européen des brevets

(11) **EP 4 707 393 A1**

(12) **EUROPEAN PATENT APPLICATION**
published in accordance with Art. 153(4) EPC

(43) Date of publication:
**11.03.2026 Bulletin 2026/11**

(21) Application number: **24810467.1**

(22) Date of filing: **23.05.2024**

(51) International Patent Classification (IPC):
***C12N 15/113** (2010.01)* ***A61K 31/713** (2006.01)*
***A61P 3/06** (2006.01)* ***A61P 9/00** (2006.01)*
***A61P 9/10** (2006.01)*

(52) Cooperative Patent Classification (CPC):
**A61K 31/713; A61P 3/06; A61P 9/00; A61P 9/10; C12N 15/113**

(86) International application number:
**PCT/CN2024/094986**

(87) International publication number:
**WO 2024/240230 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**24.05.2023 CN 202310598735**
**05.09.2023 CN 202311140396**
**27.11.2023 CN 202311601195**
**25.01.2024 CN 202410112975**
**25.04.2024 CN 202410505495**
**17.05.2024 CN 202410621293**

(71) Applicant: **CMS Research & Development Pte. Ltd.**
**Singapore 179803 (SG)**

(72) Inventors:
- **HU, Yanbin**
  **Shanghai 200131 (CN)**
- **HE, Huijun**
  **Shanghai 200131 (CN)**
- **LU, Jianyu**
  **Shanghai 200131 (CN)**
- **HE, Haiying**
  **Shanghai 200131 (CN)**
- **CHEN, Shuhui**
  **Shanghai 200131 (CN)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

# (54) DOUBLE-STRANDED SIRNA, CONJUGATE THEREOF AND USE THEREOF

(57) The present invention discloses a series of double-stranded siRNAs for inhibiting LPA expression, a conjugate thereof and the use thereof.

EP 4 707 393 A1

## Description

### REFERENCES TO RELATED APPLICATIONS

This application claims priority to and benefits of the Chinese patent application No. 202310598735.8 filed with the CNIPA on May 24, 2023, the Chinese patent application No. 202311140396.5 filed with the CNIPA on September 5, 2023, the Chinese patent application No. 202311601195.0 filed with the CNIPA on November 27, 2023, the Chinese patent application No. 202410112975.7 filed with the CNIPA on January 25, 2024, the Chinese patent application No. 202410505495.7 filed with the CNIPA on April 25, 2024, and the Chinese patent application No. 202410621293.9 filed with the CNIPA on May 17, 2024, the entire contents of which are incorporated herein by reference.

### TECHNICAL FIELD

The present invention relates to the field of medicine, and specifically to a series of double-stranded siRNAs inhibiting LPA expression, conjugates thereof, and uses thereof.

### BACKGROUND

Lp(a) is a lipoprotein particle synthesized in the liver, composed of low-density lipoprotein (LDL)-like particles and Apolipoprotein A [Apo(a), also called LPA], both of which are covalently linked by disulfide bonds. Apo(a) is encoded by the highly polymorphic LPA gene, in which a variable number of KIV type 2 repeats results in Apo(a) subtypes of various sizes. The LPA gene evolves from the plasminogen gene (PLG), and the sequences of both genes are highly homologous (see Konrad Schmidt et al., J. Lipid Res 2016 (57), 1339-1359). Plasma Lp(a) levels vary nearly 1000-fold among individuals, and approximately 20-30% of the population have highly elevated Lp(a) levels (≥ 50 mg/dL). See Hopewell et al., J. Intern. Med., (2013) 273 (1): 260-8; Wilson et al., J Clin Lipidol., (2019) 13 (3): 374-92 for related literature. High Lp(a) levels and apo(a) subtypes with low copy number of KIV type 2 are associated with an increased risk of cardiovascular disease. High Lp(a) is an independent high-risk factor for atherosclerotic cardiovascular disease (ASCVD), including coronary heart disease, myocardial infarction, stroke, peripheral artery disease, calcific aortic valve disease, and atherosclerosis, etc.

In RNA interference technology, small interfering RNA (siRNA) binds to an RNA-induced silencing complex (RISC), where the sense strand is displaced and the antisense strand, with the RISC, binds to the complementary RNA (target RNA). Upon binding, the target RNA is cleaved by the RNA endonuclease Argonaute (AGO) within the RISC and further degraded by an RNA exonuclease. RNAi has now been used to develop a novel class of therapeutic agents for the treatment of a disease caused by genetic abnormalities or abnormal gene expression. High Lp(a) level is an independent high-risk factor for atherosclerotic cardiovascular disease (ASCVD), creating an urgent need for Lp(a) inhibitors to improve the current situation of the high incidence of some cardiovascular diseases. RNA-targeted therapy has high selectivity and affinity for target mRNA. It has been successfully applied in several disease areasso far and represents a highly promising intervention for reducing Lp(a). siRNAs targeting liver LPA can effectively reduce the level of Lp(a) by silencing the expression of Apo(a) gene and blocking the synthesis of Apo(a) protein.

### SUMMARY OF THE INVENTION

The present disclosure provides a double-stranded siRNA for inhibiting LPA expression or a pharmaceutically acceptable salt thereof, comprising a sense strand and an antisense strand capable of forming a double-stranded region, wherein the sense strand comprises any one of the sequences as set forth in SEQ ID NOs: 1-18, and the antisense strand comprises any one of the sequences as set forth in SEQ ID NOs: 19-26, wherein each nucleotide on the sense strand and the antisense strand is an optionally modified nucleotide.

In some embodiments of the disclosure, at least one nucleotide on the sense strand and the antisense strand is a modified nucleotide.

In some embodiments of the disclosure, 0, 1, 2, 3 or 4 nucleotides on the sense strand and the antisense strand are unmodified nucleotides, and the remaining nucleotides are modified nucleotides.

The present disclosure provides a double-stranded siRNA or a pharmaceutically acceptable salt thereof, comprising a sense strand and an antisense strand capable of forming a double-stranded region, wherein the antisense strand comprises any one of the antisense strand sequences shown in Table 1, and each nucleotide on the antisense strand is an optionally modified nucleotide.

In some embodiments of the disclosure, provided is the double-stranded siRNA or the pharmaceutically acceptable salt thereof described above, wherein the sense strand comprises any one of the sense strand sequences shown in Table 1, and each nucleotide on the sense strand is an optionally modified nucleotide.

In some embodiments of the disclosure, the double-stranded siRNA comprises any one of the duplexes shown in

Table 1, wherein each nucleotide in the duplexes is an optionally modified nucleotide.

In some embodiments of the disclosure, the modified nucleotide is selected from the group consisting of a 2'-OMe-modified nucleotide, a 2'-F-modified nucleotide, and a 2'-deoxynucleotide.

In some embodiments of the disclosure, the 5' end of the antisense strand optionally comprises one nucleotide selected from the group consisting of mDVPU, B29, B30, B33, B34 and B36, wherein,

the mDVPU is

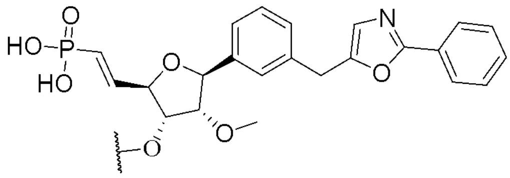

;

the B29 is

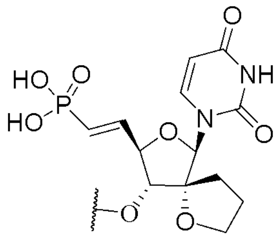

;

the B30 is

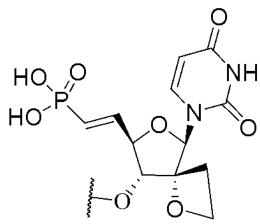

;

the B33 is

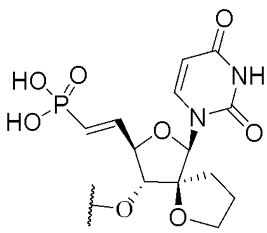

;

the B34 is

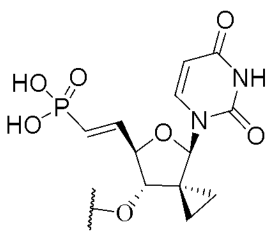

;

and

the B36 is

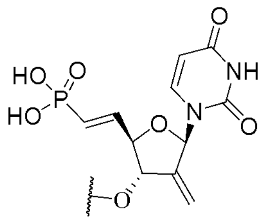

.

In some embodiments of the disclosure, the 5' end of the sense strand optionally comprises one nucleotide selected from invAb, wherein the invAb is

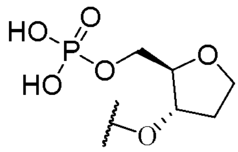

.

In some embodiments of the disclosure, each internucleoside linking group in the double-stranded region of the double-stranded siRNA is independently a phosphodiester internucleoside linking group or a phosphorothioate internucleoside linking group.

The present disclosure provides a double-stranded siRNA or a pharmaceutically acceptable salt thereof, comprising a sense strand and an antisense strand capable of forming a double-stranded region, wherein each nucleotide in the double-stranded siRNA is independently a modified or unmodified nucleotide, and the antisense strand comprises any one of the antisense strand sequences shown in Table 1.

In some embodiments of the disclosure, provided is the double-stranded siRNA or the pharmaceutically acceptable salt thereof described above, wherein the sense strand comprises any one of the sense strand sequences shown in Table 1.

In some embodiments of the disclosure, the double-stranded siRNA comprises any one of the duplexes shown in Table 1, each nucleotide in the duplexes is an optionally modified nucleotide.

In some embodiments of the disclosure, the double-stranded siRNA is as shown in Table 1, for example, U-S1 represents the double-stranded structure composed of SEQ ID NO:1 and SEQ ID NO:19.

**Table 1: Double-Stranded siRNA**

| No. | SEQ ID NO: | Sense strand sequence (5' →3') | SEQ ID NO: | Antisense strand sequence (5'→3') |
|---|---|---|---|---|
| U-S1 | 1 | C, C, C, U, U, A, U, U, G, U, U, A, U, A, C, G | 19 | A, U, C, C, C, U, C, G, U, A, Z, A, A, C, A, A, U, A, A, G, G, G, G, C, U, G, U, U |
| U-S2 | 2 | C, C, C, C, U, U, A, U, U, G, U, U, A, U, A, C | 19 | A, U, C, C, C, U, C, G, U, A, Z, A, A, C, A, A, U, A, A, G, G, G, G, C, U, G, U, U |
| U-S3 | 3 | G, C, C, C, C, U, U, A, U, U, G, U, U, A, U, A | 19 | A, U, C, C, C, U, C, G, U, A, Z, A, A, C, A, A, U, A, A, G, G, G, G, C, U, G, U, U |
| U-S4 | 4 | A, G, C, C, C, C, U, U, A, U, U, G, U, U, A, U | 19 | A, U, C, C, C, U, C, G, U, A, Z, A, A, C, A, A, U, A, A, G, G, G, G, C, U, G, U, U |

(continued)

| No. | SEQ ID NO: | Sense strand sequence (5' →3') | SEQ ID NO: | Antisense strand sequence (5'→3') |
|---|---|---|---|---|
| U-S5 | 5 | C, A, G, C, C, C, C, U, U, A, U, U, G, U, U, A | 19 | A, U, C, C, C, U, C, G, U, A, Z, A, A, C, A, A, U, A, A, G, G, G, G, C, U, G, U, U |
| U-S6 | 1 | C, C, C, U, U, A, U, U, G, U, U, A, U, A, C, G | 20 | U, C, G, U, A, Z, A, A, C, A, A, U, A, A, G, G, G, G, C, U, G, U, U |
| U-S7 | 1 | C, C, C, U, U, A, U, U, G, U, U, A, U, A, C, G | 21 | A, U, C, C, C, U, C, G, U, A, Z, A, A, C, A, A, U, A, A, G, G, G, G, C, U, G, C, C |
| U-S8 | 1 | C, C, C, U, U, A, U, U, G, U, U, A, U, A, C, G | 21 | A, U, C, C, C, U, C, G, U, A, Z, A, A, C, A, A, U, A, A, G, G, G, G, C, U, G |
| U-S9 | 6 | G, C, C, C, C, U, U, A, U, U, G, U, U, A, U, A, C, G | 19 | A, U, C, C, C, U, C, G, U, A, Z, A, A, C, A, A, U, A, A, G, G, G, G, C, U, G, U, U |
| US10 | 7 | R, C, C, C, C, U, U, A, U, U, G, U, U, A, U, A, C, R | 19 | A, U, C, C, C, U, C, G, U, A, Z, A, A, C, A, A, U, A, A, G, G, G, G, C, U, G, U, U |
| US11 | 7 | R, C, C, C, C, U, U, A, U, U, G, U, U, A, U, A, C, R | 21 | A, U, C, C, C, U, C, G, U, A, Z, A, A, C, A, A, U, A, A, G, G, G, G, C, U, G |
| US12 | 8 | G, C, C, C, C, U, U, A, U, U, R, U, U, A, U, A, C, G | 21 | A, U, C, C, C, U, C, G, U, A, Z, A, A, C, A, A, U, A, A, G, G, G, G, C, U, G |
| US13 | 9 | R, C, C, C, C, U, U, A, U, U, R, U, U, A, U, A, C, G | 21 | A, U, C, C, C, U, C, G, U, A, Z, A, A, C, A, A, U, A, A, G, G, G, G, C, U, G |
| US14 | 10 | R, C, C, C, C, U, U, A, U, U, G, U, U, A, U, A, C, R, A | 22 | U, C, U, C, G, U, A, Z, A, A, C, A, A, U, A, A, G, G, G, G, C, U, G, U, U |
| US15 | 11 | R, C, C, C, C, U, U, A, U, U, G, U, U, A, U, A, C, G, R | 22 | U, C, U, C, G, U, A, Z, A, A, C, A, A, U, A, A, G, G, G, G, C, U, G, U, U |
| US16 | 12 | R, C, C, C, C, U, U, A, U, U, G, U, U, A, U, A, C, G, A, G, R | 22 | U, C, U, C, G, U, A, Z, A, A, C, A, A, U, A, A, G, G, G, G, C, U, G, U, U |

(continued)

| No. | SEQ ID NO: | Sense strand sequence (5' →3') | SEQ ID NO: | Antisense strand sequence (5'→3') |
|---|---|---|---|---|
| US17 | 13 | R, C, C, C, C, U, U, A, U, U, G, U, U, A, U, A, C, G, R, G, A | 22 | U, C, U, C, G, U, A, Z, A, A, C, A, A, U, A, A, G, G, G, G, C, U, G, U, U |
| US18 | 14 | C, C, C, U, U, A, U, U, G, U, U, A, R, A, C, G, A | 23 | U, C, U, C, G, U, A, U, R, A, C, A, A, U, A, A, G, G, G, G, C, U, U |
| US19 | 15 | C, A, G, C, C, C, C, U, U, A, U, U, G, U, U, A, R, A, C, G, A | 24 | U, C, U, C, G, U, A, U, R, A, C, A, A, U, A, A, G, G, G, G, C, U, G |
| US20 | 16 | U, G, C, C, A, A, G, C, U, U, G, G, U, C, A, U, C, U, A | 25 | U, A, G, A, U, R, A, C, C, A, A, G, C, U, U, G, G, C, A, R, G |
| US21 | 17 | G, C, C, A, A, G, C, U, U, G, G, U, C, A, U, C, U, A | 25 | U, A, G, A, U, R, A, C, C, A, A, G, C, U, U, G, G, C, A, R, G |
| US22 | 18 | U, U, G, C, C, A, A, G, C, U, U, G, G, U, C, A, U, C, U, A | 26 | U, A, G, A, U, R, A, C, C, A, A, G, C, U, U, G, G, C, A, R, G, G |

The Z described herein is

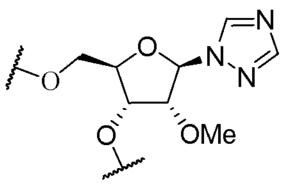

.

In some embodiments of the disclosure, the structure of the intermediate for synthesizing the Z is as shown in Formula (Z-M):

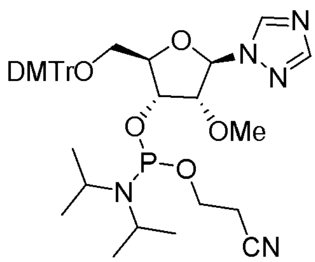

**Z-M**

.

The R described herein is

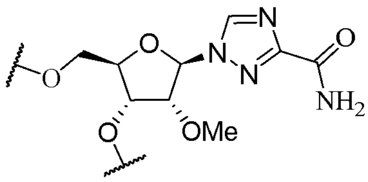

.

The dR described herein is

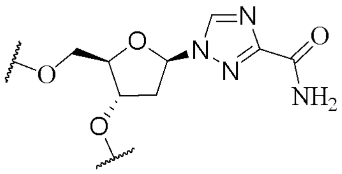

.

In some embodiments of the disclosure, the structure of the intermediate for synthesizing the dR is as shown in Formula (dR-M):

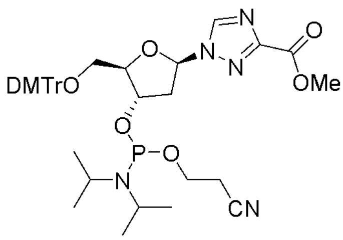

**dR-M** .

In some embodiments of the disclosure, the sense strand of the double-stranded siRNA comprises 0, 1, 2, 3, 4, 5 or 6 unmodified nucleotides.

In some embodiments of the disclosure, the antisense strand of the double-stranded siRNA comprises 0, 1, 2, 3, 4, 5 or 6 unmodified nucleotides.

In some embodiments of the disclosure, the sense strand of the double-stranded siRNA comprises 0, 1 or 2 nucleotide(s) with nucleoside replaced by Z.

In some embodiments of the disclosure, the antisense strand of the double-stranded siRNA comprises 0, 1 or 2 nucleotide(s) with nucleoside replaced by Z.

In some embodiments of the disclosure, the sense strand of the double-stranded siRNA comprises 0, 1, 2 or 3 nucleotide(s) with nucleoside replaced by R.

In some embodiments of the disclosure, the antisense strand of the double-stranded siRNA comprises 0, 1, 2 or 3 nucleotide(s) with nucleoside replaced by R.

In some embodiments of the disclosure, the double-stranded siRNA comprises a double-stranded structure shown in Table 2, for example, S1 represents the double-stranded structure composed of SEQ ID NO:27 and SEQ ID NO:56.

Table 2: Modified Double-Stranded siRNA

| No. | SEQ ID NO: | Sense strand sequence (5'→3') | SEQ ID NO: | Antisense strand sequence (5'→3') |
|---|---|---|---|---|
| S1 | 27 | mCs, mCs, mC, mU, fU, fA, fU, mU, mG, mU, mU, mA, mU, mA, mC, mG | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S2 | 28 | mCs, mCs, mC, mC, mU, fU, fA, fU, mU, mG, mU, mU, mA, mU, mA, mC | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S3 | 29 | mGs, mCs, mC, mC, mC, mU, fU, fA, fU, mU, mG, mU, mU, mA, mU, mA | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S4 | 30 | mAs, mGs, mC, mC, mC, mC, mU, fU, fA, fU, mU, mG, mU, mU, mA, mU | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S5 | 31 | mCs, mAs, mG, mC, mC, mC, mC, mU, fU, fA, fU, mU, mG, mU, mU, mA | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S6 | 27 | mCs, mCs, mC, mU, fU, fA, fU, mU, mG, mU, mU, mA, mU, mA, mC, mG | 57 | mVPUs, fCs, mG, fU, mA, Z, mA, mA, mC, mA, mA, fU, mA, fA, mG, fG, mG, fG, mC, fU, mGs, mUs, mU |
| S7 | 27 | mCs, mCs, mC, mU, fU, fA, fU, mU, mG, mU, mU, mA, mU, mA, mC, mG | 58 | mUs, fCs, mG, fU, mA, Z, mA, mA, mC, mA, mA, fU, mA, fA, mG, fG, mG, fG, mC, fU, mGs, mUs, mU |

(continued)

| No. | SEQ ID NO: | Sense strand sequence (5'→3') | SEQ ID NO: | Antisense strand sequence (5'→3') |
|---|---|---|---|---|
| S8 | 32 | mCs, mCs, mC, mU, mU, fA, fU, fU, mG, mU, mU, mA, mU, mA, mC, mG | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S9 | 33 | mCs, mCs, mC, mU, mU, mA, fU, fU, fG, mU, mU, mA, mU, mA, mC, mG | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S10 | 34 | mCs, mCs, mC, mU, mU, mA, mU, fU, fG, fU, mU, mA, mU, mA, mC, mG | 56 | mAs, mU, mC, C, C, mUs, fCs, mG, fU, mA, Z, mA, mA, mC, mA, mA, fU, mA, fA, mG, fG, mG, fG, mC, fU, mGs, mUs, mU |
| S11 | 27 | mCs, mCs, mC, mU, fU, fA, fU, mU, mG, mU, mU, mA, mU, mA, mC, mG | 59 | mAs, mU, mC, C, C, mUs, fCs, mG, fU, mA, Z, mA, mA, mC, mA, mA, fU, mA, fA, mG, fG, mG, fG, mC, fU, mGs, mCs, mC |
| S12 | 27 | mCs, mCs, mC, mU, fU, fA, fU, mU, mG, mU, mU, mA, mU, mA, mC, mG | 60 | mAs, mU, mC, C, C, mUs, fCs, mG, fU, mA, Z, mA, mA, mC, mA, mA, fU, mA, fA, mG, fG, mG, fG, mCs, fUs. mG |
| S13 | 27 | mCs, mCs, mC, mU, fU, fA, fU, mU, mG, mU, mU, mA, mU, mA, mC, mG | 61 | mAs, mU, mC, mC, C, mUs, fCs, mG, fU, mA, Z, mA, mA, mC, mA, mA, fU, mA, fA, mG, fG, mG, fG, mC, fU, mGs, mUs, mU |

(continued)

| No. | SEQ ID NO: | Sense strand sequence (5'→3') | SEQ ID NO: | Antisense strand sequence (5'→3') |
|---|---|---|---|---|
| S14 | 27 | mCs, mCs, mC, mU, fU, fA, fU, mU, mG, mU, mU, mA, mU, mA, mC, mG | 62 | mAs, mUs, mC, mC, C, mUs, fCs, mG, fU, mA, Z, mA, mA, mC, mA, mA, fU, mA, fA, mG, fG, mG, fG, mC, fU, mGs, mUs, mU |
| S15 | 27 | mCs, mCs, mC, mU, fU, fA, fU, mU, mG, mU, mU, mA, mU, mA, mC, mG | 63 | mAs, mU, mC, mC, C, mUs, fCs, mG, fU, mA, Z, mA, mA, mC, mA, mA, fU, mA, fA, mG, fG, mG, fG, mCs, fUs, mG |
| S16 | 35 | (invAb)s, mCs, mC, mC, mU, fU, fA, fU, mU, mG, mU, mU, mA, mU, mA, mC, mG | 61 | mAs, mU, mC, mC, C, mUs, fCs, mG, fU, mA, Z, mA, mA, mC, mA, mA, fU, mA, fA, mG, fG, mG, fG, mC, fU, mGs, mUs, mU |
| S17 | 36 | (invAb)s, Rs, mC, mC, mC, mC, mU, fU, fA, fU, mU, mG, mU, mU, mA, mU, mA, mC, R | 63 | mAs, mU, mC, mC, C, mUs, fCs, mG, fU, mA, Z, mA, mA, mC, mA, mA, fU, mA, fA, mG, fG, mG, fG, mCs, fUs, mG |
| S18 | 36 | (invAb)s, Rs, mC, mC, mC, mC, mU, fU, fA, fU, mU, mG, mU, mU, mA, mU, mA, mC, R | 61 | mAs, mU, mC, mC, C, mUs, fCs, mG, fU, mA, Z, mA, mA, mC, mA, mA, fU, mA, fA, mG, fG, mG, fG, mC, fU, mGs, mUs, mU |
| S19 | 37 | (invAb)s, Rs, mG, mC, mC, mC, mC, mU, fU, fA, fU, mU, mG, mU, mU, mA, mU, mA, mC, R | 61 | mAs, mU, mC, mC, C, mUs, fCs, mG, fU, mA, Z, mA, mA, mC, mA, mA, fU, mA, fA, mG, fG, mG, fG, mC, fU, mGs, mUs, mU |

(continued)

| No. | SEQ ID NO: | Sense strand sequence (5'→3') | SEQ ID NO: | Antisense strand sequence (5'→3') |
|---|---|---|---|---|
| S20 | 36 | (invAb)s, Rs, mC, mC, mC, mC, mU, fU, fA, fU, mU, mG, mU, mU, mA, mU, mA, mC, R | 64 | mU, C, mUs, fCs, mG, fU, mA, Z, mA, mA, mC, mA, mA, fU, mA, fA, mG, fG, mG, fG, mC, fU, mGs, mUs, mU |
| S21 | 38 | (invAb)s, Rs, mC, mC, mC, mC, mU, fU, fA, fU, mU, mG, mU, mU, mA, mU, mA, mC, R, mA | 64 | mU, C, mUs, fCs, mG, fU, mA, Z, mA, mA, mC, mA, mA, fU, mA, fA, mG, fG, mG, fG, mC, fU, mGs, mUs, mU |
| S22 | 39 | (invAb)s, Rs, mC, mC, mC, mC, mU, fU, fA, fU, mU, mG, mU, mU, mA, mU, mA, mC, mG, R | 64 | mU, C, mUs, fCs, mG, fU, mA, Z, mA, mA, mC, mA, mA, fU, mA, fA, mG, fG, mG, fG, mC, fU, mGs, mUs, mU |
| S23 | 40 | (invAb)s, Rs, mC, mC, mC, mC, mU, fU, fA, fU, mU, mG, mU, mU, mA, mU, mA, mC, mG, mA, mG, R | 64 | mU, C, mUs, fCs, mG, fU, mA, Z, mA, mA, mC, mA, mA, fU, mA, fA, mG, fG, mG, fG, mC, fU, mGs, mUs, mU |
| S24 | 41 | (invAb)s, Rs, mC, mC, mC, mC, mU, fU, fA, fU, mU, mG, mU, mU, mA, mU, mA, mC, mG, R, mG, mA | 64 | mU, C, mUs, fCs, mG, fU, mA, Z, mA, mA, mC, mA, mA, fU, mA, fA, mG, fG, mG, fG, mC, fU, mGs, mUs, mU |
| S25 | 42 | mCs, mCs, mC, mU, fU, fA, fU, mU, mG, mU, mU, mA, R, mA, mC, mG, mA | 65 | mU, C, mUs, fCs, mG, mU, mA, fU, R, mA, mC, mA, mA, mU, mA, fA, mG, fG, mG, mG, mCs, mUs, mU |
| S26 | 43 | mCs, mAs, mG, mC, mC, mC, mC, mU, fU, fA, fU, mU, mG, mU, mU, mA, R, mA, mC, mG, mA | 66 | mU, C, mUs, fCs, mG, fU, mA, fU, R, mA, mC, mA, mA, fU, mA, fA, mG, fG, mG, fG, mCs, fUs, mG |

(continued)

| No. | SEQ ID NO: | Sense strand sequence (5'→3') | SEQ ID NO: | Antisense strand sequence (5'→3') |
|---|---|---|---|---|
| S27 | 44 | mUs, mGs, mC, mC, mA, fA, mG, fC, fU, fU, mG, mG, mU, mC, mA, mU, mC, mU, mA | 67 | mUs, fAs, mG, mA, mU, R, fA, mC, mC, mA, mA, mG, fC, mU, fU, mG, mG, mC, mAs, Rs, mG |
| S28 | 45 | mGs, mCs, mC, fA, mA, fG, fC, fU, mU, mG, mG, mU, mC, mA, mU, mC, mU, mA | 68 | mUs, fAs, mG, mA, mU, R, fA, mC, mC, mA, mA, mG, mC, fU, mU, fG, mG, mC, mAs, Rs, mG |
| S29 | 46 | mUs, mU, mG, mC, mC, mA,mA, fG, fC, fU, fU, mG, mG, mU, mC, mA, mU, mC, mU, mA | 69 | mUs, fAs, fGs, fA, fU, R, fA, mC, mC, fA, mA, mG, mC, fU, mU, mG, mG, mC, mA, Rs, mGs, mG |
| S30 | 43 | mCs, mAs, mG, mC, mC, mC, mC, mU, fU, fA, fU, mU, mG, mU, mU, mA, R, mA, mC, mG, mA | 70 | mU, C, mUs, fCs, mG, fU, mA, fU, Z, mA, mC, mA, mA, fU, mA, fA, mG, fG, mG, fG, mCs, fUs, mG |
| S31 | 47 | mGs, mCs, mC, mC, mC, mU, fU, fA, fU, mU, mG, mU, mU, mA, R, mA, mC, mG, mA | 71 | mVPUs, fCs, mG, fU, mA, fU, R, mA, mC, mA, mA, fU, mA, fA, mG, fG, mG, fG, mCs, fUs, mG, U |
| S32 | 47 | mGs, mCs, mC, mC, mC, mU, fU, fA, fU, mU, mG, mU, mU, mA, R, mA, mC, mG, mA | 72 | mVPUs, fCs, mG, fU, mA, fU, Z, mA, mC, mA, mA, fU, mA, fA, mG, fG, mG, fG, mCs, fUs, mG, U |
| S33 | 48 | mCs, mAs, mG, mC, mC, mC, fC, mU, fU, fA, fU, mU, mG, mU, mU, mA, R, mA, mC, mG, mA | 73 | mVPUs, fCs, mG, mU, mA, fU, R, mA, mC, mA, mA, mU, mA, fA, mG, fG, mG, mG, mC, R, mGs, mUs, mU |
| S34 | 49 | mCs, mAs, mG, mC, mC, mC, mC, mU, fU, fA, fU, mU, mG, mU, mU, mA, mU, mA, mC, mG, mA | 74 | mVPUs, dCs, mG, mU, dA, mU, dR, mA, mC, mA, mA, dT, mA, fA, mG, fG, mG, mG, mC, R, mGs, mUs, mU |

(continued)

| No. | SEQ ID NO: | Sense strand sequence (5'→3') | SEQ ID NO: | Antisense strand sequence (5'→3') |
|---|---|---|---|---|
| S35 | 50 | mCs, mCs, mU, mG, mC, mC, fA, mA, fG, fC, fU, mU, mG, mG, mU, mC, mA, mU, mC, mU, mA | 75 | mVPUs, fAs, mG, mA, mU, fG, R, mC, mC, mA, mA, mG, mC, fU, mU, fG, mG, mC, R, mG, mGs, mUs, mU |
| S36 | 51 | mCs, mCs, mU, mG, mC, mC, mA, mA, fG, fC, fU, mU, mG, mG, mU, mC, mA, mU, mC, mU, mA | 76 | mVPUs, dAs, mG, mA, dT, mG, dR, mC, mC, mA, mA, dG, mC, fU, mU, fG, mG, mC, R, mG, mGs, mUs, mU |
| S37 | 49 | mCs, mAs, mG, mC, mC, mC, mC, mU, fU, fA, fU, mU, mG, mU, mU, mA, mU, mA, mC, mG, mA | 77 | mDVPUs, dCs, mG, mU, dA, mU, dR, mA, mC, mA, mA, dT, mA, fA, mG, fG, mG, mG, mC, R, mGs, mUs, mU |
| S39 | 52 | mCs, mCs, mU, mG, mC, mC, mA, mA, fG, fC, fU, mU, mG, mG, mU, mC, R, mU, mC, mU, mA | 78 | mU, C, mUs, fAs, mG, fA, mU, fG, R, mC, mC, mA, mA, fG, mC, fU, mU, fG, mG, fC, Rs, fGs, mG |
| S40 | 49 | mCs, mAs, mG, mC, mC, mC, mC, mU, fU, fA, fU, mU, mG, mU, mU, mA, mU, mA, mC, mG, mA | 79 | (B29)s, dCs, mG, mU, dA, mU, dR, mA, mC, mA, mA, dT, mA, fA, mG, fG, mG, mG, mC, R, mGs, mUs, mU |
| S41 | 49 | mCs, mAs, mG, mC, mC, mC, mC, mU, fU, fA, fU, mU, mG, mU, mU, mA, mU, mA, mC, mG, mA | 80 | (B33)s, dCs, mG, mU, dA, mU, dR, mA, mC, mA, mA, dT, mA, fA, mG, fG, mG, mG, mC, R, mGs, mUs, mU |
| S42 | 49 | mCs, mAs, mG, mC, mC, mC, mC, mU, fU, fA, fU, mU, mG, mU, mU, mA, mU, mA, mC, mG, mA | 81 | (B30)s, dCs, mG, mU, dA, mU, dR, mA, mC, mA, mA, dT, mA, fA, mG, fG, mG, mG, mC, R, mGs, mUs, mU |

(continued)

| No. | SEQ ID NO: | Sense strand sequence (5'→3') | SEQ ID NO: | Antisense strand sequence (5'→3') |
|---|---|---|---|---|
| S43 | 51 | mCs, mCs, mU, mG, mC, mC, mA, mA, fG, fC, fU, mU, mG, mG, mU, mC, mA, mU, mC, mU, mA | 82 | mDVPUs, dAs, mG, mA, dT, mG, dR mC, mC, mA, mA, dG, mC, fU, mU fG, mG, mC, R, mG, mGs, mUs, mU |
| S44 | 50 | mCs, mCs, mU, mG, mC, mC, fA, mA, fG, fC, fU, mU, mG, mG, mU, mC, mA, mU, mC, mU, mA | 82 | mDVPUs, dAs, mG, mA, dT, mG, dR mC, mC, mA, mA, dG, mC, fU, mU fG, mG, mC, R, mG, mGs, mUs, mU |
| S46 | 51 | mCs, mCs, mU, mG, mC, mC, mA, mA, fG, fC, fU, mU, mG, mG, mU, mC, mA, mU, mC, mU, mA | 83 | (B33)s, dAs, mG, mA, dT, mG, dR mC, mC, mA, mA, dG, mC, fU, mU fG, mG, mC, R, mG, mGs, mUs, mU |
| S47 | 51 | mCs, mCs, mU, mG, mC, mC, mA, mA, fG, fC, fU, mU, mG, mG, mU, mC, mA, mU, mC, mU, mA | 84 | (B34)s, dAs, mG, mA, dT, mG, dR mC, mC, mA, mA, dG, mC, fU, mU fG, mG, mC, R, mG, mGs, mUs, mU |
| S48 | 51 | mCs, mCs, mU, mG, mC, mC, mA, mA, fG, fC, fU, mU, mG, mG, mU, mC, mA, mU, mC, mU, mA | 85 | (B36)s, dAs, mG, mA, dT, mG, dR mC, mC, mA, mA, dG, mC, fU, mU fG, mG, mC, R, mG, mGs, mUs, mU |
| S49 | 51 | mCs, mCs, mU, mG, mC, mC, mA, mA, fG, fC, fU, mU, mG, mG, mU, mC, mA, mU, mC, mU, mA | 86 | mU, C, mUs, fAs, mG, fA, mU, R mA, mC, mC, mA, mA, fG, mC, fU mU, fG, mG, fC, mAs, fGs, mG |
| S50 | 49 | mCs, mAs, mG, mC, mC, mC, mC, mU, fU, fA, fU, mU, mG, mU, mU, mA, mU, mA, mC, mG, mA | 87 | mUs, dCs, mG, mU, dA, mU, dR, mA mC, mA, mA, dT, mA, fA, mG, fG mG, mG, mC, R, mGs, mUs, mU |

(continued)

| No. | SEQ ID NO: | Sense strand sequence (5'→3') | SEQ ID NO: | Antisense strand sequence (5'→3') |
|---|---|---|---|---|
| S51 | 53 | mCs, mUs, mU, mG, mC, mC, mA, mA, fG, fC, fU, mU, mG, mG, mU, mC, mA, mU, mC, mU, mA | 88 | mU, C, mUs, fAs, mG, fA, mU, fG, R mC, mC, mA, mA, fG, mC, fU, mU fG, mG, fC, mAs, fAs, mG |
| S52 | 49 | mCs, mAs, mG, mC, mC, mC, mC, mU, fU, fA, fU, mU, mG, mU, mU, mA, mU, mA, mC, mG, mA | 89 | (B33)s, dCs, mG, mU, dA, mU, mA mA, mC, mA, mA, dT, mA, fA, mG fG, mG, mG, mC, R, mGs, mUs, mU |
| S53 | 49 | mCs, mAs, mG, mC, mC, mC, mC, mU, fU, fA, fU, mU, mG, mU, mU, mA, mU, mA, mC, mG, mA | 90 | (B33)s, dCs, mG, mU, mA, mU, dR mA, mC, mA, mA, dT, mA, fA, mG fG, mG, mG, mC, R, mGs, mUs, mU |
| S54 | 49 | mCs, mAs, mG, mC, mC, mC, mC, mU, fU, fA, fU, mU, mG, mU, mU, mA, mU, mA, mC, mG, mA | 91 | (B33)s, mCs, mG, mU, dA, mU, dR mA, mC, mA, mA, dT, mA, fA, mG fG, mG, mG, mC, R, mGs, mUs, mU |
| S55 | 43 | mCs, mAs, mG, mC, mC, mC, mC, mU, fU, fA, fU, mU, mG, mU, mU, mA, R, mA, mC, mG, mA | 92 | mU, C, mUs, fCs, mG, fU, mA, fU mA, mA, mC, mA, mA, fU, mA, fA mG, fG, mG, fG, mCs, fUs, mG |
| S56 | 49 | mCs, mAs, mG, mC, mC, mC, mC, mU, fU, fA, fU, mU, mG, mU, mU, mA, mU, mA, mC, mG, mA | 93 | (B33)s, dCs, mGs, mU, dA, mU, dR, mA, mC, mA, mA, dT, mA, fA, mG, mG, mG, mG, mC, Rs, mGs, mUs, mU |
| S57 | 43 | mCs, mAs, mG, mC, mC, mC, mC, mU, fU, fA, fU, mU, mG, mU, mU, mA, R, mA, mC, mG, mA | 94 | mU, mC, C, mUs, fCs, mG, fU, mA, fU, R, mA, mC, mA, mA, fU, mA, fA, mG, fG, mG, fG, mCs, fUs, mG |

(continued)

| No. | SEQ ID NO: | Sense strand sequence (5'→3') | SEQ ID NO: | Antisense strand sequence (5'→3') |
|---|---|---|---|---|
| S58 | 43 | mCs, mAs, mG, mC, mC, mC, mC, mU, fU, fA, fU, mU, mG, mU, mU, mA, R, mA, mC, mG, mA | 95 | mAs, mU, mC, C, C, mUs, fCs, mG, fU, mA, fU, R, mA, mC, mA, mA, fU, mA, fA, mG, fG, mG, fG, mCs, fUs, mG |
| S59 | 43 | mCs, mAs, mG, mC, mC, mC, mC, mU, fU, fA, fU, mU, mG, mU, mU, mA, R, mA, mC, mG, mA | 96 | mAs, mU, mC, mC, C, mUs, fCs, mG, fU, mA, fU, R, mA, mC, mA, mA, fU, mA, fA, mG, fG, mG, fG, mCs, fUs, mG |
| S60 | 54 | mCs, mAs, mG, mC, mC, fC, fC, mU, fU, fA, fU, mU, mG, mU, mU, mA, mU, mA, mC, mG, mA | 97 | (B33)s, dCs, mGs, mU, dA, mU, dR, mA, mC, mA, mA, mU, mA, fA, mG, mG, mG, mG, mC, Rs, mGs, mUs, mU |
| S61 | 49 | mCs, mAs, mG, mC, mC, mC, mC, mU, fU, fA, fU, mU, mG, mU, mU, mA, mU, mA, mC, mG, mA | 94 | mU, mC, C, mUs, fCs, mG, fU, mA, fU, R, mA, mC, mA, mA, fU, mA, fA, mG, fG, mG, fG, mCs, fUs, mG |
| S62 | 49 | mCs, mAs, mG, mC, mC, mC, mC, mU, fU, fA, fU, mU, mG, mU, mU, mA, mU, mA, mC, mG, mA | 95 | mAs, mU, mC, C, C, mUs, fCs, mG, fU, mA, fU, R, mA, mC, mA, mA, fU, mA, fA, mG, fG, mG, fG, mCs, fUs, mG |
| S63 | 49 | mCs, mAs, mG, mC, mC, mC, mC, mU, fU, fA, fU, mU, mG, mU, mU, mA, mU, mA, mC, mG, mA | 96 | mAs, mU, mC, mC, C, mUs, fCs, mG, fU, mA, fU, R, mA, mC, mA, mA, fU, mA, fA, mG, fG, mG, fG, mCs, fUs, mG |
| S64 | 55 | mCs, Rs, mG, mC, mC, mC, mC, mU, fU, fA, fU, mU, mG, mU, mU, mA, mU, mA, mC, mG, mA | 95 | mAs, mU, mC, C, C, mUs, fCs, mG, fU, mA, fU, R, mA, mC, mA, mA, fU, mA, fA, mG, fG, mG, fG, mCs, fUs, mG |

In some embodiments of the disclosure, the double-stranded siRNA is S26, S41, S42, S49, S51, S55, S57, S58, S59, S61, S62, S63, or S64.

In the present disclosure, the mDVPU is

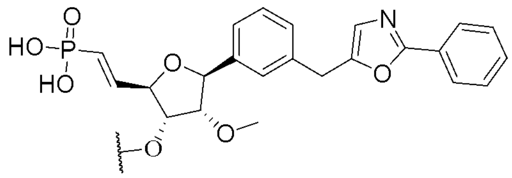

;

the B29 is

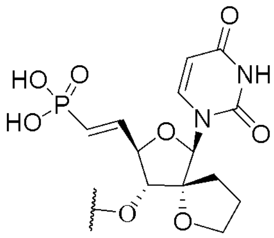

;

the B30 is

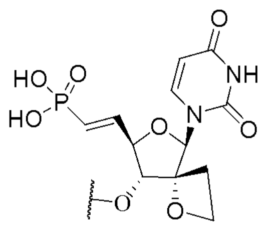

;

the B33 is

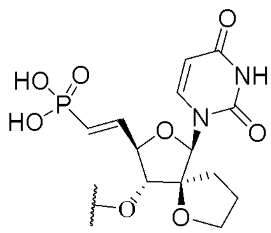

;

the B34 is

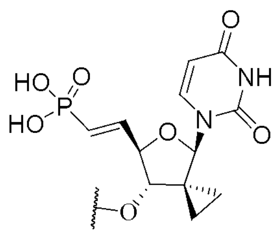

;

the B36 is

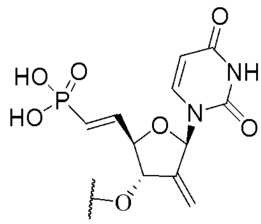
;

and the invAb is

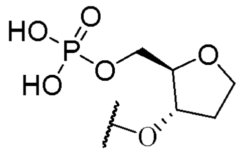
.

In some embodiments of the disclosure, the structures of the intermediates for synthesizing the mDVPU, B29, B30, B33, B34 and B36 are respectively as shown in Formulas mDVPU-M, B29-M, B30-M, B33-M, B34-M and B36-M:

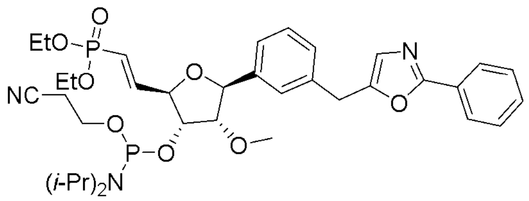
**mDVPU-M** ,

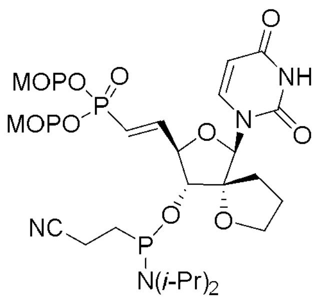
**B29-M**

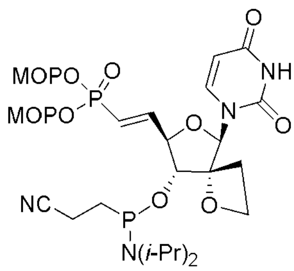
**B30-M** ,

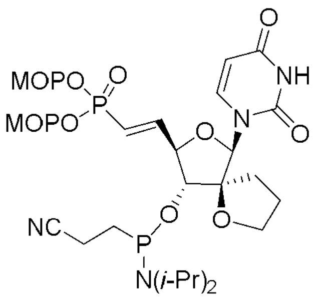
**B33-M** ,

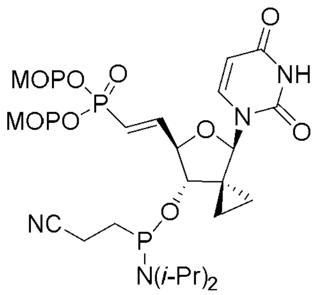
**B34-M** ,

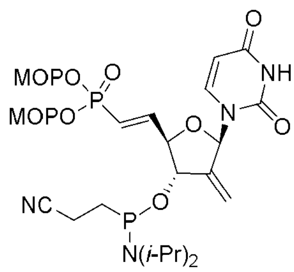
**B36-M** .

The present disclosure also provides a double-stranded siRNA conjugate for inhibiting LPA expression or a pharmaceutically acceptable salt thereof, wherein the double-stranded siRNA conjugate is formed by conjugating the double-stranded siRNA of any of the preceding embodiments to a ligand.

In some embodiments of the disclosure, provided is the double-stranded siRNA conjugate or the pharmaceutically acceptable salt thereof described above, wherein the double-stranded siRNA comprises a sense strand and an antisense strand capable of forming a double-stranded region, wherein the antisense strand comprises any one of the antisense strand sequences shown in Table 1, and each nucleotide on the antisense strand is an optionally modified nucleotide.

In some embodiments of the disclosure, provided is the double-stranded siRNA conjugate or the pharmaceutically acceptable salt thereof described above, wherein the sense strand comprises any one of the sense strand sequences shown in Table 1, and each nucleotide on the antisense strand is an optionally modified nucleotide.

In some embodiments of the disclosure, provided is the double-stranded siRNA conjugate or the pharmaceutically acceptable salt thereof described above, wherein each nucleotide on the duplexes is independently optionally a

modified nucleotide.

In some embodiments of the disclosure, provided is the double-stranded siRNA conjugate or the pharmaceutically acceptable salt thereof described above, wherein the modified nucleotide is selected from the group consisting of a 2'-OMe-modified nucleotide, a 2'-F-modified nucleotide, and a 2'-deoxynucleotide.

In some embodiments of the disclosure, provided is the double-stranded siRNA conjugate or the pharmaceutically acceptable salt thereof described above, wherein the 5' end of the antisense strand optionally comprises one nucleotide selected from the group consisting of mDVPU, B29, B30, B33, B34 and B36.

In some embodiments of the disclosure, provided is the double-stranded siRNA conjugate or the pharmaceutically acceptable salt thereof described above, wherein the 5' end of the sense strand optionally comprises one nucleotide selected from invAbs.

In some embodiments of the disclosure, provided is the double-stranded siRNA conjugate or the pharmaceutically acceptable salt thereof described above, wherein the double-stranded siRNA comprises any one of the duplexes shown in Table 2.

In some embodiments of the disclosure, provided is the double-stranded siRNA conjugate or the pharmaceutically acceptable salt thereof, wherein the ligand is conjugated at any position of the double-stranded siRNA.

In some embodiments of the disclosure, provided is the double-stranded siRNA conjugate or the pharmaceutically acceptable salt thereof described above, wherein the ligand is conjugated to the 3' end of the sense strand of the double-stranded siRNA.

In some embodiments of the disclosure, provided is the double-stranded siRNA conjugate or the pharmaceutically acceptable salt thereof described above, wherein the ligand is conjugated to the double-stranded siRNA via a phosphodiester linkage or a phosphorothioate linkage.

In some embodiments of the disclosure, provided is the double-stranded siRNA conjugate or the pharmaceutically acceptable salt thereof described above, wherein the ligand is one or more GalNAc derivatives attached by using bivalent, trivalent, or tetravalent branched linkages.

In some embodiments of the disclosure, provided is the double-stranded siRNA conjugate or the pharmaceutically acceptable salt thereof described above, wherein the ligand is selected from D01, D08, D10, D11, D18, D21 or L96, as follows.

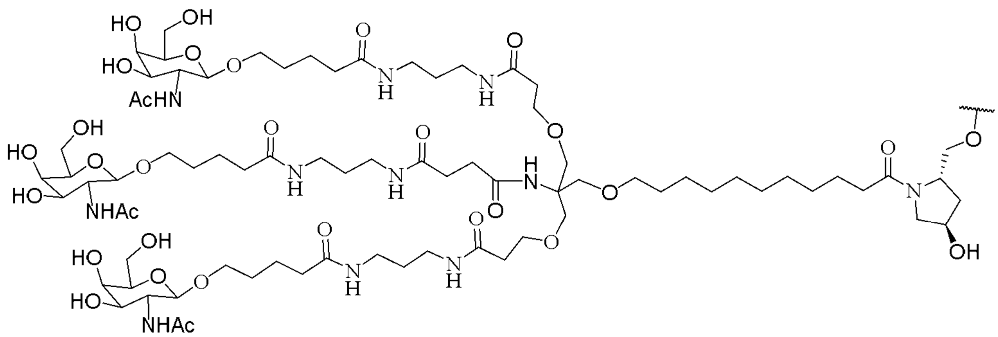

**D01**

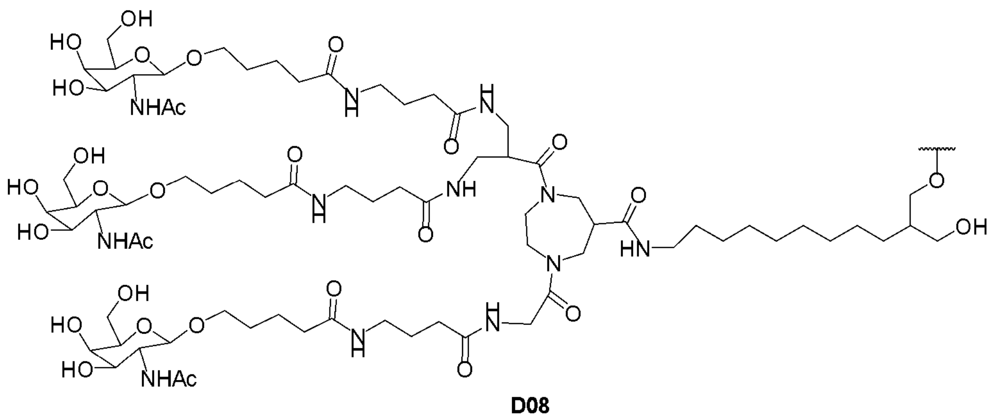

**D08**

,

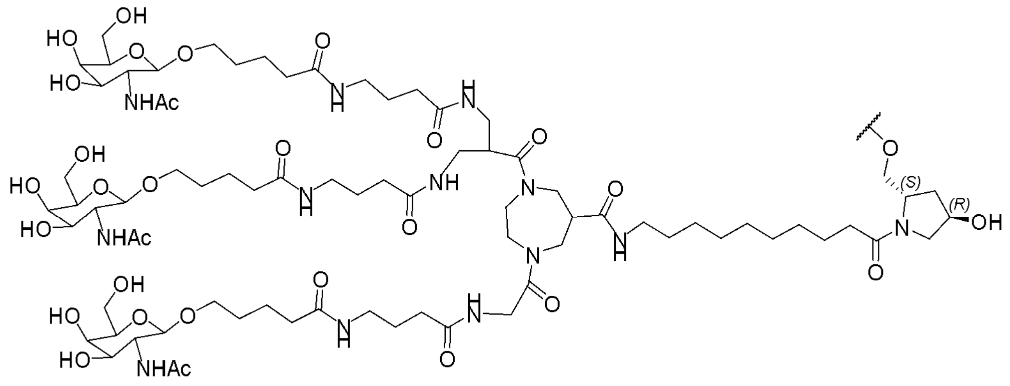

D10

,

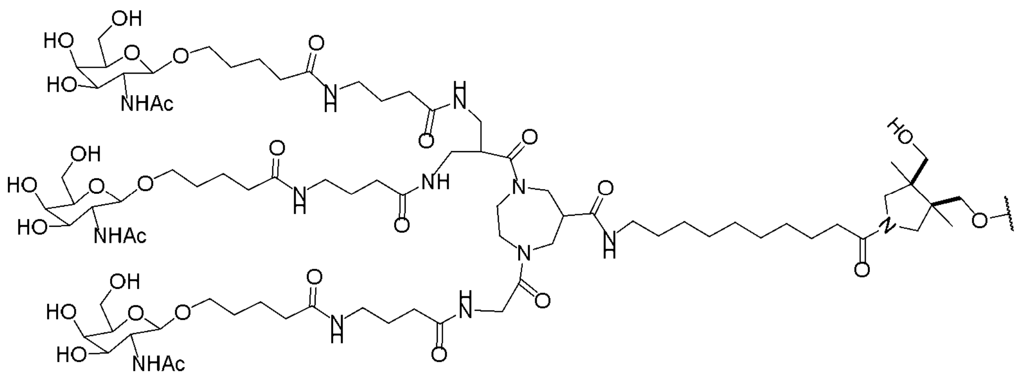

D11

,

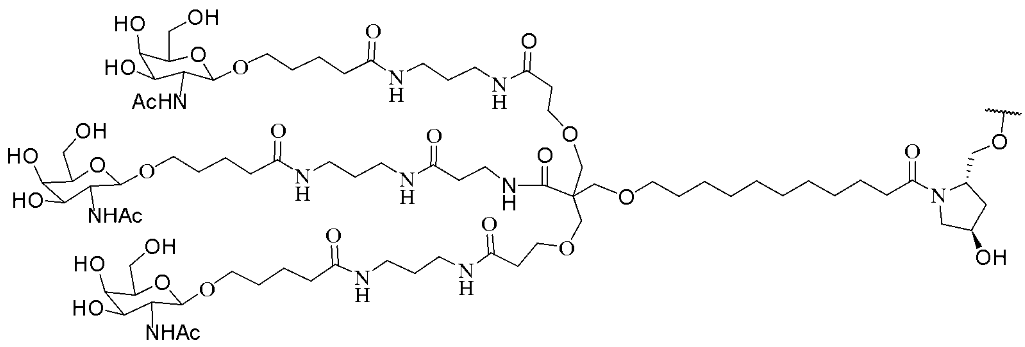

D18

,

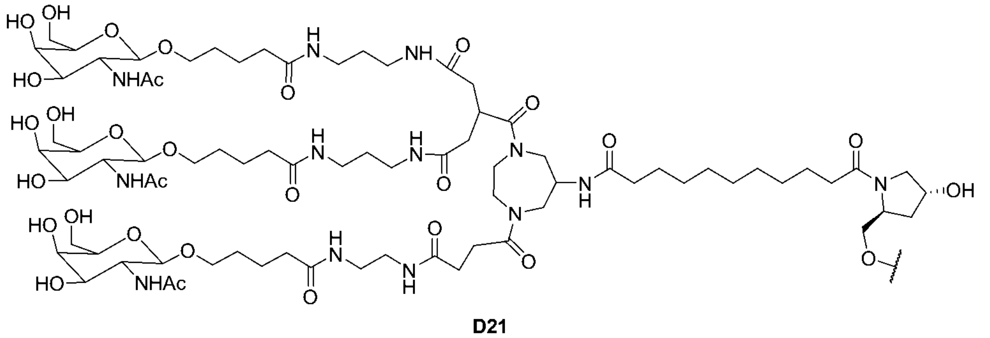

D21

,

and

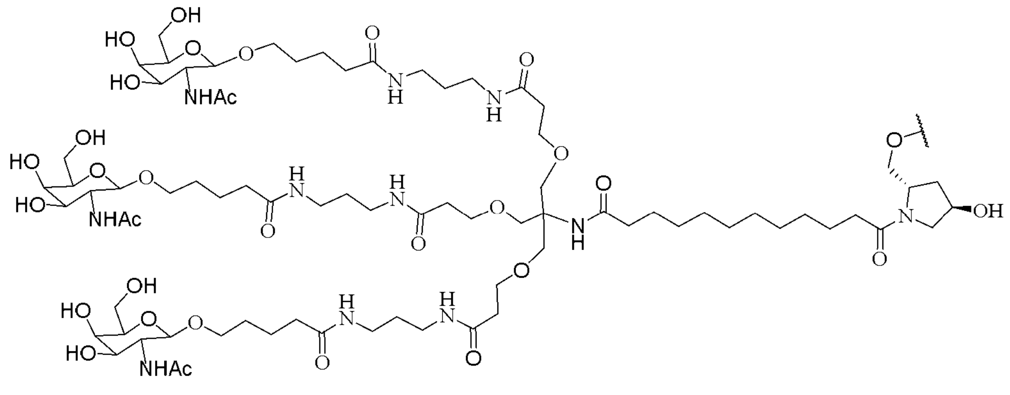

L96

.

In some embodiments of the disclosure, the structure of the intermediate for synthesizing the D08 is as shown in Formula (D08-M):

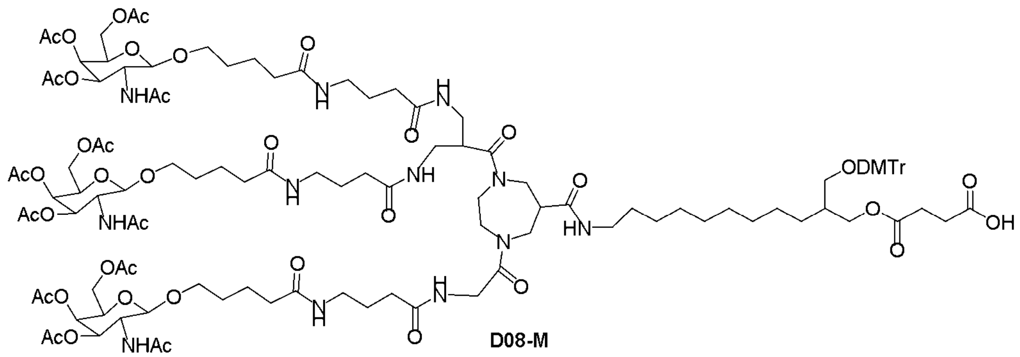

D08-M

.

In some embodiments of the disclosure, the structure of the intermediate for synthesizing the D10 is as shown in Formula (D10-M):

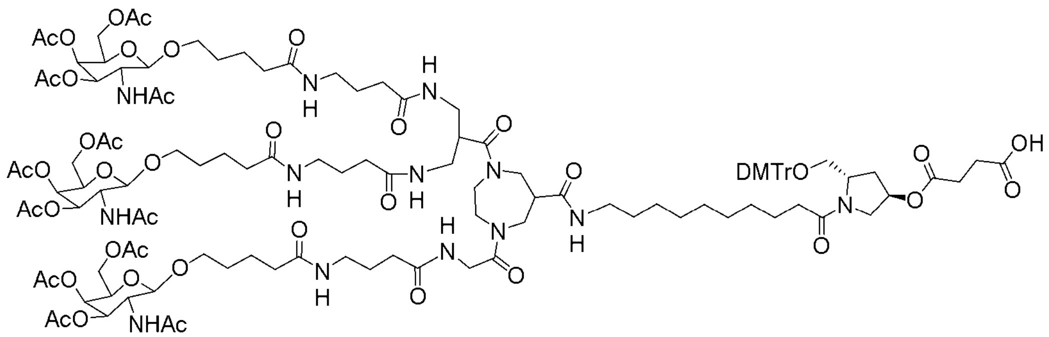

D10-M

.

In some embodiments of the disclosure, the structure of the intermediate for synthesizing the D11 is as shown in Formula (D11-M):

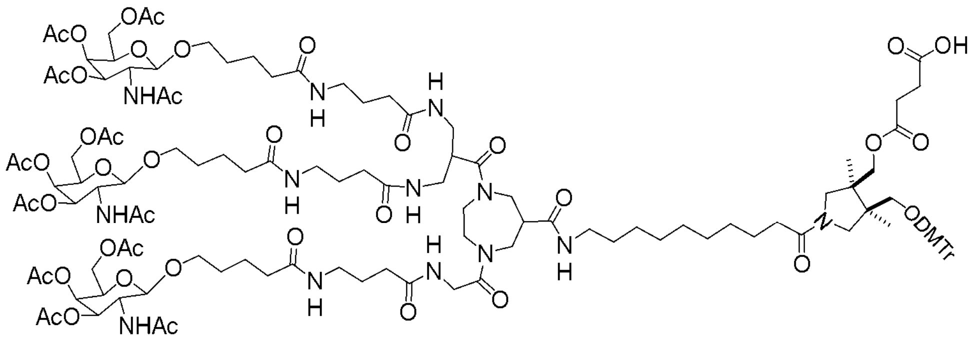

D11-M

.

In some embodiments of the disclosure, the structure of the intermediate for synthesizing the D18 is as shown in Formula (D18-M):

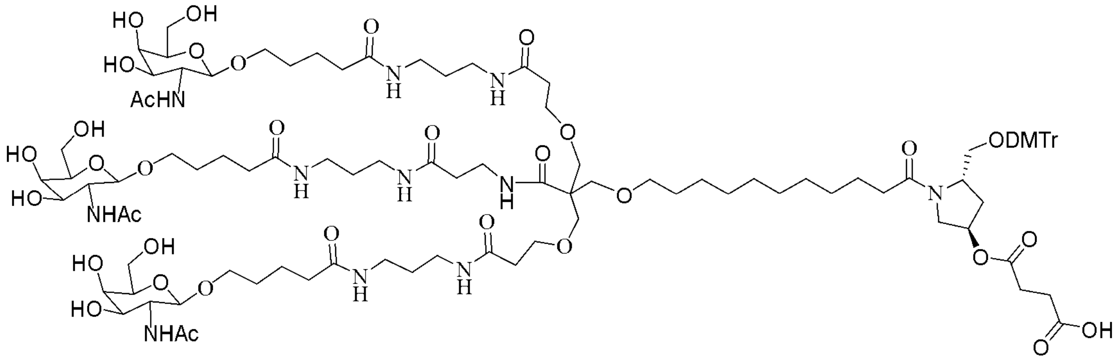

D18-M

.

In some embodiments of the disclosure, the structure of the intermediate for synthesizing the D21 is as shown in Formula (D21-M):

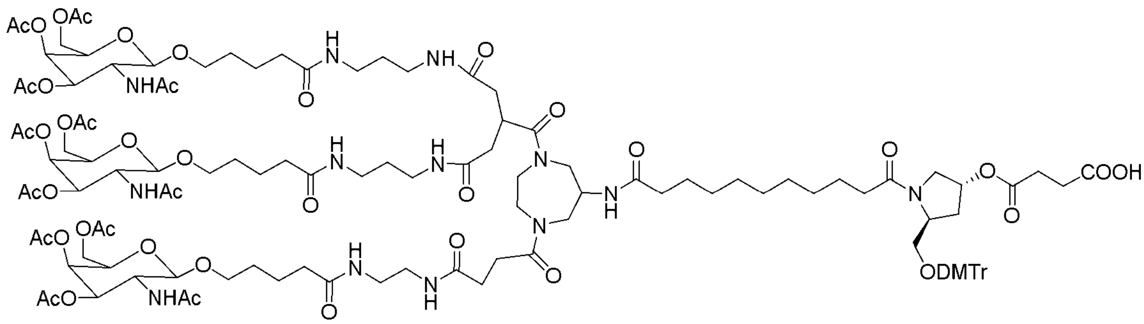

D21-M

.

Some embodiments of the present disclosure are from any combination of the embodiments as described above.
In the present disclosure, the ligation of the double-stranded siRNA conjugate is as follows:

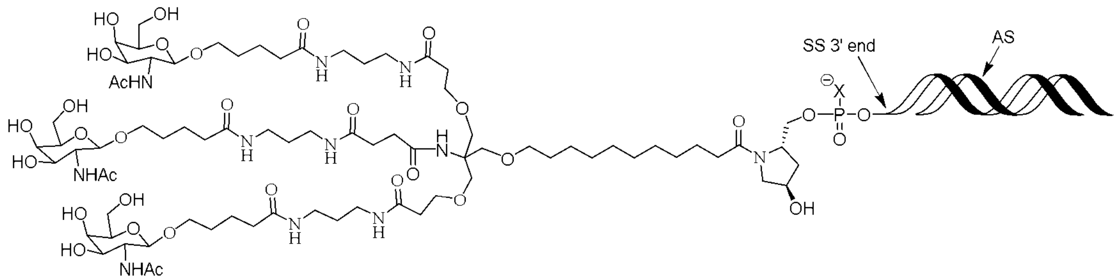

(Formula I),

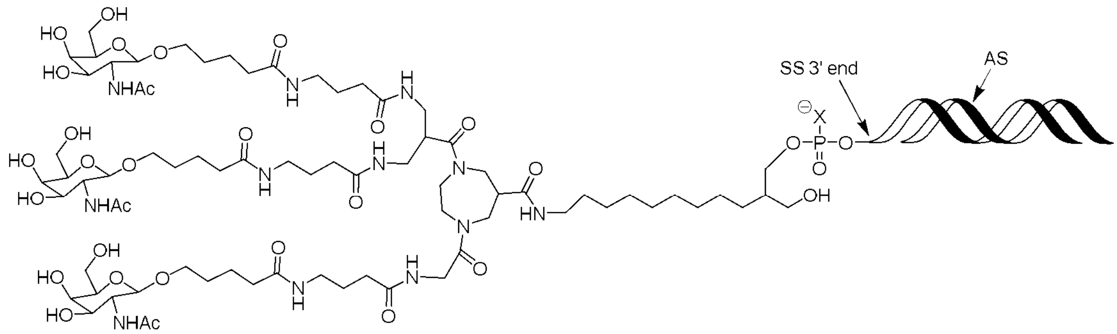

(Formula II),

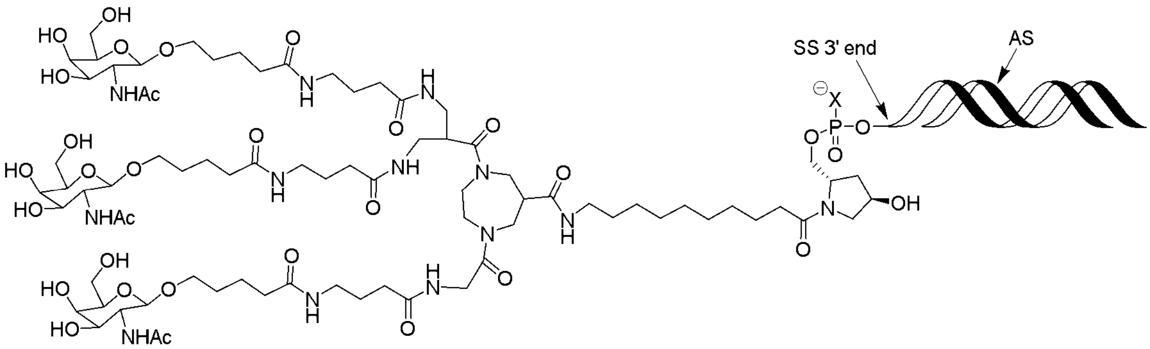

(Formula III),

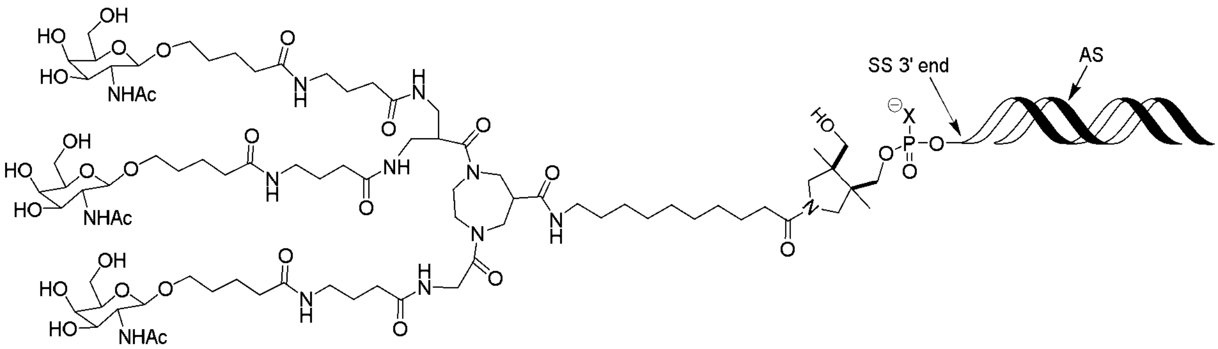

(Formula IV),

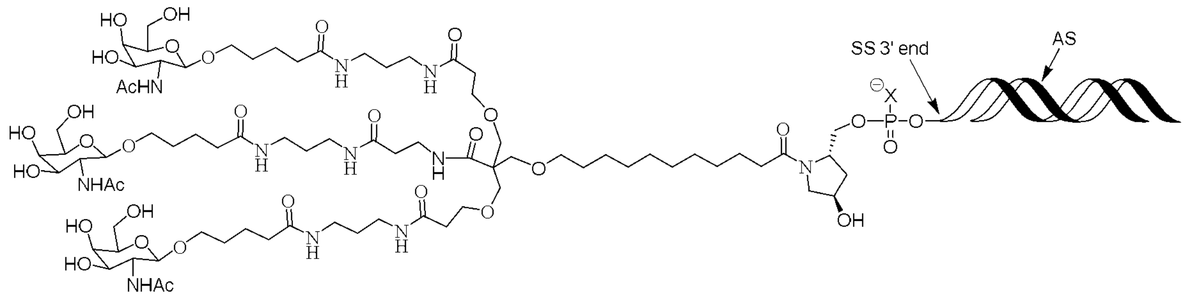

(Formula V),

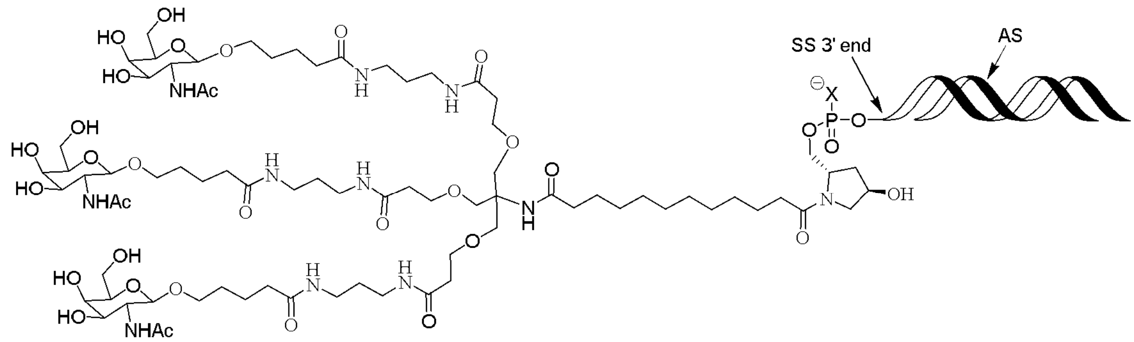

(Formula VI),

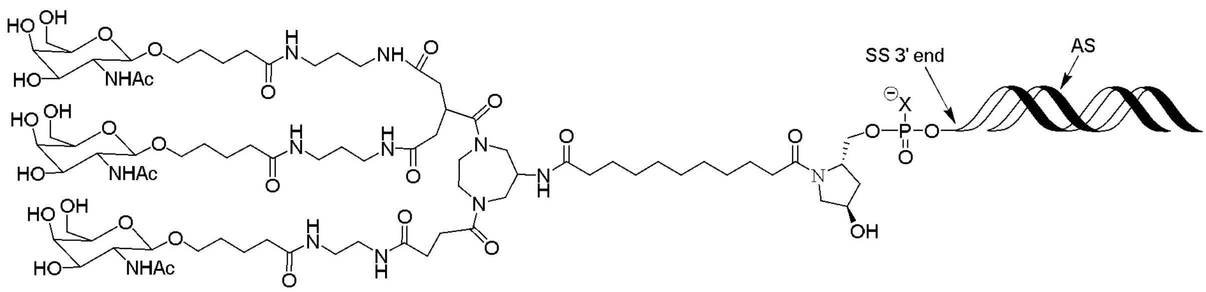

(Formula VII),

wherein SS represents the sense strand and AS represents the antisense strand;

X is selected from O and S.

The double-stranded siRNA conjugate provided by the present disclosure is as shown in Table 3.

Table 3: Double-stranded siRNA conjugates

| Conjugates No. | Sense strand sequences SEQ ID NO: | Ligands (conjugated at the 3' end of the sense chain via a phosphodiester linkage) | Antisense strand sequences SEQ ID NO: |
|---|---|---|---|
| Z1 | 27 | D08 | 56 |
| Z2 | 28 | D08 | 56 |
| Z3 | 29 | D08 | 56 |
| Z4 | 30 | D08 | 56 |
| Z5 | 31 | D08 | 56 |

(continued)

| Conjugates No. | Sense strand sequences SEQ ID NO: | Ligands (conjugated at the 3' end of the sense chain via a phosphodiester linkage) | Antisense strand sequences SEQ ID NO: |
|---|---|---|---|
| Z6 | 27 | D08 | 57 |
| Z7 | 27 | D08 | 58 |
| Z8 | 32 | D08 | 56 |
| Z9 | 33 | D08 | 56 |
| Z10 | 34 | D08 | 56 |
| Z11 | 27 | D08 | 59 |
| Z12 | 27 | D08 | 60 |
| Z13 | 27 | D08 | 61 |
| Z14 | 27 | D08 | 62 |
| Z15 | 27 | D08 | 63 |
| Z16 | 35 | D01 | 61 |
| Z17 | 36 | D01 | 63 |
| Z18 | 36 | D01 | 61 |
| Z19 | 37 | D01 | 61 |
| Z20 | 27 | D01 | 56 |
| Z21 | 36 | D01 | 64 |
| Z22 | 38 | D18 | 64 |
| Z23 | 39 | D18 | 64 |
| Z24 | 40 | D18 | 64 |
| Z25 | 41 | D18 | 64 |
| Z26 | 42 | D18 | 65 |
| Z27 | 43 | D18 | 66 |
| Z28 | 44 | D18 | 67 |
| Z29 | 45 | D18 | 68 |
| Z30 | 46 | D18 | 69 |
| Z31 | 43 | D18 | 70 |
| Z32 | 47 | D18 | 71 |
| Z33 | 47 | D18 | 72 |
| Z34 | 48 | D18 | 73 |
| Z35 | 49 | D18 | 74 |
| Z36 | 50 | D18 | 75 |
| Z37 | 51 | D18 | 76 |
| Z38 | 49 | D01 | 77 |
| Z39 | 49 | D21 | 77 |
| Z40 | 52 | D01 | 78 |
| Z41 | 49 | D01 | 79 |
| Z42 | 49 | D01 | 80 |

(continued)

| Conjugates No. | Sense strand sequences SEQ ID NO: | Ligands (conjugated at the 3' end of the sense chain via a phosphodiester linkage) | Antisense strand sequences SEQ ID NO: |
|---|---|---|---|
| Z43 | 49 | D01 | 81 |
| Z44 | 51 | D01 | 82 |
| Z45 | 50 | D01 | 82 |
| Z46 | 43 | D01 | 66 |
| Z47 | 51 | D01 | 83 |
| Z48 | 51 | D01 | 84 |
| Z49 | 51 | D01 | 85 |
| Z50 | 51 | D01 | 86 |
| Z51 | 49 | D01 | 87 |
| Z52 | 53 | D01 | 88 |
| Z53 | 49 | D01 | 89 |
| Z54 | 49 | D01 | 90 |
| Z55 | 49 | D01 | 91 |
| Z56 | 43 | D01 | 92 |
| Z57 | 49 | D01 | 93 |
| Z58 | 43 | D01 | 94 |
| Z59 | 43 | D01 | 95 |
| Z60 | 43 | D01 | 96 |
| Z61 | 54 | D01 | 97 |
| Z62 | 49 | D01 | 94 |
| Z63 | 49 | D01 | 95 |
| Z64 | 49 | D01 | 96 |
| Z65 | 55 | D01 | 95 |

In some embodiments of the present disclosure, the double-stranded siRNA conjugate is selected from the group consisting of Z27, Z42, Z43, Z46, Z50, Z52, Z56, Z58, Z59, Z60, Z62, Z63, Z64 and Z65.

In some embodiments of the present disclosure, the double-stranded siRNA conjugate is as shown in Formula I:

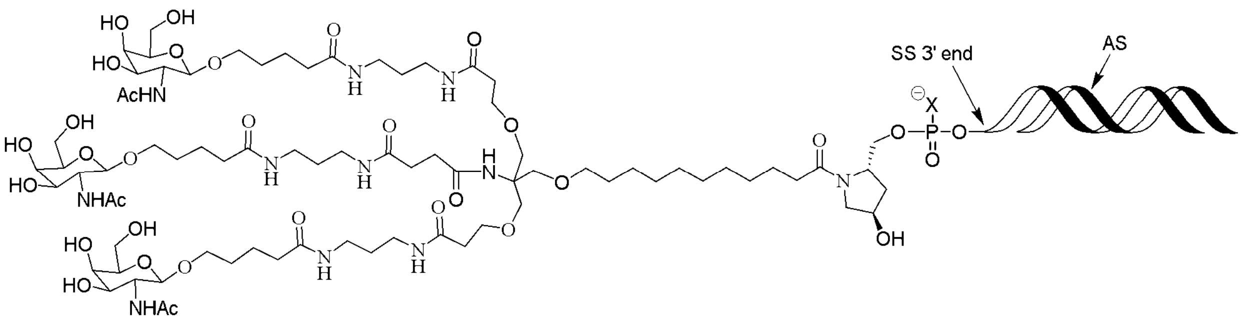


(Formula I),

SS is the sense strand and AS is the antisense strand; wherein, X is O; the sense strand and the antisense strand

are selected from any one of the combinations shown in (1) to (13):

(1) the sense strand set forth in SEQ ID NO: 49 and the antisense strand set forth in SEQ ID NO: 80;
(2) the sense strand set forth in SEQ ID NO: 49 and the antisense strand set forth in SEQ ID NO: 81;
(3) the sense strand set forth in SEQ ID NO: 43 and the antisense strand set forth in SEQ ID NO: 66;
(4) the sense strand set forth in SEQ ID NO: 51 and the antisense strand set forth in SEQ ID NO: 86;
(5) the sense strand set forth in SEQ ID NO: 53 and the antisense strand set forth in SEQ ID NO: 88;
(6) the sense strand set forth in SEQ ID NO: 43 and the antisense strand set forth in SEQ ID NO: 92;
(7) the sense strand set forth in SEQ ID NO: 43 and the antisense strand set forth in SEQ ID NO: 94;
(8) the sense strand set forth in SEQ ID NO: 43 and the antisense strand set forth in SEQ ID NO: 95;
(9) the sense strand set forth in SEQ ID NO: 43 and the antisense strand set forth in SEQ ID NO: 96;
(10) the sense strand set forth in SEQ ID NO: 49 and the antisense strand set forth in SEQ ID NO: 94;
(11) the sense strand set forth in SEQ ID NO: 49 and the antisense strand set forth in SEQ ID NO: 95;
(12) the sense strand set forth in SEQ ID NO: 49 and the antisense strand set forth in SEQ ID NO: 96;
(13) the sense strand set forth in SEQ ID NO: 55 and the antisense strand set forth in SEQ ID NO: 95.

The present disclosure also provides use of the double-stranded siRNA, the conjugate thereof, the salt thereof, or the salt of the conjugate thereof in the manufacture of a medicament for reducing Lp(a) levels.

In some embodiments of the present disclosure, the use is characterized in that the medicament for reducing Lp(a) levels is a medicament for treating a cardiovascular disease.

In some embodiments of the present disclosure, the cardiovascular disease include, but are not limited to, Burger's disease, peripheral artery disease, coronary artery disease, metabolic syndrome, aortic valve stenosis, aortic valve regurgitation, aortic dissection, retinal artery occlusion, mesenteric ischemia, superior mesenteric artery occlusion, renal artery stenosis, stable/unstable angina pectoris, acute coronary syndrome, heterozygous or homozygous familial hypercholesterolemia, hyperapolipoprotein beta lipoproteinemia, cerebrovascular atherosclerosis, and venous thrombosis.

### Technical effects

The double-stranded siRNAs, conjugates thereof, salts thereof, or salts of conjugates thereof of the present disclosure have excellent activity in reducing Lp(a) levels. They demonstrate excellent efficacy in the *in vitro* mouse HDI model and cynomolgus macaques (a.k.a Macaca fascicularis) PD model, significantly reducing LPA mRNA. Meanwhile, the double-stranded siRNA conjugates exhibit good stability, show long-lasting effects in *in vivo* experiments, and can be retained long-term in organisms and released slowly, enabling a long-interval administration. Double-stranded siRNA conjugates have a low risk of off-target effects, no risk of cytotoxicity, low risk of immunogenicity, and good safety profiles. Furthermore, the double-stranded siRNA conjugates can be easily synthesized and are suitable for industrial production.

### Definitions

Unless otherwise indicated, the following terms and phrases used herein are intended to have the meanings set forth below. A specific term or phrase without a specific definition should not be considered indefinite or unclear, but should be understood in the meaning as commonly understood by one of the ordinary skilled in the art. A trade name when described herein is intended to refer to the respective commercial products or active ingredient thereof.

Unless otherwise indicated, the terms "comprising," "including," and "containing" or equivalents as used herein are open-ended expressions, meaning that other unspecified elements, components, or steps may be encompassed in addition to those listed.

The term "optional" or "optionally" means that the subsequently described event or condition may, but does not necessarily, occur, and this expression covers instances where the event or condition occurs and instances where it does not.

Unless otherwise specified, the term "nucleic acid" as used herein refers to a polymer containing at least two nucleotides (i.e., deoxyribonucleotides or ribonucleotides) in single-stranded or double-stranded form and includes DNA and RNA. A "nucleotide" comprises a sugar (deoxyribose for DNA or ribose for RNA), a base, and a phosphate group. Nucleotides are linked together by phosphate groups. "Bases" include purines and pyrimidines, which further include natural compounds, adenine, thymine, guanine, cytosine, uracil, and inosine, and a natural analog thereof.

Unless otherwise specified, the term "oligonucleotide" or "oligo" as used herein refers to a polymer or an oligomer of nucleotide or nucleoside monomers composed of naturally occurring bases, sugars, and intersugar (backbone) linkages, and also includes a polymer or an oligomer comprising non-naturally occurring monomers or portions thereof, which functions similarly.

Unless otherwise specified, the "oligonucleotide" or "oligo" described herein is a nucleotide sequence comprising from 14 to 30 nucleotides or nucleotide base pairs. In some embodiments of the disclosure, the oligo has a nucleobase sequence that is at least partially complementary to a coding sequence in a target nucleic acid or target gene expressed within a cell. The nucleotides may be optionally modified. In some embodiments of the disclosure, the oligo upon delivery to a cell expressing a gene is capable of inhibiting or blocking the expression of the gene *in vitro* or *in vivo.* "Oligos" include, but are not limited to, single-stranded oligonucleotides, single-stranded antisense oligonucleotides, short interfering RNAs (siRNAs), double-stranded RNAs (dsRNAs), microRNAs (miRNAs), short hairpin RNAs (shRNAs), ribozymes, interfering RNA molecules, and Dicer enzyme substrates.

Unless otherwise specified, "short interfering RNAs (siRNAs)" as used herein are a class of RNA molecules with a length of 14-30 base pairs, similar to miRNAs. The siRNA operates within the RNA interference (RNAi) pathway, where it interferes with the translation of mRNA from a particular gene complementary to the nucleotide sequence, resulting in mRNA degradation. The short interfering RNA (siRNA) as used herein includes double-stranded siRNA (comprising the sense strand and the antisense strand) and single-stranded siRNA (such as with only the antisense strand).

Unless otherwise specified, "inhibition" as used herein, when referring to the expression of a given gene, means that the expression of the gene is reduced when a cell, population of cells or tissue is treated with the oligonucleotide of the present disclosure, compared to a cell, population of cells or tissue not so treated.

Unless otherwise specified, a "sequence" or "nucleotide sequence" as used herein denotes the order or sequence of nucleobases or nucleotides described using a sequence of letters in standard nucleotide nomenclature.

Unless otherwise specified, an "antisense strand (AS)", "template strand" or "guide strand" as used herein refers to a strand in the oligonucleotide compound that is substantially complementary to the corresponding region of a target sequence (e.g., LPA mRNA).

Unless otherwise specified, the term "sense strand", "sense strands" or "SS", "coding strand", or "passenger strand" as used herein refers to a strand capable of forming a substantially complementary region with the antisense strand. By "substantially complementary," it is meant that the corresponding positions of the two sequences may be completely complementary, or there may be one or more mismatches, with typically no more than 3, 2, or 1 mismatched base pair if there is. In a double-stranded nucleic acid molecule, bases of one strand are paired with bases on the other strand in a complementary manner. The purine base adenine (A) always pairs with the pyrimidine base uracil (U); the purine base guanine (G) always pairs with the pyrimidine base cytosine (C).

Unless otherwise specified, the "pharmaceutically acceptable conjugating group" of the present disclosure facilitates *in vivo* delivery of nucleic acids as well as compositions suitable for *in vivo* therapeutic use. In some embodiments, the "pharmaceutically acceptable conjugating group" of the present disclosure facilitates enhancing the affinity of nucleic acids as well as compositions suitable for *in vivo* therapeutic use for the target (such as target tissue/cell).

In some embodiments of the present disclosure, a "modification" as used herein refers to a modification to the base on a nucleotide or a substituted group of a nucleotide, a sugar ring, an internucleoside linkage, and/or a linkage between substituted groups of nucleotides, and the like. By way of example, the "modification" of the present disclosure includes, but is not limited to, MOE modification, methoxy modification, fluoro modification, (E)-vinyl phosphate modification, phosphorothioate group modification, or replacement of a nucleotide with glycerol nucleic acid (GNA), and the like. By way of example, the "modification" of the present disclosure may comprise one or more locked nucleic acids (LNAs). The locked nucleic acid comprises nucleotides having a modified ribose moiety, wherein the ribose moiety comprises an additional bridge connecting the 2' carbon and the 4' carbon. This structure effectively "locks" the ribose within the 3'-endostructure conformation.

Unless otherwise specified, "nucleotide is optionally modified" as used herein means that each nucleotide may independently be an unmodified nucleotide or a modified nucleotide, and the modification on each modified nucleotide is also independent. The "modification" includes, but is not limited to, a modification to a nucleobase, a modification to a ribose, and a modification to a phosphate. The "unmodified nucleotide" refers to a nucleotide composed of a naturally occurring nucleobase, a natural sugar ring, and a natural phosphate. The "modified nucleotide" refers to a nucleotide comprising at least one of a modified nucleobase, a modified sugar ring, and a modified phosphate. In some embodiments of the disclosure, a "modified nucleotide" refers to a nucleotide composed of a modified nucleobase, and/or a modified sugar ring, and/or a modified phosphate. In some embodiments of the disclosure, a "modified nucleotide" is composed of a modified nucleobase, a natural sugar ring, and a natural phosphate. In some embodiments of the disclosure, a "modified nucleotide" is composed of a natural nucleobase, a modified sugar ring, and a natural phosphate. In some embodiments of the disclosure, a "modified nucleotide" is composed of a natural nucleobase, a natural sugar ring, and a modified phosphate. In some embodiments of the disclosure, a "modified nucleotide" is composed of a natural nucleobase, a modified sugar ring, and a modified phosphate. In some embodiments of the disclosure, a "modified nucleotide" is composed of a modified nucleobase, a natural sugar ring, and a modified phosphate. In some embodiments of the disclosure, a "modified nucleotide" is composed of a modified nucleobase, a modified sugar ring, and a natural phosphate. In some embodiments of the disclosure, a "modified nucleotide" is composed of a modified nucleobase, a modified sugar

ring, and a modified phosphate.

Unless otherwise specified, the "natural sugar ring" of the present disclosure is selected from a five-membered sugar ring with a 2'-OH.

Unless otherwise specified, the "natural base" of the present disclosure is selected from the group consisting of the purine bases adenine (A) and guanine (G), and the pyrimidine bases thymine (T), cytosine (C), and uracil (U).

Unless otherwise specified, the "modified nucleobase" as used herein refers to a 5-12 membered saturated, partially unsaturated or aromatic heterocyclic ring other than a natural base, including monocyclic or fused rings, examples of which include, but are not limited to, thiophene, thianthrene, furan, pyran, isobenzofuran, benzothiazine, pyrrole, imidazole, substituted or unsubstituted triazole, pyrazole, isothiazole, isoxazole, pyridazine, indolizine, indole, isoindole, isoquinoline, quinoline, naphthyridine, quinazoline, carbazole, phenanthridine, piperidine, phenazine, phenothiazine, furanidine, phenoxazine, pyrrolidine, pyrroline, imidazolidine, imidazoline, pyrazolidine, 5-methylcytosine (5-me-C), 5-hydroxymethylcytosine, xanthine, hypoxanthine, 2-aminoadenine, 2-aminoguanine, 2-propyladenine and 2-propylguanine and other alkyl derivatives, 2-thiouracil, 2-thiothymine, 2-thiocytosine, 5-halouracil and 5-halocytosine, 5-propynyluracil and 5-propynylcytosine, 6-azouracil, 6-azocytosine, 6-azothymine, 5-uracil (pseudouracil), 4-thiouracil, 8-halo-, 8-amino-, 8-thiol-, 8-thioalkyl-, 8-hydroxy- and other 8-substituted adenines and guanines, 5-halo- especially 5-bromo-, 5-trifluoromethyl- and other 5-substituted uracils and cytosines, 7-methylguanine and 7-methyladenine, 8-azaguanine and 8-azaadenine, 7-deazaguanine and 7-deazaadenine, and 3-deazaguanine and 3-deazaadenine, and the like.

Unless otherwise specified, the "modified sugar ring" of the present disclosure may comprise, but is not limited to, one of the following modifications at the 2' position: H; F; O-, S-, or N-alkyl; O-, S-, or N-alkenyl; O-, S-, or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. Exemplary suitable modifications include $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, wherein n and m are from 1 to 10. In other embodiments, the modification at the 2' position include, but are not limited to, one of the following: substituted or unsubstituted $C_1$ to $C_{10}$ lower alkyl, alkylaryl, aralkyl, O-alkylaryl, or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, RNA-cleaving group, reporter group, intercalator, group for improving pharmacokinetic properties of iRNAs, or group for improving the pharmacodynamic characteristics of iRNAs, and other substituents with similar properties.

Unless otherwise specified, the "modified phosphate" in the present disclosure includes, but is not limited to: 1. Modifications to a phosphorothioate, including (R)- and (S)-isomers and/or mixtures thereof; 2. Modifications to a (E)-vinyl phosphate (E-VP or VP). Unless otherwise specified, the VP as used herein refers to the E-type vinyl phospholipid modification, for example, the structure of the mVPU at the 5' end is

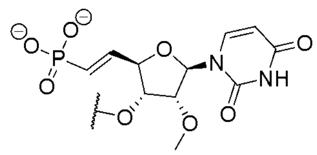

.

In some embodiments of the disclosure, the modified nucleotide may comprise one or more locked nucleic acids (LNAs). The locked nucleic acid comprises a nucleotide having a modified ribose moiety, wherein the ribose moiety comprises an additional bridge connecting the 2' carbon and the 4' carbon. This structure effectively "locks" the ribose within the 3'-endostructure conformation.

In some embodiments of the disclosure, the modified nucleotide comprises one or more monomers that are UNA (unlocked nucleic acid) nucleotides. The UNA is an unlocked acyclic nucleic acid in which any sugar bonds have been removed, thereby forming an unlocked "sugar" residue. In one example of the present disclosure, UNA also encompasses monomers from which bonds on C1'-C4' (i.e., covalent carbon-oxygen-carbon bonds between C1' and C4' carbons) have been removed. In another example of the present disclosure, the C2'-C3' bond (i.e., the covalent carbon-carbon bond between the C2'and C3' carbons) of the sugar has been removed.

In some embodiments of the disclosure, the modified nucleotide comprises one or more monomers that are GNA (glycerol nucleic acid) nucleotides. The GNA includes GNA-A, GNA-T, GNA-C, GNA-G, and GNA-U. The GNA-A structure is

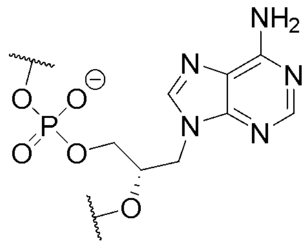

;

the GNA-T structure is

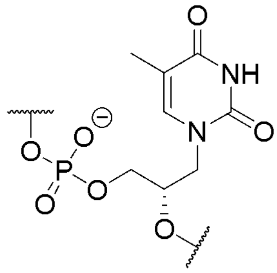

;

the GNA-C structure is

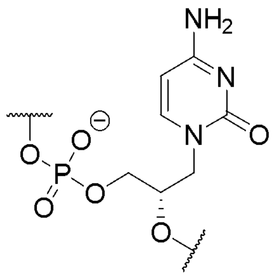

;

the GNA-G structure is

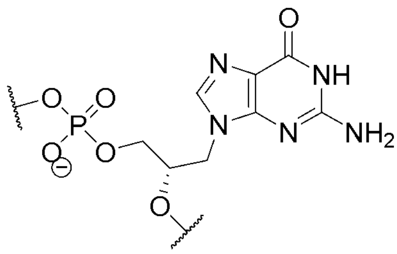

;

the GNA-U structure is

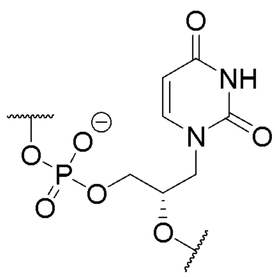

.

In some embodiments of the disclosure, the modified nucleotide may further include one or more bicyclic sugar

moieties. The "bicyclic sugar" is a furanosyl ring modified by the bridging of two atoms. A "bicyclic nucleoside" ("BNA") is a nucleoside having a sugar moiety comprising a bridge connecting two carbon atoms of a sugar ring, thereby forming a bicyclic ring system. In particular embodiments, the bridge connects the 4'-carbon and the 2'-carbon of the sugar ring.

Unless otherwise specified, the term "double-stranded siRNA" as used herein refers to a complex of ribonucleic acid molecules having a double-stranded structure, comprising two antiparallel and substantially complementary nucleotide strands, which have "sense" and "antisense" orientations relative to the target RNA. In the present disclosure, the "complementary" has a meaning well-known to those skilled in the art, i.e., in a double-stranded nucleic acid molecule, bases of one strand are paired in a complementary manner with bases on the other strand. The purine base adenine (A) is always paired with the pyrimidine base uracil (U); the purine base guanine (G) is always paired with the pyrimidine base cytosine (C). Each base pair comprises one purine and one pyrimidine. Two strands are considered complementary to each other when adenine on one strand is always paired with uracil on the other and guanine is always paired with cytosine, and the sequence of a strand can be inferred from the sequence of its complementary strand.

Unless otherwise indicated, the term "substantially complementary" as used herein means that the corresponding positions of the two sequences may be completely complementary, or there may be one or more mismatches, with typically no more than 5, 4, 3, 2, or 1 mismatched base pairs if there is.

Unless otherwise specified, the term "conjugation" as used herein means that two or more chemical moieties, each having a specific function, are linked to each other via a covalent linkage. Accordingly, a "conjugate" refers to a compound formed by the covalent linkage between each chemical moiety.

Unless otherwise specified, the term "conjugate of a double-stranded siRNA" as used herein refers to a compound formed by linking a double-stranded siRNA and a pharmaceutically acceptable conjugating group, and the double-stranded siRNA and a pharmaceutically acceptable conjugating group are covalently linked.

Unless otherwise specified, the term "covalent linkage" in the present disclosure includes, but is not limited to, linkage such as "phosphoester linkage", "phosphodiester linkage", and "phosphorothioate linkage".

In some embodiments of the present disclosure, the "modification" to a nucleotide in the present disclosure includes modification to a nucleobase, modification to a sugar ring, modification to an internucleotide linkage, and the like. By way of example, the "modification" to a nucleotide in the present disclosure includes, but is not limited to, a methoxy modification, a fluoro modification, a (E)-vinyl phosphate modification, a phosphorothioate modification, or replacement of the nucleotide with an (S)-glycerol nucleic acid, and the like.

In some embodiments of the disclosure, "A", "U", "C" and "G" in the sequence represent adenosine, uridine, cytidine and guanosine, respectively. When marked preceding the "A", "U", "C" and "G", an "m" represents a nucleotide or a substituted nucleotide is 2'-O-methyl modified (belonging to the methoxy modification), e.g., mA represents a 2'-O-methyladenosine; an "f" represents a nucleotide or a substituted nucleotide is a 2'-fluoronucleotide modified (belonging to the fluoro modification), e.g., fG represents a 2'-fluoroguanosine; an "d" represents a deoxygen modification at the 2' position of a nucleotide or a substituted nucleotide, e.g., dA represents a 2'-deoxyadenosine, and a dT represents a 2'-deoxythymidine.

In some embodiments of the disclosure, when marked between the "Z", "R", "A", "U", "C" and "G", an "s" indicates that nucleotides are connected via a phosphorothioate linkage, e.g., "fGs, mA" indicates that the fG and the mAa are connected via the phosphorothioate linkage, i.e., the 2'-fluoroguanosine and 2'-O-methyladenosine are connected via the phosphorothioate linkage. By way of example, the chemical structure of 5'-fCs, mG, fU, mA, Z-3' is as follows:

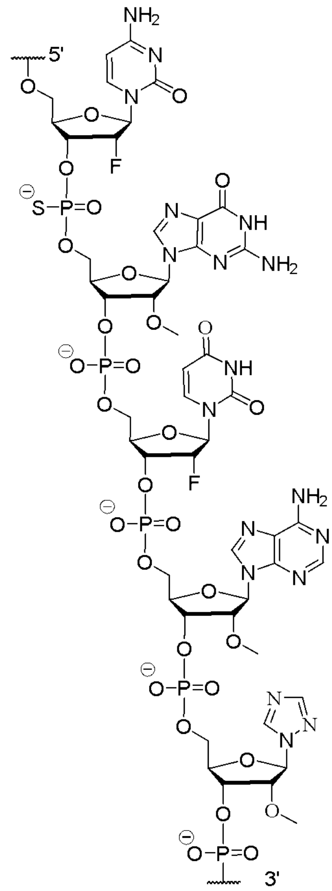

.

Unless otherwise specified, the prefix "multi-" in the terms "multiple" and "multivalent" as used herein refers to an integer greater than or equal to 2, including but not limited to 2, 3, 4, 5, 6, 7, 8, 9, 10, 11, 12, 13, 14, 15, 16, 17, 18, 19, or 20, up to the theoretical maximum of the siRNA or the GalNAc derivative attached via a branched linkage.

The sense strand or antisense strand of the double-stranded siRNA of the disclosure may further comprise an "overhang", such as unpaired overhang nucleotides not directly involved in the RNA double helix structure, wherein the RNA double helix structure is typically formed by a pair of the "sense strand" and "antisense strand" as defined herein. By way of example, such overhangs may comprise one or more modified or unmodified C, G, U, A, Z, R, and T.

The compounds of the present disclosure may be present in specific geometric or stereoisomeric forms. The present disclosure contemplates all such compounds, including a (*R*)- and (S)-enantiomer, a diastereomer, and a racemic mixture and other mixtures thereof, such as an enantiomerically or diastereomerically enriched mixture, all of which are within the scope of the present disclosure. Additional asymmetric carbon atoms may be present in a substituent such as an alkyl group. All such isomers, as well as mixtures thereof, are included within the scope of the disclosure.

Unless otherwise specified, the term "enantiomer" or "optical isomer" refers to stereoisomers that are mirror images of each other.

Unless otherwise specified, the term "diastereomer" refers to stereoisomers where the molecule has two or more chiral centers and the molecules are non-mirror images of each other.

Unless otherwise specified, a wedge solid bond ( ) and a wedge dashed bond ( ) are used to indicate the absolute configuration of a stereocenter, a straight solid bond ( ) and a straight dashed bond ( ) are used to indicate the relative configuration of a stereocenter, and a wavy line ( ) is used to represent a solid wedge bond ( ) or a dashed wedge bond ( ), or a wavy line ( ) is used to represent a solid straight line bond ( ) and/or a dashed straight line bond ( ).

Unless otherwise specified, the term "enriched in one isomer", "isomerically enriched", "enriched in one enantiomer" or "enantiomerically enriched" means that the content of one isomer or enantiomer is less than 100%, and the content of the isomer or enantiomer is greater than or equal to 60%, or greater than or equal to 70%, or greater than or equal to 80%, or greater than or equal to 90%, or greater than or equal to 95%, , or greater than or equal to 96%, or greater

than or equal to 97%, or greater than or equal to 98%, or greater than or equal to 99%, or greater than or equal to 99.5%, or greater than or equal to 99.7%, or greater than or equal to 99.8%, or greater than or equal to 99.9%.

Unless otherwise specified, the term "isomeric excess" or "enantiomeric excess" refers to the differentials between the relative percentages of the two isomers or the two enantiomers. For example, where the content of one isomer or enantiomer is 90% and the content of the other isomer or enantiomer is 10%, the isomeric or enantiomeric excess (ee value) is 80%.

Optically active (R)- and (S)-isomers, and *D* and *L* isomers, may be prepared by chiral synthesis or chiral reagents, or other conventional techniques. If one enantiomer of a compound of the present disclosure is desired, it may be prepared by asymmetric synthesis or derivatization with a chiral auxiliary, where the resulting diastereomeric mixture is separated and the auxiliary group is cleaved to provide the pure desired enantiomer. Alternatively, in the case the molecule contains a basic functional group (such as an amino group) or an acidic functional group (such as a carboxyl group), a diastereomeric salt is formed with an appropriate optically active acid or base, followed by resolution of the diastereomer by conventional methods well known in the art, and then recovery of the pure enantiomer. Furthermore, separation of enantiomers and diastereomers is usually accomplished using chromatography employing a chiral stationary phase, optionally in combination with chemical derivatization (e.g., carbamate generation from amines). The compound of the present disclosure may contain atomic isotopes in unnatural proportions on one or more of the atoms constituting such compound. For example, the compound may be labeled with radioisotopes such as tritium ($^3H$), iodine-125 ($^{125}I$), or C-14 ($^{14}C$). Also, for example, hydrogen may be replaced with heavy hydrogen to form deuterated drugs. The bond formed between deuterium and carbon is stronger than that between ordinary hydrogen and carbon. Compared with unduterated drugs, the deuterated drugs have advantages such as reduced toxic side effects, increased drug stability, enhanced efficacy, and prolonged biological half-life of drugs. All variations in the isotopic composition of the compounds of the present disclosure, whether radioactive or not, are encompassed within the scope of the present disclosure.

The term "salt" refers to a salt of the compound of the present disclosure, prepared from a compound found in the present disclosure having a specific substituent and a relatively non-toxic acid or base. When the compound of the disclosure contains a functional group that is relatively acidic, a base addition salt may be obtained by contacting such a compound with a sufficient amount of a base, either in a pure solution or in a suitable inert solvent. Pharmaceutically acceptable base addition salts include sodium, potassium, calcium, ammonium, organic amine or magnesium salts, or similar salts. When the compound of the disclosure contains a functional group that is relatively basic, an acid addition salt may be obtained by contacting such a compound with a sufficient amount of an acid, either in a pure solution or in a suitable inert solvent. Examples of pharmaceutically acceptable acid addition salts include salts of inorganic acids including, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and salts of organic acids including, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic, acid, citric acid, tartaric acid and methanesulfonic acid, and similar acids; also included are salts of amino acids such as arginine, as well as salts of organic acids such as glucuronic acid. Certain specific compounds of the present disclosure contain both basic and acidic functional groups, allowing conversion into either a base or acid addition salt.

The salts of the present disclosure may be synthesized from the parent compound containing an acid radical or a basic group by conventional chemical methods. In general, such salts are prepared by reacting the compound in free acid or base form with a stoichiometric amount of the appropriate base or acid in water or in an organic solvent or in a mixture of both.

The compound of the present disclosure may be prepared by a variety of synthetic methods well-known to those skilled in the art, including the specific embodiments listed below, embodiments formed by combination with other chemical synthetic methods, and equivalent alternatives well-known to those skilled in the art. Preferred embodiments include, but are not limited to, the embodiments of the present disclosure.

The structure of the compounds of the present disclosure may be confirmed by conventional methods well-known to those skilled in the art. If the present invention relates to an absolute configuration of a compound, the absolute configuration may be confirmed by conventional techniques in the art. For example, single-crystal X-ray diffraction (SXRD) may be used. The cultivated single crystal is subjected to diffraction intensity data collection using a Bruker D8 Venture diffractometer with CuK$\alpha$ radiation as the light source, scanning method: $\varphi/\omega$ scanning. After collecting relevant data, the crystal structure is further resolved using the direct method (Shelxs97) to confirm the absolute configuration.

The solvents used in the present disclosure are commercially available.

Unless otherwise specified, the solvent ratios for column chromatography and preparative thin-layer silica gel chromatography of the present disclosure are all by volume ratio.

The following abbreviationsare used in the present disclosure: DMSO represents a dimethyl sulfoxide; Cbz represents a benzyloxycarbonyl; Boc represents a tert-butoxycarbonyl; Ac represents an acetyl; DMTr represents a dimethoxytrityl; DCM represents a dichloromethane; i-PrOH represents an isopropanol; Bn represents a benzyl; Bz

represents a benzoyl; Me represents a methyl; i-Pr represents an isopropyl; OSu represents a

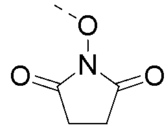

;

M is a concentration unit, in mol/L; TsOH represents a p-toluenesulfonic acid; TBS represents a tertbutyldimethylsilyl; i-Pr represents an isopropyl; LPS represents a lipopolysaccharide.

Compounds are designated according to conventional nomenclature in the art or using ChemDraw® Software; commercially available compounds are referred to by their supplier catalog names.

## DETAILED DESCRIPTION

The present invention is described in detail below by means of examples, but it does not imply any undue limitation of the present invention. The compound of the present disclosure may be prepared by a variety of synthetic methods well-known to those skilled in the art, including the specific embodiments listed below, embodiments formed by combination with other chemical synthetic methods, and equivalent alternatives well-known to those skilled in the art. Preferred embodiments include, but are not limited to, the examples of the present disclosure. Various changes and modifications to the specific embodiments of the present disclosure will be apparent to those skilled in the art without departing from the spirit and scope of the present invention.

### Example 1: Synthesis of Double-Stranded siRNAs or Conjugates Thereof

Synthesis of Single-Stranded Oligoribonucleotides Containing Conjugating Groups: Oligoribonucleotides were synthesized according to the phosphoramidite solid-phase synthesis technique. The synthesis was carried out on a solid support made by covalent bonding of controlled-porous glass (CPG, 500 Å) to conjugating group intermediate compounds. All RNA phosphoramidite monomers on a sequence were dissolved in anhydrous acetonitrile successively according to the order from 3' to 5' of the sequence, and molecular sieves (3 Å) were added. 5-Ethylthio-1H-tetrazole (ETT) was used as an activator for 5 minutes. A 50 mM I2-water/pyridine (1/9 by volume) solution was used for the reaction for 3 minutes to generate a phosphate linkage. A 50 mM solution of 3-((dimethylamino-methylene) amino)-3H-1, 2, 4-dithiazole-3-thione (DDTT) in anhydrous acetonitrile/pyridine (1/1 by volume) was used for the reaction for 3 minutes to generate a phosphorothioate linkage. The resulting sequence was synthesized upon a final removal of the DMT group.

Synthesis of Single-Stranded Oligoribonucleotides Without Conjugating Groups: Oligoribonucleotides were synthesized according to the phosphoramidite solid-phase synthesis technique. The synthesis was carried out on a solid support made of controlled-porous glass (CPG). All RNA phosphoramidite monomers on a sequence were dissolved in anhydrous acetonitrile successively according to the order from 3' to 5' of the sequence and molecular sieves (3 Å) were added. 5-Ethylthio-1H-tetrazole (ETT) was used as an activator for a coupling time of 5 minutes. A 50 mM $I_2$-water/pyridine (1/9 by volume) solution was used for the reaction for 3 minutes to generate a phosphate linkage. A 50 mM solution of 3-((dimethylamino-methylene) amino)-3H-1, 2, 4-dithiazole-3-thione (DDTT) in anhydrous acetonitrile/pyridine (1/1 by volume) was used for the reaction for 3 minutes to generate a phosphorothioate linkage. The resulting sequence was synthesized upon a final removal of the DMT group.

Cleavage and Deprotection of Oligomers Bound on the CPG: After completion of solid-phase synthesis, protecting groups were removed by treatment with a solution of 10% diethylamine in acetonitrile for 30 min without cleavage of nucleotides from the CPG. Subsequently, the dried CPG was treated with concentrated aqueous ammonia at 40 °C for 18 hours. After centrifugation, the supernatant was transferred to a new tube and the CPG was washed with water. The combined solutions were concentrated to obtain a solid mixture. The solid mixture, in case of containing natural nucleotides, was dissolved in DMSO (10V), followed by the addition of a triethylamine hydrofluoric acid salt (2V), with stirring at 40 °C for 16 h. The reaction was purified after completion.

Purification of Single-Stranded Oligoribonucleotides: The oligomers were purified by HPLC through anion exchange using NanoQ. Buffer A: 10 mM sodium perchlorate solution, 20 mM Tris, 1 mM EDTA, pH 7.4 and containing 20% acetonitrile, and buffer B: 500 mM sodium perchlorate, 20 mM Tris, 1 mM EDTA, pH 7.4 and containing 20% acetonitrile. The desired product was isolated and desalted using a reverse-phase C18 column.

Annealing of Single-Stranded Oligoribonucleotides to Produce siRNA: The single-stranded oligoribonucleotides to be annealed were prepared as a 200 μM solution with sterile RNase Free $H_2O$ (free of RNA hydrolase). The annealing reaction system was set up as follows: a total volume of 100 μL of the mixture, when containing 10 nmol single-stranded oligoribonucleotides, was placed in a 95°C water bath for 10 minutes (it requires 20 minutes at the high temperature when ≥

100 nmol) → quickly transferred into a 60°C water bath and allowed to cool naturally → the solution after annealing should not be stored at the high temperature. Complementary strands were formed by combining equimolar solutions of single-stranded oligoribonucleotides.

Example 2: Synthesis of Z-M

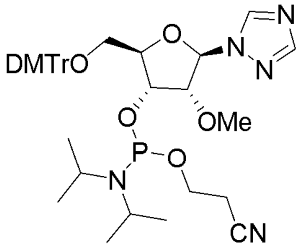

**Z-M**

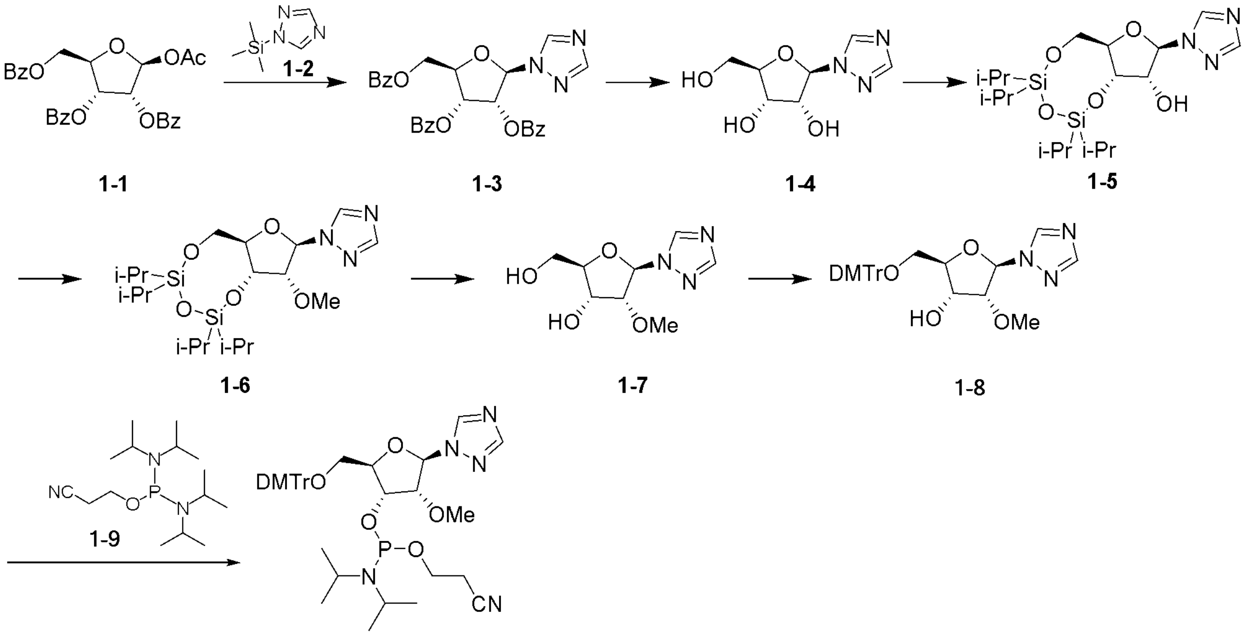


1-1 1-3 1-4 1-5

1-6 1-7 1-8

Z-M

Step A: Compound 1-1 (10 g, 19.82 mmol) was dissolved in acetonitrile (120 mL) and 1,2-dichloroethane (80 mL), to which Compound 1-2 (6.02 g, 42.62 mmol) and trimethylsilyltrifluoro-methanesulfonate (11.01 g, 49.56 mmol) were added at 0 °C. The mixture was stirred at 35 °C for 12 hours. The reaction mixture was slowly quenched by adding saturated aqueous sodium bicarbonate solution (100 mL) at 0 °C and extracted with dichloromethane (100 mL × 2). The resulting organic phase was washed with saturated aqueous sodium chloride solution (200 mL), dried over anhydrous sodium sulfate, filtered, and concentrated under reduced pressure to give a crude product. The crude product was purified by silica gel column chromatography (eluent: petroleum ether/ethyl acetate = 20:1) to give Compound 1-3.

Step B: Compound 1-3 (6.7 g, 13.05 mmol) was dissolved in an ammonia solution in methanol (7 M, 50 mL). The mixture was stirred at 40 °C for 12 hours. The reaction mixture was concentrated under reduced pressure to give a crude product, which was purified by silica gel column chromatography (eluent: ethyl acetate/methanol = 50/1 to 10/1) to give Compound **1-4.**

Step C: Compound **1-4** (2.3 g, 11.43 mmol) was dissolved in anhydrous pyridine (25 mL), to which 1,3-dichloro-1,1,3,3-tetraisopropyldisiloxane (3.64 g, 11.55 mmol) was added at 0 °C. The mixture was stirred at 20 °C for 12 hours. The reaction mixture was quenched by adding water (30 mL) and extracted with ethyl acetate (30 mL × 2). The resulting organic phase was successively washed with hydrochloric acid (1 M, 30 mL × 3) and saturated brine (30 mL), dried over anhydrous sodium sulfate, filtered, and concentrated under reduced pressure to give a crude product. The crude product was purified by silica gel column chromatography (eluent: petroleum ether/ethyl acetate = 7/1 to 5/1) to give

Compound **1-5.**

Step D: Compound **1-5** (4 g, 9.02 mmol) was dissolved in acetonitrile (40 mL), to which silver oxide (8.36 g, 36.06 mmol), 4 Å molecular sieve (3 g), anhydrous pyridine (1.78 g, 22.54 mmol) and methyl iodide (6.4 g, 45.08 mmol) were added successively. The mixture was stirred at 25 °C for 12 hours. Ethyl acetate (50 mL) was added to the reaction mixture with stirring at 20 °C for 1 hour, filtered, and the filtrate was collected. The filtrate was extracted with water (100 mL) and ethyl acetate (100 mL × 2). The resulting organic phase was washed with saturated brine (200 mL), dried over anhydrous sodium sulfate, filtered, and concentrated under reduced pressure to give a crude product. The crude product was purified by silica gel column (eluent: petroleum ether/ethyl acetate = 15/1 to 10/1 to 8/1) to give Compound **1-6.**

Step E: Compound **1-6** (2 g, 4.37 mmol) was dissolved in anhydrous tetrahydrofuran (20 mL), to which triethylamine trihydrofluoride (1.55 g, 9.61 mmol, 1.57 mL) was added. The mixture was stirred at 20 °C for 12 hours. The reaction mixture was concentrated under reduced pressure to obtain a crude product, which was purified by silica gel column chromatography (eluent: ethyl acetate/methanol = 100/1 to 50/1) to give Compound **1-7.**

Step F: Compound **1-7** (1 g, 4.65 mmol) was dissolved in anhydrous pyridine (10 mL), to which 4, 4-bismethoxytrityl chloride (1.57 g, 4.65 mmol) was added. The mixture was stirred at 20 °C for 12 hours. The reaction mixture was quenched by adding water (20 mL) and extracted with ethyl acetate (20 mL × 2). The resulting organic phase was washed with saturated brine (40 mL), dried over anhydrous sodium sulfate, filtered, and concentrated under reduced pressure to give a crude product. The crude product was purified by silica gel column chromatography (eluent: petroleum ether/ethyl acetate = 3/1 to 1/1) to give Compound **1-8.** $^1H$ NMR (400 MHz, DMSO-$d_6$):$\delta$ = 8.78 (s, 1H), 8.14-8.03 (m, 1H), 7.41-7.31 (m, 2H), 7.29-7.16 (m, 7H), 6.92-6.76 (m, 4H), 6.08 (d, *J* = 3.2 Hz, 1H), 5.25 (d, *J* = 6.5 Hz, 1H), 4.48-4.34 (m, 1H), 4.13-4.01 (m, 2H), 3.73 (s, 6H), 3.38 (s, 3H), 3.18-3.03 (m, 2H).

Step G: Compounds **1-8** (3 g, 5.80 mmol) and **1-9** (1.92 g, 6.38 mmol) were dissolved in anhydrous dichloromethane (30 mL), and 4, 5-dicyanoimidazole (342.26 mg, 2.90 mmol) was added under a nitrogen atmosphere. The mixture was stirred at 25 °C for 2 hours. 15 mL of saturated aqueous sodium bicarbonate solution and 15 mL of water were added to the reaction mixture, which was then extracted with dichloromethane (30 mL × 2). The combined organic phases were washed with saturated brine (30 mL x 2), dried over anhydrous sodium sulfate, filtered, and concentrated to give a crude product, which was purified by silica gel column chromatography (eluent: petroleum ether/ethyl acetate (0.1% triethylamine) = 1/0 to 2/1) to give Compound **Z-M.** $^1H$ NMR (400 MHz, DMSO-$d_6$):$\delta$ = 8.83 (s, 1H), 8.11 (d, *J* = 6.2 Hz, 1H), 7.38 (br t, *J* = 6.9 Hz, 2H), 7.32-7.15 (m, 7H), 6.92-6.76 (m, 4H), 6.26-6.05 (m, 1H), 4.78-4.56 (m, 1H), 4.31 (br d, *J* = 3.8 Hz, 1H), 4.25-4.12 (m, 1H), 3.85-3.77 (m, 1H), 3.73 (br s, 6H), 3.66-3.46 (m, 3H), 3.39 (br d, *J* = 15.7 Hz, 3H), 3.32-3.19 (m, 1H), 3.10 (br dd, *J* = 5.4, 10.0 Hz, 1H), 2.78 (br t, *J* = 5.7 Hz, 1H), 2.60 (br t, *J* = 5.5 Hz, 1H), 1.22-1.04 (m, 9H), 0.98 (br d, *J* = 6.5 Hz, 3H). $^{31}P$ NMR (162 MHz, DMSO-$d_6$):$\delta$ =149.33 (br d, *J* = 11.7 Hz, 1P). LCMS (ESI) m/z: 718 $[M+H]^+$.

**Example 3: Synthesis of D08-M**

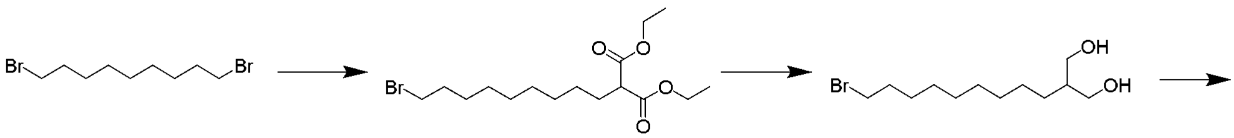

2-11-1 2-11-2 2-11-3

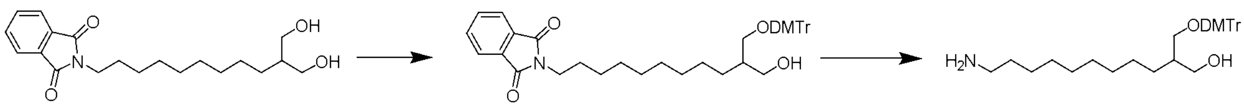

2-11-4 2-11-5 2-11

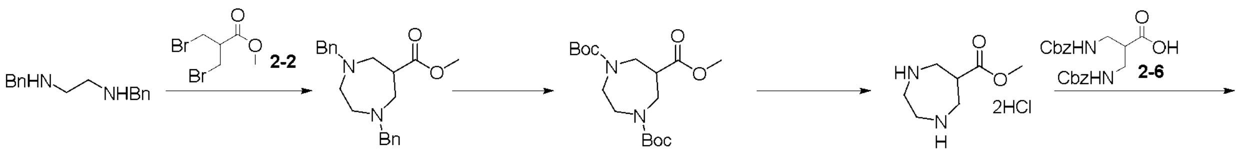


2-1 2-3 2-4 2-5

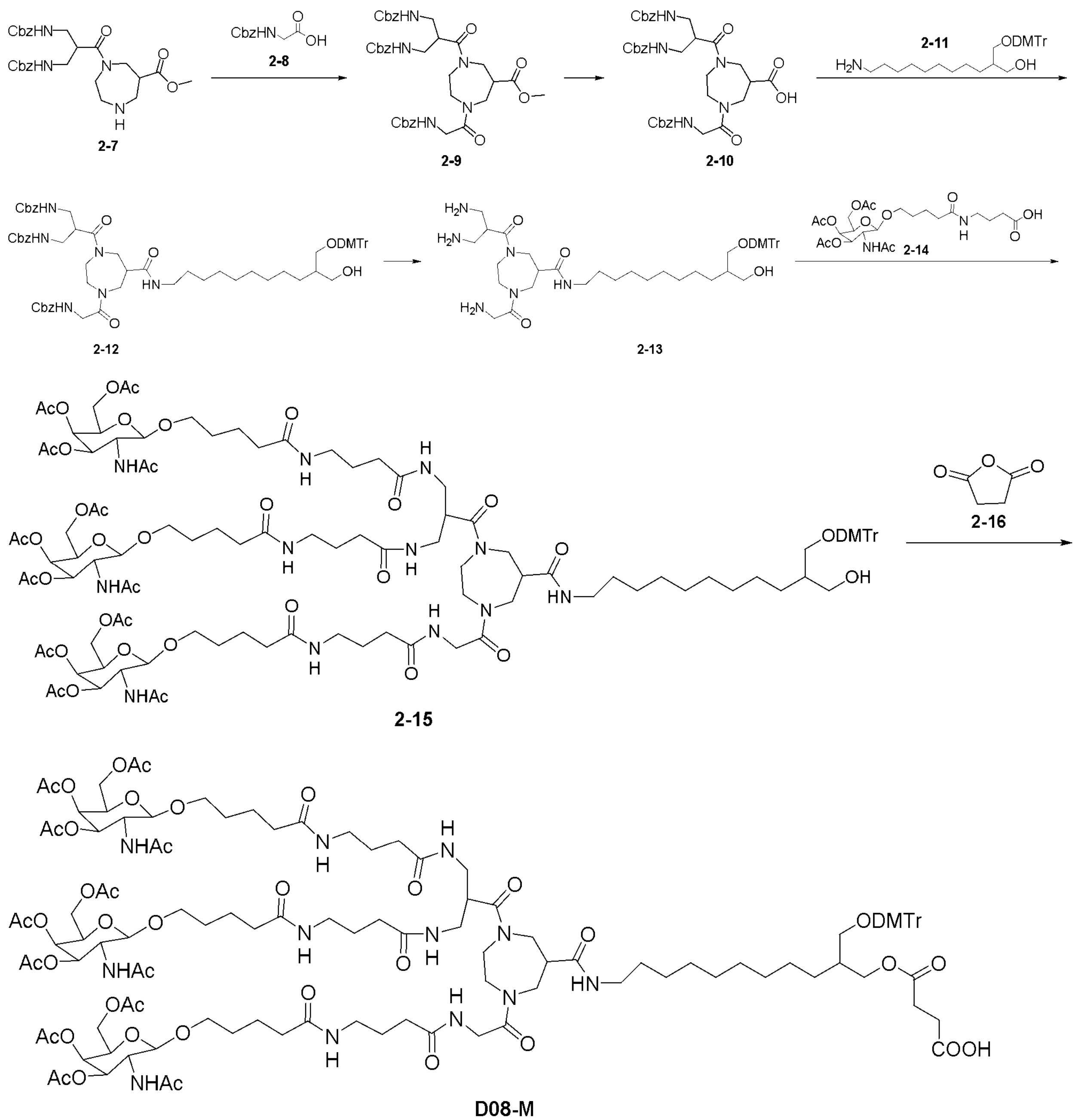


Step A: Under nitrogen protection, the sodium hydride (2.75 g, 68.68 mmol, 60% purity) was added portionwise to a solution of diethyl malonate (10 g, 62.43 mmol) in tetrahydrofuran (100 mL) at 0 °C. After the addition, the reaction was stirred for 0.5 hours, and then a solution of Compound **2-11-1** (17.86 g, 62.43 mmol) in tetrahydrofuran (50 mL) was added dropwise at 0 °C. The mixture was stirred at 25 °C for 15.5 hours. At 10-25 °C, the reaction was quenched by adding saturated ammonium chloride (20 mL), diluted with water (20 mL), and extracted with ethyl acetate (50 mL * 2). The extract was washed with saturated brine (30 mL * 2), dried over anhydrous sodium sulfate, filtered, and concentrated under reduced pressure to obtain a crude product. The crude product was purified by silica gel column chromatography (petroleum ether/ethyl acetate = 1/0 to 9/1) to give Compound **2-11-2.**

Step B: Under nitrogen protection, sodium borohydride (15.2 g, 401.77 mmol) was added in portions to a solution of Compound **2-11-2** (15 g, 41.06 mmol) in methanol (150 mL) at 60 °C. The addition was completed over 0.5 hours, with stirring for 0.5 hours for the reaction. The mixture was stirred at 65 °C for 1 hour. At 0-25 °C, the reaction was quenched with water, stirred for 0.5 hours, diluted with water (50 mL), concentrated at 30-35 °C under reduced pressure to remove methanol, and extracted with ethyl acetate (100 mL * 2). The extract was washed with saturated brine (50 mL * 2), dried with anhydrous sodium sulfate, filtered, and concentrated under reduced pressure to give Compound **2-11-3.**

Step C: Compound **2-11-3** (8.5 g, 30.22 mmol) and the phthalimide potassium salt (14.00 g, 75.56 mmol) were mixed in N, N-dimethylformamide (90 mL) solution. The system was subjected to nitrogen displacement three times, and

the reaction mixture was reacted at 100 °C under nitrogen protection for 16 hours. The reaction mixture was diluted with water (300 mL), resulting in the precipitation of a solid. A filter cake was collected by filtration, and the filter cake was purified by silica gel column chromatography (petroleum ether/ethyl acetate = 1/0 to 1/1) to give Compound **2-11-4.**

Step D: Compound **2-11-4** (6.1 g, 17.56 mmol), 4,4-dimethoxytrityl chloride (7.14 g, 21.07 mmol) and triethylenediamine (2.95 g, 26.33 mmol) were mixed in dichloromethane (60 mL) solution. The system was subjected to nitrogen displacement three times, and the reaction mixture was reacted at 25 °C for 16 hours under nitrogen protection. The reaction mixture was diluted with water (30 mL), extracted with dichloromethane (50 mL). The resulting organic phase was washed with saturated brine (30 mL * 2), dried over anhydrous sodium sulfate, filtered, and concentrated under reduced pressure to obtain a crude product. The crude product was purified by silica gel column chromatography (petroleum ether/ethyl acetate = 1/0 to 4/1, 0.1% triethylamine was added) to give Compound **2-11-5.**

Step E: Compound **2-11-5** (7.1 g, 10.93 mmol) and hydrazine monohydrate (1.52 g, 29.76 mmol, 98% purity) were mixed in ethanol (80 mL), and then the system was subjected to nitrogen displacement three times. The reaction mixture was reacted at 70 °C for 16 hours under nitrogen protection. The ethanol **was** then removed by concentration under reduced pressure at 35-40 °C to obtain intermediate **2-11.**

Step F: At 25 °C, triethylamine (10.10 g, 99.86 mmol) and a solution of Compound **2-1** (12 g, 49.93 mmol) in toluene (100 mL) were added to a solution of Compound **2-2** (13.24 g, 50.93 mmol) in toluene (150 mL). The reaction was stirred at 100 °C for 16 hours. A saturated aqueous sodium carbonate solution (200 mL) was added to the reaction mixture, and then the mix was subjected to liquid separation. The resulting organic phase was dried over anhydrous sodium sulfate, filtered, and concentrated to give the crude product. The crude product was isolated by preparative HPLC (column: Agela Innoval ODS-2 250 mm * 100 mm * 10 μm; mobile phase: [water (formic acid)-acetonitrile]; % acetonitrile: 15%-35%, 25 min) to give Compound **2-3.**

Step G: A solution of Compound **2-3** (12.6 g, 31.04 mmol), palladium on carbon (5 g, 10% palladium content), and di-tert-butyl carbonic anhydride (14.90 g, 68.28 mmol) in methanol (120 mL) was subjected to argon displacement three times, followed by hydrogen displacement three times. The reaction mixture was stirred at 25 °C under a hydrogen atmosphere at ordinary pressure for 16 hours. The reaction mixture was filtered and concentrated under reduced pressure. The crude product was purified by silica gel column chromatography to give Compound **2-4.** $^1$H NMR (400 MHz, $CDCl_3$) δ ppm 3.60 - 3.95 (m, 7H) 3.36 - 3.50 (m, 2H) 3.19 - 3.34 (m, 2H) 2.96 - 3.12 (m, 1H) 1.46 (s, 18H).

Step H: Compound **2-4** (7.5 g, 18.66 mmol) was dissolved in hydrochloric acid ethyl acetate solution (4 mol/L, 18.66 mL). The system was subjected to nitrogen displacement three times, and then stirred at 25 °C for 1 hour under nitrogen production. The reaction mixture was concentrated under reduced pressure to give Compound **2-5.**

Step I: A solution of Compound **2-6** (2.2 g, 5.69 mmol), Compound **2-5** (2.24 g, 9.68 mmol), triethylamine (2.88 g, 28.47 mmol), and 1-propylphosphoric cyclic anhydride (5.06 g, 7.95 mmol, 50% ethyl acetate solution) in N,N-dimethylformamide (25 mL) was subjected to nitrogen displacement three times and then stirred at 25 °C for 1 hour under nitrogen production. Water (50 mL) was added to the reaction mixture, which was then extracted with ethyl acetate (80 mL * 2). The combined organic phases were washed with saturated brine (100 mL), dried over anhydrous sodium sulfate, filtered, and concentrated. The resulting crude product was purified by preparative HPLC (column: Phenomenex luna C18 150 * 40 mm * 15 μm; mobile phase: [water (0.1% formic acid)-acetonitrile]; % acetonitrile: 14%-44%, 15 min) to give Compound **2-7.** $^1$H-NMR (400 MHz, $CDCl_3$) δ ppm 7.30 - 7.46 (m, 10 H) 5.59 - 5.92 (m, 2 H) 4.97 - 5.27 (m, 4 H) 3.79 - 3.92 (m, 1 H) 3.67 - 3.78 (m, 3 H) 3.36 - 3.65 (m, 4 H) 3.09 - 3.32 (m, 4 H) 2.79 - 3.04 (m, 4 H).

Step J: A solution of Compound **2-7** (2.2 g, 4.06 mmol), Compound **2-8** (1.50 g, 6.09 mmol), O-benzotriazole-N,N,N',N'-tetramethylurea tetrafluoroborate (2.22 g, 6.90 mmol), and triethylamine (1.23 g, 12.18 mmol) in N,N-dimethylformamide (20 mL) was subjected to nitrogen displacement three times and then stirred at 25 °C for 1 hour under nitrogen protection. Water (80 mL) was added to the reaction mixture, which was then extracted with ethyl acetate (80 mL * 2). The combined organic phases were washed with saturated brine (100 mL), dried over anhydrous sodium sulfate, and then concentrated and filtered to give the crude product. The crude product was isolated by silica gel column chromatography (eluent: petroleum ether/ethyl acetate, gradient elution) to give Compound **2-9.** $^1$H NMR (400 MHz, $CDCl_3$) δ ppm 7.29 - 7.56 (m, 15 H) 5.72 (br s, 2H) 4.93 - 5.32 (m, 6H) 4.00 - 4.07 (m, 1H) 3.82 - 3.99 (m, 2H) 3.76 (s, 3H) 3.56 - 3.70 (m, 2H) 3.06 - 3.54 (m, 8H) 2.98 (s, 1H).

Step K: Lithium hydroxide monohydrate (504.37 mg, 12.02 mmol) was added to a solution of Compound **2-9** (2.5 g, 3.43 mmol) in a mixture of methanol (22 mL) and water (7 mL). The reaction mixture was stirred at 25 °C for 12 hours. Water (50 mL) was added to the reaction mixture. The aqueous phase was adjusted to pH 4-5 with 2M hydrochloric acid, and extracted with ethyl acetate (100 mL * 2). The combined organic phases were washed with saturated brine (50 mL). After drying over anhydrous sodium sulfate, the organic phase was filtered, and the filtrate was concentrated to give Compound **2-10.**

Step L: A solution of Compound **2-10** (500 mg, 710.49 μmol), Compound **2-11** (406.18 mg, 781.54 μmol), benzotriazole-N, N, N', N'-tetramethylurea hexafluorophosphate (485.01 mg, 1.28 mmol) and triethylamine (215.68 mg, 2.13 mmol) in N, N-dimethylformamide (5 mL) was subjected to nitrogen displacement three times, and then stirred at 25 ° C for 1 hour under nitrogen atmosphere. Water (80 mL) was added to the reaction mixture, which was then extracted with

dichloromethane (80 mL * 2). The combined organic phases were washed with saturated brine (100 mL * 1), dried over anhydrous sodium sulfate, and filtered. The filtrate was concentrated and then purified by preparative HPLC (column: Waters Xbridge C18 150 * 50 mm * 10 μm; mobile phase: [water (0.1% ammonium bicarbonate)-acetonitrile]; % acetonitrile: 57%-87%, 10 minutes) to give Compound **2-12.** $^1$H NMR (400 MHz, DMSO-d6) δ ppm 7.21 - 7.38 (m, 24 H) 6.79 - 6.95 (m, 4 H) 4.74 - 5.19 (m, 6 H) 4.35 (t, J=4.83 Hz, 1 H) 3.59 - 4.07 (m, 13 H) 3.39 - 3.55 (m, 6 H) 2.87 - 3.23 (m, 10 H) 2.58 - 2.78 (m, 6 H) 1.04 - 1.28 (m, 14 H).

Step M: The palladium on carbon (100 mg, 10% content) was added to a solution (10 mL) of Compound **2-12** (320 mg, 203.93 μmol) in methanol, and then the system was subjected to argon displacement three times, followed by hydrogen displacement three times. The reaction mixture was stirred at 25 °C for 16 hours under a hydrogen atmosphere at ordinary pressure. The reaction mixture was filtered, and then the filtrate was concentrated under reduced pressure to give Compound **2-13.** $^1$H NMR (400 MHz, DMSO-d6) δ ppm 8.02 - 8.28 (m, 1H) 7.34 - 7.43 (m, 2H) 7.27 - 7.33 (m, 2H) 7.18 - 7.27 (m, 5H) 6.64 - 7.00 (m, 4H) 3.86 - 4.21 (m, 3H) 2.82 - 3.56 (m, 24H) 1.55 - 1.69 (m, 2H) 1.37 - 1.45 (m, 2H) 1.05 - 1.29 (m, 14H).

Step N: A solution of Compound **2-13** (178 mg, 221.66 μmol), Compound **2-14** (413.14 mg, 775.80 μmol), benzotriazole-N,N,N',N'-tetramethylurea hexafluorophosphate (336.25 mg, 886.63 μmol), and triethylamine (179.43 mg, 1.77 mmol) in N,N-dimethylformamide (4 mL) was subjected to nitrogen displacement three times, and the reaction mixture was stirred at 25 °C for 1 hour under nitrogen atmosphere. Water (100 mL) was added to the reaction mixture, which was then extracted twice with 50 mL DCM/i-PrOH (v/v = 3/1). The combined organic phases were washed with saturated brine (150 mL * 1), dried over anhydrous sodium sulfate and filtered. The filtrate was concentrated under reduced pressure and then purified by preparative HPLC (column: Waters Xbridge C18 150 * 50 mm * 10 μm; mobile phase: [water (0.1% ammonium bicarbonate)-acetonitrile]; % acetonitrile: 36%-66%, 10 minutes) to give Compound **2-15.** $^1$H-NMR (400 MHz, DMSO-d6) δ ppm 8.10 - 8.23 (m, 1H) 7.92 - 8.01 (m, 1H) 7.66 - 7.91 (m, 8H) 7.35 - 7.40 (m, 2H) 7.30 (t, J=7.64 Hz, 2H) 7.21 - 7.27 (m, 5H) 6.88 (d, J=8.80 Hz, 4H) 5.22 (d, J=3.30 Hz, 3H) 4.97 (dd, J=11.25, 3.30 Hz, 3H) 4.49 (d, J=8.56 Hz, 3H) 4.36 (t, J=4.89 Hz, 1H) 3.94 - 4.10 (m, 12H) 3.81 - 3.92 (m, 5H) 3.64 - 3.80 (m, 12H) 3.37 - 3.49 (m, 6 H) 2.84 - 3.23 (m, 17 H) 2.53 (br d, J=1.96 Hz, 5 H) 2.08 - 2.16 (m, 11 H) 2.00 (s, 12 H) 1.89 (s, 9 H) 1.78 (s, 9 H) 1.54 - 1.67 (m, 7 H) 1.38 - 1.53 (m, 14 H) 1.01 - 1.32 (m, 16 H) .

Step O: A solution of Compound **2-15** (240 mg, 97.49 μmol), Compound **2-16** (34.15 mg, 341.21 μmol), 4-dimethylaminopyridine (11.91 mg, 97.49 μmol), triethylamine (9.86 mg, 97.49 μmol) in dichloromethane (5 mL) was subjected to nitrogen displacement three times and stirred at 25 °C for 16 hours under nitrogen protection. The reaction mixture was concentrated and then purified by preparative HPLC (column: Waters Xbridge C18 150 * 50 mm * 10 μm; mobile phase: [water (0.1% ammonium bicarbonate)-acetonitrile]; % acetonitrile: 19%-49%, 10 minutes) to give Compound **D08-M.** $^1$H NMR (400 MHz, DMSO-d6) δ ppm 7.65 - 8.20 (m, 11 H) 7.28 - 7.39 (m, 4 H) 7.17 - 7.28 (m, 5 H) 6.78 - 7.03 (m, 4 H) 5.13 - 5.33 (m, 3 H) 4.89 - 5.10 (m, 3 H) 4.39 - 4.60 (m, 3 H) 3.94 - 4.15 (m, 15 H) 3.82 - 3.92 (m, 5 H) 3.64 - 3.81 (m, 12 H) 2.83 - 3.19 (m, 16 H) 2.08 - 2.19 (m, 14 H) 1.96 - 2.07 (m, 19 H) 1.86 - 1.92 (m, 9 H) 1.74 - 1.81 (m, 10 H) 1.54 - 1.65 (m, 7 H) 1.34 - 1.54 (m, 16 H) 1.03 - 1.33 (m, 16 H) ; LCMS (ESI): 1221.8.

**Example 4: Synthesis of D10-M**

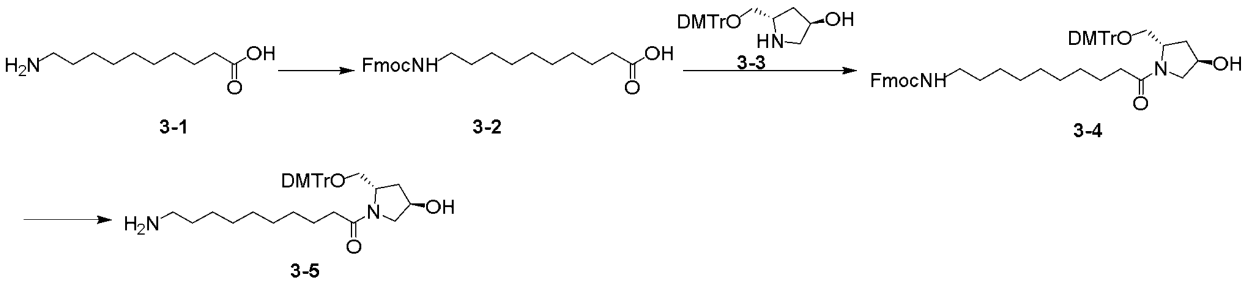


3-1 3-2 3-4 3-5

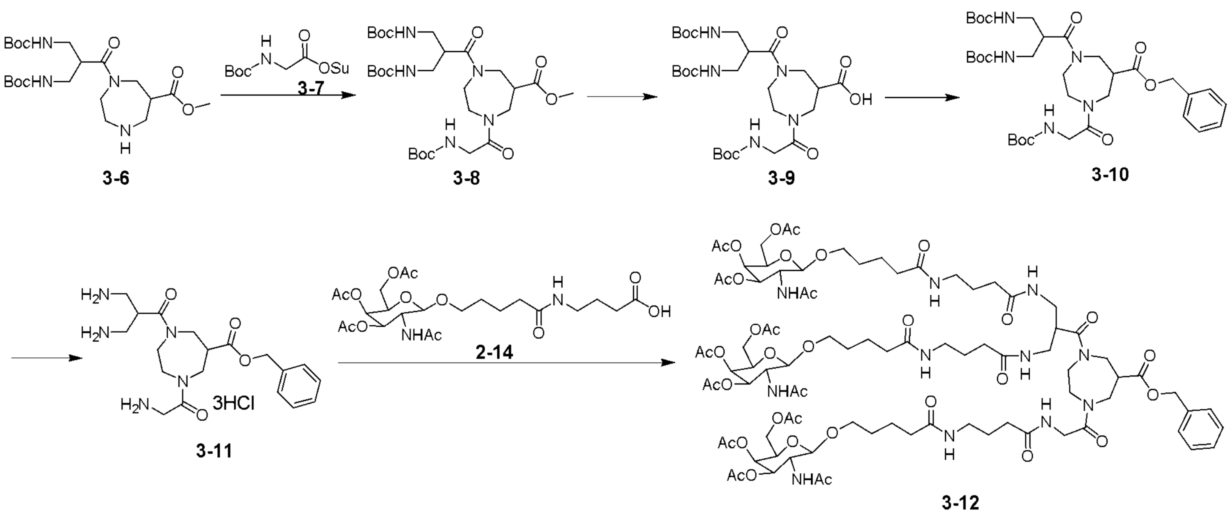


3-6 3-8 3-9 3-10 3-11 3-12

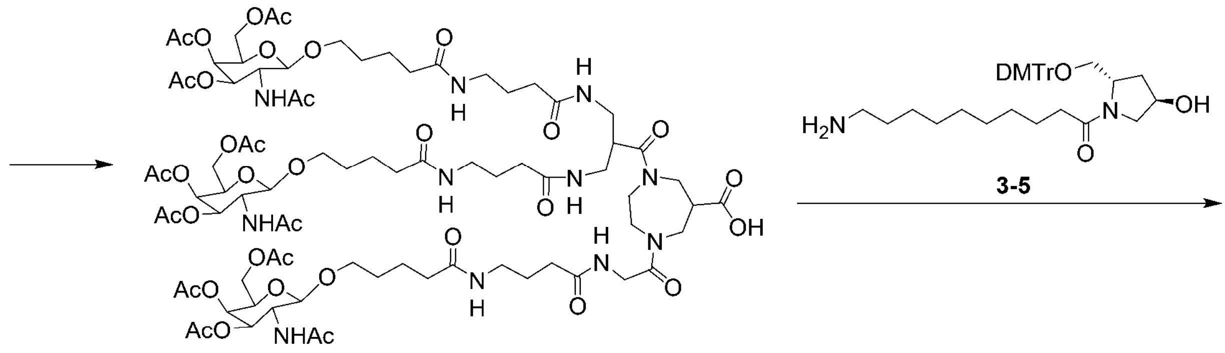


3-13

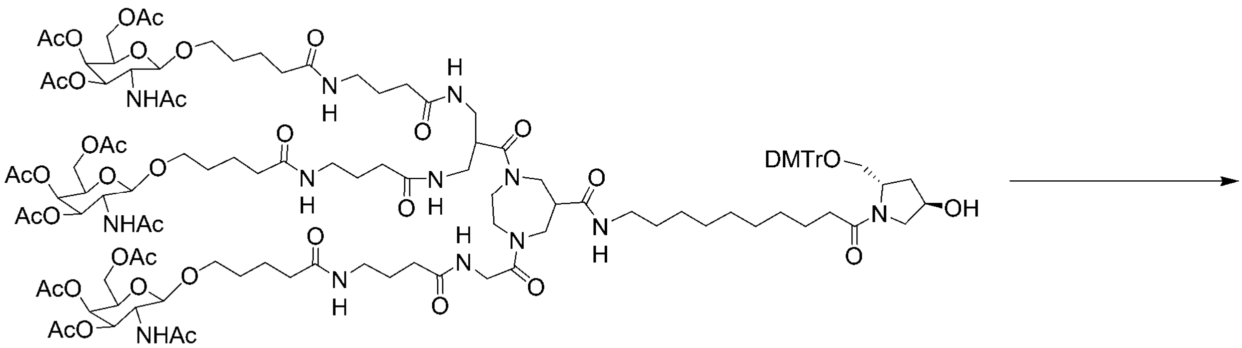

3-14

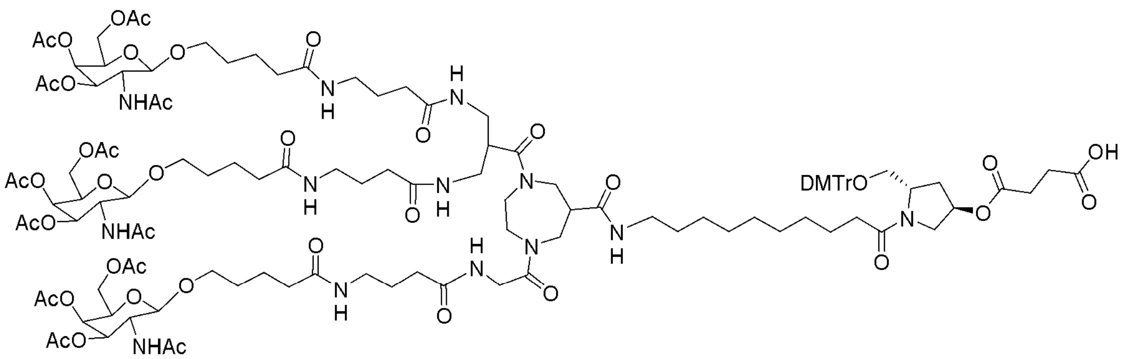

D10-M

Step A: Compound **3-1** (2 g, 10.68 mmol) was dissolved in a mixed solution of acetonitrile (20 mL) and water (20 mL), to which sodium bicarbonate (1.79 g, 21.36 mmol, 831.06 μL) and N-(9-fluorenemethoxycarbonyloxy) succinimide (4.32 g, 12.82 mmol) were added successively. The reaction mixture was reacted at 25 °C for 12 hours. The reaction mixture was diluted with 1 M aqueous hydrochloric acid solution (200 mL), extracted with ethyl acetate (100 mL * 2), dried over anhydrous sodium sulfate, filtered, and concentrated under reduced pressure to obtain a crude product. The crude product was triturated with a mixture of petroleum ether (10 mL) and ethyl acetate (10 mL) for 12 hours to give Compound **3-2.**

Step B: Compound **3-2** (3.2 g, 7.81 mmol) was dissolved in dichloromethane (50 mL), to which N,N-diisopropylethylamine (23.44 mmol, 4.08 mL), 1-hydroxy-7-azabenzotriazole (1.60 g, 11.72 mmol), 1-(3-dimethylaminopropyl)-3-ethylcarbodiimide hydrochloride (2.25 g, 11.72 mmol) and Compound **3-3** (3.11 g, 7.42 mmol) were added successively. The reaction mixture was reacted at 25 °C for 12 hours. The reaction mixture was diluted with water (200 mL), extracted with dichloromethane (100 mL * 2), dried over anhydrous sodium sulfate, filtered, and concentrated under reduced pressure to obtain a crude product. The crude product was purified by silica gel column chromatography to give Compound **3-4.**

Step C: Compound **3-4** (3.8 g, 4.69 mmol) and diethylamine (13.71 g, 187.42 mmol) were dissolved in dichloromethane (30 mL). The reaction mixture was reacted at 25 °C for 12 hours. The reaction mixture was concentrated under reduced pressure to give Compound **3-5.**

Step D: Compound **3-6** (37.7 g, 68.72 mmol, oxalate salt), Compound **3-7** (22.45 g, 82.47 mmol), and triethylamine (240.53 mmol, 33.48 mL) were dissolved in acetonitrile (400 mL), and the system was subjected to nitrogen displacement three times. The mixture was stirred at 25 °C for 3.5 hours. The mixture was diluted with methyl tert-butyl ether (400 mL), washed with saturated aqueous sodium bicarbonate (400 mL * 2), brine (400 mL), saturated ammonium chloride (400 mL * 2), and brine (400 mL) successively. The resulting organic phase was dried over anhydrous sodium sulfate, filtered, and concentrated under reduced pressure to give Compound **3-8.**

Step E: Compound **3-8** (42.31 g, 68.72 mmol) was dissolved in methanol (200 mL) and water (50 mL), to which sodium hydroxide (6.87 g, 171.79 mmol) was then added at 5 °C. The mixture was stirred at 25 °C for 2 hours. The mixture was concentrated to remove most of the methanol, diluted with water (100 mL), and the solution was adjusted to pH=4-5 with 1 M hydrochloric acid and extracted with dichloromethane (300 mL * 2) at 5 °C. The resulting organic phase was dried over anhydrous sodium sulfate, filtered, and concentrated under reduced pressure to give Compound **3-9.**

Step F: Compound **3-9** (5 g, 8.31 mmol) was dissolved in dichloromethane (50 mL), to which 1, 8-diazabicyclo [5.4.0] undec-7-ene (9.97 mmol, 1.50 mL) and benzyl bromide (9.97 mmol, 1.18 mL) were added successively. The reaction mixture was reacted at 25 °C for 12 hours. The reaction mixture was concentrated under reduced pressure to remove dichloromethane. Water (100 mL) was added to the mixture, which was extracted with ethyl acetate (100 mL * 2). The extract was dried over anhydrous sodium sulfate, filtered, and concentrated under reduced pressure to obtain a crude product. The crude product was purified by preparative HPLC (column: Kromasil Eternity XT 250*80 mm*10 μm; mobile phase: [water (0.1% ammonium bicarbonate)-acetonitrile]; gradient: 43%-73% acetonitrile, 20 minutes) to give Compound **3-10.**

Step G: Hydrochloric acid in ethyl acetate (2 M, 92.50 mL) was added to Compound 3-10 (3.7 g, 5.35 mmol). The reaction mixture was reacted at 25 °C for 12 hours. The reaction mixture was concentrated under reduced pressure to give Compound **3-11.**

Step H: Compound **2-14** (9.47 g, 17.79 mmol) was dissolved in dichloromethane (60 mL), to which N,N-diisopropylethylamine (48.52 mmol, 8.45 mL), O-(7-azabenzotriazol-1-yl)-N,N,N,N-tetramethylurea hexafluorophosphate (8.20 g, 21.56 mmol) and Compound **3-11** (2.7 g, 5.39 mmol) were added successively. The reaction mixture was reacted at 25 °C for 12 hours. The reaction mixture was concentrated under reduced pressure to give a crude product, and the crude product was purified by preparative HPLC (column: Kromasil Eternity XT 250* 80 mm*10 μm; mobile phase: [water (0.1% ammonium bicarbonate)-acetonitrile]; gradient: 20%-50% acetonitrile, 20 minutes) to give Compound **3-12.**

Step I: Compound **3-12** (5.2 g, 2.69 mmol) was dissolved in methanol (50 mL), and Pd/C (1 g, 939.67 μmol, 10% palladium content) was added to the reaction mixture under the protection of a nitrogen atmosphere. The system was subjected to hydrogen displacement three times, and reacted under a hydrogen atmosphere (15 Psi) at 25 °C for 12 hours. The palladium on carbon was removed by filtration, and the filtrate was concentrated under reduced pressure to give Compound **3-13.**

Step J: Compound **3-13** (1 g, 542.03 μmol) was dissolved in dichloromethane (10 mL), to which N,N-diisopropylethylamine (1.63 mmol, 283.24 μL), 1-hydroxy-7-azabenzotriazole (110.67 mg, 813.05 μmol), 1-(3-dimethylaminopropyl)-3-ethylcarbodiimide hydrochloride (155.86 mg, 813.05 μmol) and Compound **3-5** (462.52 mg, 542.03 μmol) were added successively. The reaction mixture was reacted at 25 °C for 12 hours. The reaction mixture was concentrated under reduced pressure to give a crude product, and the crude product was purified by preparative HPLC (column: Waters Xbridge C18 150 * 50 mm * 10 μm; mobile phase: [water (0.1% ammonium bicarbonate)-acetonitrile]; gradient: 31%-61% acetonitrile; 10 minutes) to give Compound **3-14.**

Step K: Compound **3-14** (0.47 g, 194.56 μmol) was dissolved in dichloromethane (5 mL), to which triethylamine (1.07 mmol, 148.94 μL), 4-dimethylaminopyridine (23.77 mg, 194.56 μmol), and butanedioic anhydride (97.35 mg, 972.82 μmol) were added successively. The reaction mixture was reacted at 25 °C for 12 hours, and the reaction was detected to be complete. The reaction mixture was concentrated under reduced pressure to give a crude product, and the crude product was purified by preparative HPLC (column: Waters Xbridge C18 150*50 mm*10 μm; mobile phase: [water (0.1% ammonium bicarbonate)-acetonitrile]; gradient: 22%-52% acetonitrile; 10 minutes) to give Compound **D10-M.** $^1$H NMR (400 MHz, DMSO-d6) δ = 8.46 - 7.60 (m, 9H), 7.38 - 7.09 (m, 9H), 6.93 - 6.80 (m, 4H), 5.21 (d, J = 3.3 Hz, 3H), 4.97 (dd, J = 3.4, 11.1 Hz, 3H), 4.48 (d, J = 8.4 Hz, 3H), 4.26 - 4.15 (m, 1H), 4.08 - 3.93 (m, 10H), 3.92 - 3.78 (m, 5H), 3.78 - 3.63 (m, 11H), 3.57 - 3.39 (m, 6H), 3.16 - 2.86 (m, 18H), 2.40 - 2.19 (m, 6H), 2.17 - 2.08 (m, 13H), 2.07 - 2.01 (m, 8H), 1.99 (s, 10H), 1.89 (s, 9H), 1.77 (s, 9H), 1.65 - 1.33 (m, 22H), 1.33 - 1.08 (m, 11H); LCMS (ESI) m/z: 1256.8.

**Example 5: Synthesis of D11-M**

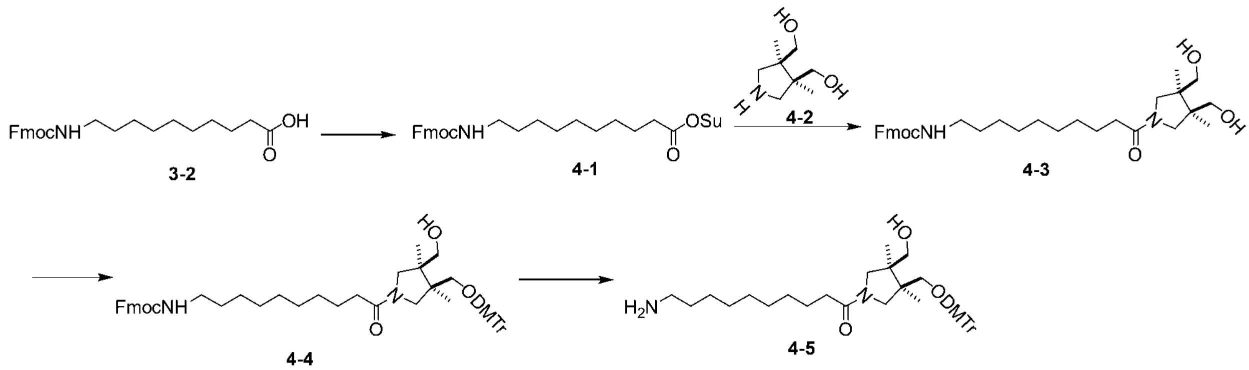


3-2 4-1 4-3 4-4 4-5

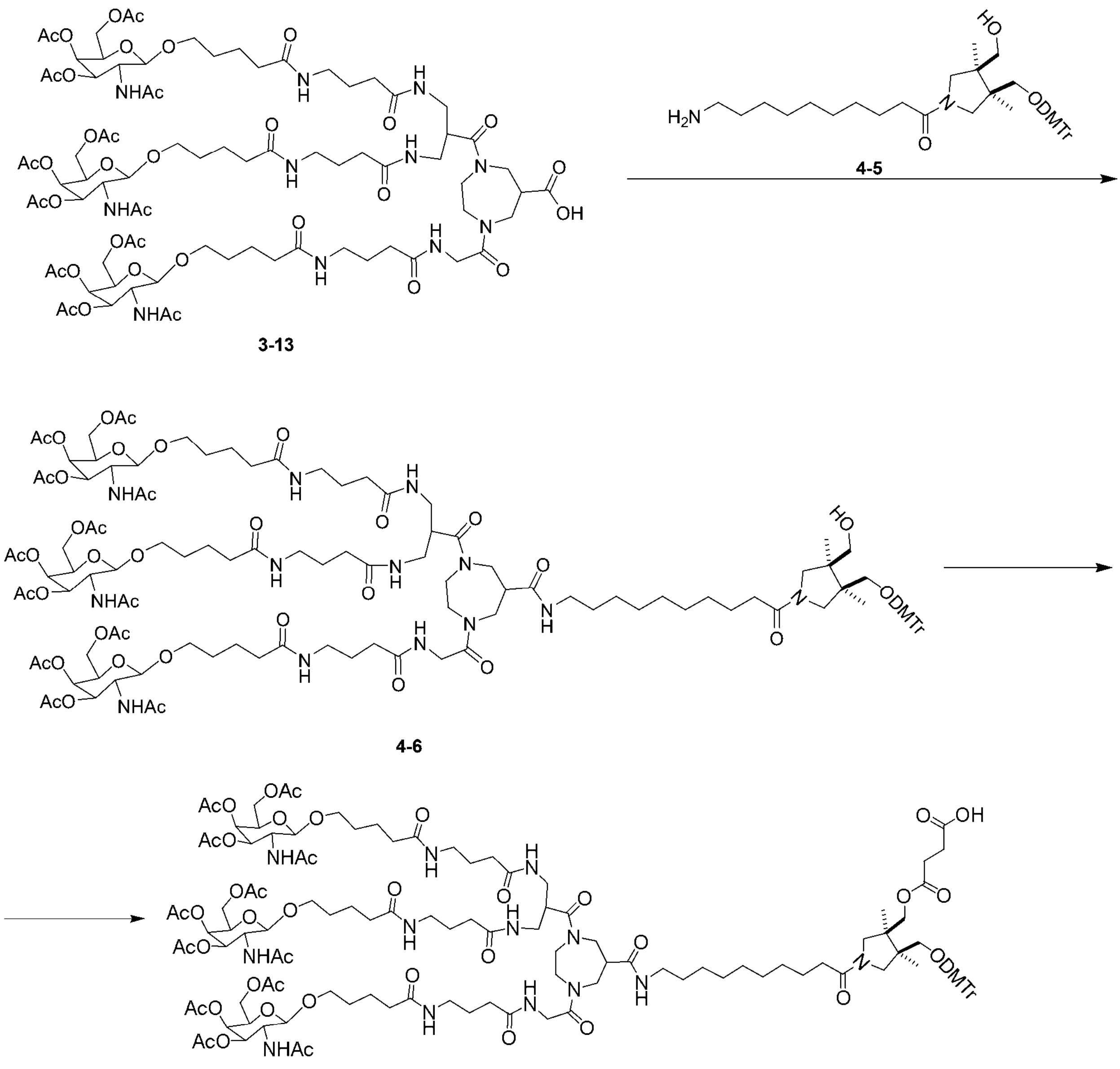


3-13

4-6

D11-M

Step A: Compound **3-2** (3 g, 7.33 mmol) was dissolved in tetrahydrofuran (30 mL), to which N,N-diisopropylethylamine (1.14 g, 8.79 mmol, 1.53 mL) and 2-succinimido-1,1,3,3-tetramethylurea tetrafluoroborate (2.43 g, 8.06 mmol) were added successively. The reaction mixture was reacted at 25 °C for 12 hours. The reaction mixture was diluted with water (100 mL) and filtered to obtain a crude product. The crude product was purified by silica gel column chromatography (eluent: petroleum ether: ethyl acetate = 2:1) to give Compound **4-1.**

Step B: Compound **4-1** (2.86 g, 5.65 mmol) was dissolved in dichloromethane (30 mL), to which Compound **4-2** (1.44 g, 9.03 mmol) was added. The reaction mixture was reacted at 25 °C for 12 hours. The reaction mixture was diluted with water (200 mL), extracted with dichloromethane (100 mL * 2), dried over anhydrous sodium sulfate, filtered, and concentrated under reduced pressure to obtain a crude product. The crude product was purified by preparative HPLC (column: Waters Xbridge C18 150*50 mm*10 μm; mobile phase: [water (0.1% ammonium bicarbonate)-acetonitrile]; gradient: 40%-70% acetonitrile, 10 minutes) to give Compound **4-3.**

Step C: Compound **4-3** (2 g, 3.63 mmol) was dissolved in dichloromethane (20 mL), to which 4A molecular sieve (2 g), triethylenediamine (488.84 mg, 4.36 mmol, 479.25 μL), and 4,4-dimethoxytrityl chloride (1.35 g, 3.99 mmol) were added successively. The reaction mixture was reacted at 25 °C for 16 hours. The reaction mixture was diluted with water (200 mL), extracted with dichloromethane (100 mL * 2), dried over anhydrous sodium sulfate, filtered, and concentrated under reduced pressure to obtain a crude product, and the crude product was purified by preparative HPLC (column: Kromasil Eternity XT 250*80 mm* 10 μm; mobile phase: [water (ammonia)-acetonitrile]; gradient: 60%-90% acetonitrile, 25 minutes) to give Compound **4-4.**

Step D: Compound **4-4** (1.78 g, 2.09 mmol) and diethylamine (7.90 g, 108.00 mmol) were dissolved in dichloromethane (15 mL). The reaction mixture was reacted at 25 °C for 12 hours, and the reaction was detected to be complete. Concentrating under reduced pressure to obtain Compound **4-5.** LCMS (M+1, m/z:632)

Step E: Compound **3-13** (1.5 g, 813.05 μmol) was dissolved in dichloromethane (15 mL), to which N,N-diisopropylethylamine (315.24 mg, 2.44 mmol), 1-hydroxy-7-azabenzotriazole (166.00 mg, 1.22 mmol), and 1-(3-dimethylaminopropyl)-3-ethylcarbodiimide hydrochloride (233.79 mg, 1.22 mmol) were added successively. After blended homogeneously, Compound **4-5** (854.86 mg, 813.05 μmol) was added. The reaction mixture was reacted at 25 °C for 12 hours, and the reaction was detected to be complete. The reaction mixture was concentrated under reduced pressure to give a crude product, and the crude product was purified by preparative HPLC (column: Waters Xbridge C18 150*50 mm*10 μm; mobile phase: [water (0.1% ammonium bicarbonate)-acetonitrile]; gradient: 34%-64% acetonitrile; 10 minutes) to give Compound **4-6.**

Step F: Compound **4-6** (450 mg, 183.09 μmol) was dissolved in dichloromethane (3 mL), to which triethylamine (166.74 mg, 1.65 mmol), 4-dimethylaminopyridine (22.37 mg, 183.09 μmol), and butanedioic anhydride (146.58 mg, 1.46 mmol) were added successively. The reaction mixture was reacted at 35°C for 24 hours under nitrogen protection. The reaction mixture was concentrated under reduced pressure to give a crude product, and the crude product was purified by preparative HPLC (column: Waters Xbridge C18 150*50 mm*10 μm; mobile phase: [water (0.1% ammonium bicarbonate)-acetonitrile]; gradient: 23%-53% acetonitrile; 10 minutes) to give Compound **D11-M.** $^{1}H$ NMR (400 MHz, DMSO-d6) δ = 8.47 - 7.70 (m, 10H), 7.41 - 7.15 (m, 9H), 6.87 (br d, J = 7.1 Hz, 4H), 5.21 (d, J = 3.2 Hz, 3H), 4.97 (br dd, J = 2.6, 11.2 Hz, 3H), 4.49 (br d, J = 8.3 Hz, 3H), 4.18 - 3.91 (m, 12H), 3.91 - 3.79 (m, 5H), 3.76 - 3.61 (m, 14H), 3.22 - 2.82 (m, 27H), 2.10 (s, 11H), 1.99 (s, 18H), 1.88 (s, 9H), 1.77 (s, 8H), 1.66 - 1.30 (m, 22H), 1.23 (br d, J = 6.2 Hz, 10H), 1.11 - 1.02 (m, 3H), 0.97 (br d, J = 2.8 Hz, 3H); LCMS (ESI) m/z: 1278.2.

**Example 6: Synthesis of D18-M**

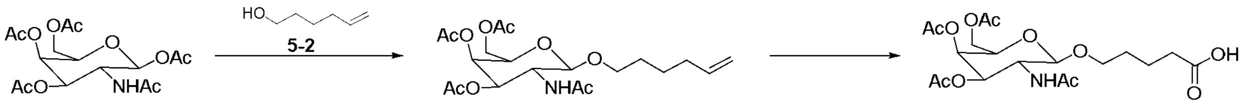


5-1 5-3 5-4

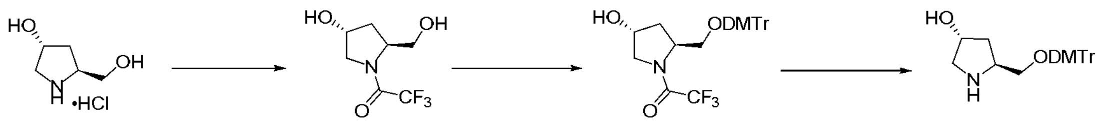

5-5 5-6 5-7 3-3

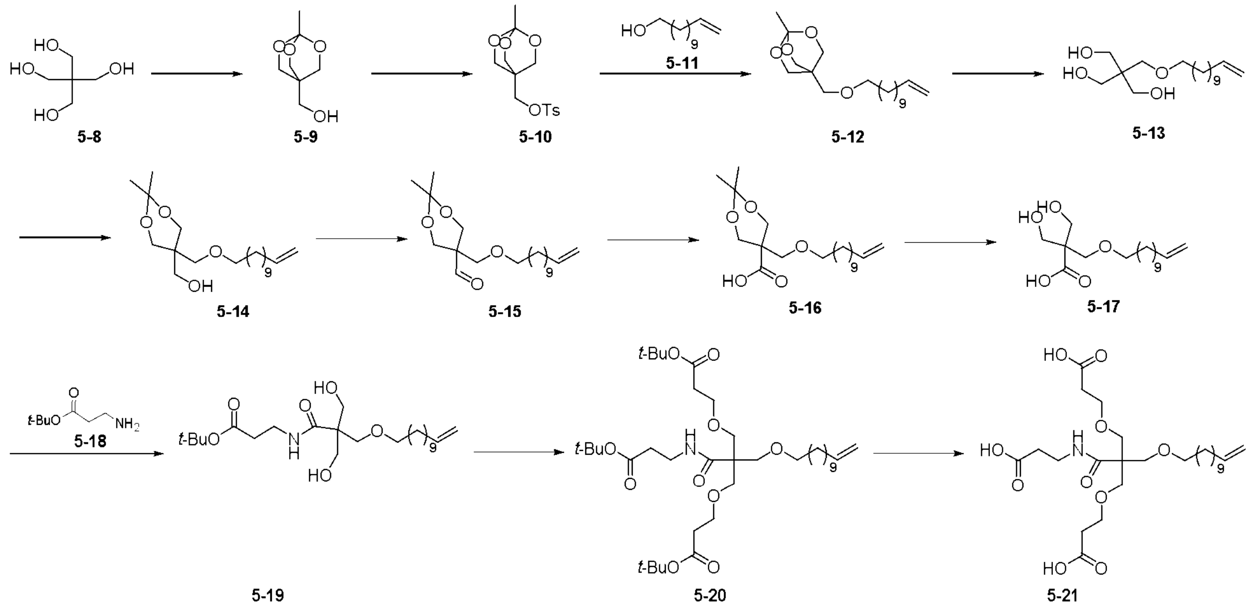


5-8 5-9 5-10 5-12 5-13

5-14 5-15 5-16 5-17

5-19 5-20 5-21

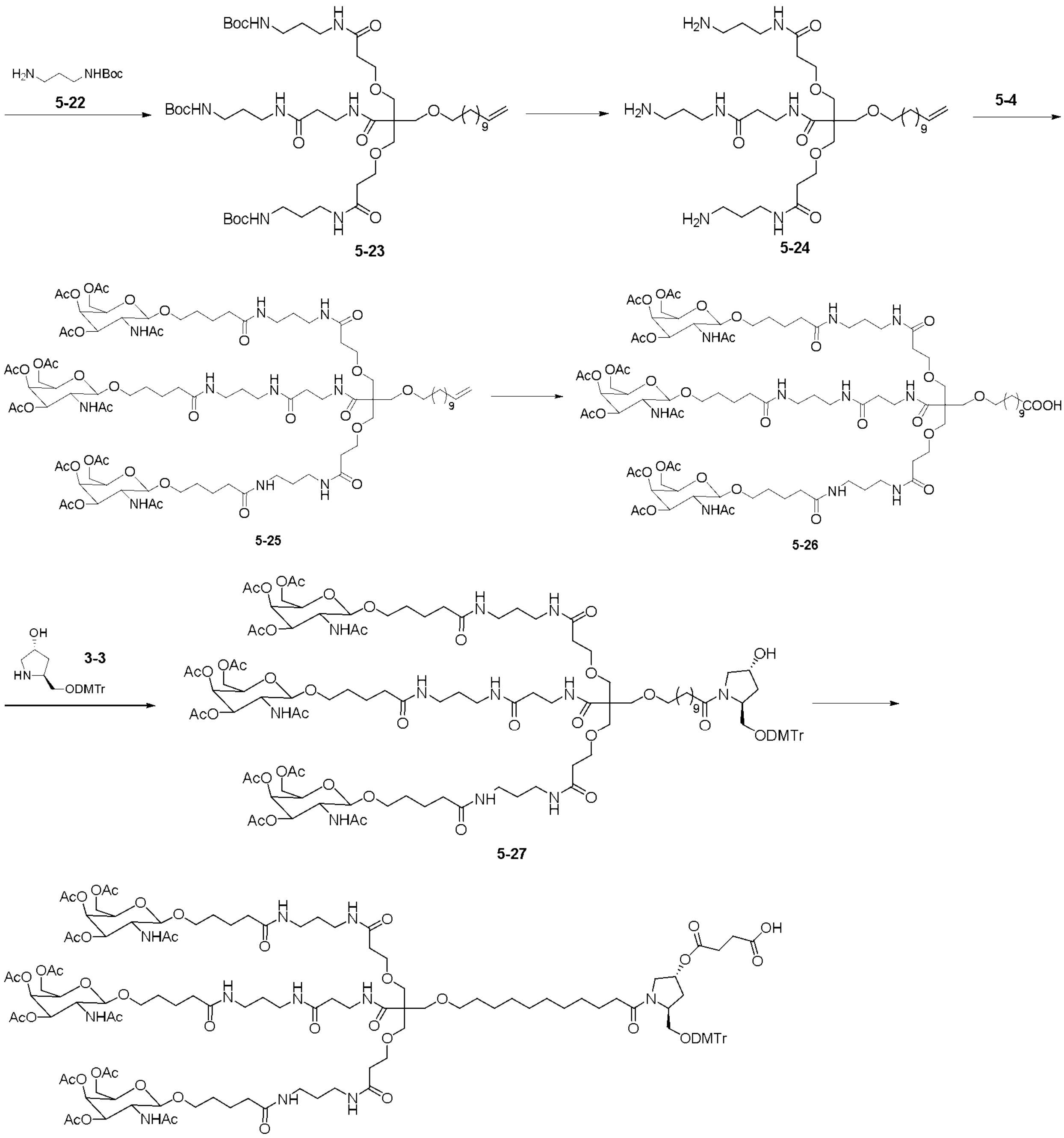


5-23 5-24 5-25 5-26 5-27

D18-M

Step A: To a solution of Compound **5-1** (10 g, 25.68 mmol) in 1,2-dichloroethane (100 mL) were added scandium trifluoromethanesulfonate (348.84 mg, 1.8 mmol) and Compound **5-2** (2.83 g, 28.25 mmol). The reaction was stirred at 60 °C for 6 hours. The reaction was detected to be complete. The reaction mixture was quenched by adding water (200 mL) at 25 °C, and extracted. The resulting organic phase was washed with 5% sodium bicarbonate (200 mL), dried over anhydrous sodium sulfate, filtered, and concentrated under reduced pressure to obtain a crude product. The crude product was recrystallized from a mixture of ethyl acetate/n-heptane (volume ratio: 1/3, 80 mL) to give Compound **5-3.**

Step B: To a mixture of Compound **5-3** (8 g, 18.63 mmol) and ruthenium trichloride trihydrate (82.8 mg, 316.68 μmol) in dichloromethane (24 mL), acetonitrile (24 mL) and water (40 mL) was added sodium periodate (19.92 g, 93.14 mmol). The reaction was stirred at 25 °C for 2 hours. The reaction was detected to be complete. The reaction mixture was filtered, and the resulting filtrate was adjusted to pH 8 with saturated sodium bicarbonate, and then extracted with dichloromethane (50 mL * 2). The aqueous phase was retained, adjusted to pH 3 with citric acid, and extracted with a mixture of dichloromethane/isopropanol (volume ratio: 3/1, 50 mL * 3). The combined organic phases were washed with saturated brine and 10% sodium bisulfite, dried over anhydrous sodium sulfate, filtered, and concentrated under reduced pressure to obtain a crude product. The crude product was recrystallized from a mixture of ethyl acetate (32 mL) and n-heptane (24 mL) to give Compound **5-4.**

Step C: To a solution of Compound **5-5** (150 g, 976.52 mmol) in acetonitrile (1500 mL) were added triethylamine (242.09 g, 2.39 mol) and trifluoroacetic anhydride (332.98 g, 2.34 mol). The reaction was stirred at 25 °C for 12 hours. The reaction was detected to be complete. The reaction mixture was filtered, and the resulting filter cake was rinsed with ethyl acetate (200 mL * 2). The mother liquor was concentrated under reduced pressure to obtain an oil. The oil was triturated with ethyl acetate (1500 mL) at 20 °C for 1 hour and filtered, and the resulting filter cake was rinsed with ethyl acetate (200 mL * 2). The mother liquor was concentrated under reduced pressure to give Compound **5-6.**

Step D: To a solution of Compound **5-6** (6 g, 28.15 mmol) in dichloromethane (40 mL) were added pyridine (22.27 g, 281.49 mmol) and 4,4-dimethoxytrityl chloride (9.54 g, 28.15 mmol) at 0 °C. The reaction was stirred at 0-25 °C for 12 hours. The reaction was detected to be complete. The reaction mixture was diluted by adding water (100 mL), and extracted with dichloromethane (100 mL). The resulting organic phase was dried over anhydrous sodium sulfate, filtered, and concentrated to obtain a crude product. The crude product was purified by silica gel column chromatography (eluted with ethyl acetate/petroleum ether from 15/1 to 5/1, with 1% triethylamine added to the eluent) to give Compound **5-7.**

Step E: To a solution of Compound **5-7** (2 g, 3.88 mmol) in methanol were added potassium hydroxide (435.33 mg, 7.76 mmol) and water (5 mL). The reaction was stirred at 25 °C for 1 hour. The reaction was detected to be complete. The reaction mixture was concentrated under reduced pressure to remove methanol, followed by the addition of water (80 mL), and then extracted with dichloromethane (50 mL * 2). The combined organic phases were dried over anhydrous sodium sulfate, filtered, and concentrated under reduced pressure to obtain Compound **3-3.**

Step F: Under nitrogen protection and at room temperature, a water separator apparatus was set up, in which. Compound **5-8** (100.85 g, 740.75 mmol), trimethyl orthoacetate (89.00 g, 740.75 mmol), and p-toluenesulfonic acid monohydrate (7.05 g, 37.04 mmol) were mixed in toluene (500 mL). The reaction mixture was heated to 110 °C and stirred for 20 hours. The reaction was detected to be complete. The reaction mixture was concentrated under reduced pressure to remove toluene, followed by the addition of the triethylamine (10 mL), and then concentrated to dryness under reduced pressure to give Compound **5-9.**

Step G: Under nitrogen protection, in dichloromethane (250 mL), Compound **5-9** (25 g, 156.09 mmol), 4-dimethylaminopyridine (1.91 g, 15.61 mmol), and p-toluenesulfonyl chloride (32.73 g, 171.70 mmol) were added. The triethylamine (23.69 g, 234.13 mmol) was then added at a controlled temperature of 10-25 °C, and the reaction was stirred at 25 °C for 16 hours. The reaction was detected to be complete. The reaction mixture was diluted with water (500 mL) and extracted with dichloromethane (100 mL * 2). The combined organic phases were washed with saturated brine (200 mL * 2), dried over anhydrous sodium sulfate, filtered, and concentrated under reduced pressure to obtain a crude product. Subsequently, the crude product was purified by reverse phase column chromatography (neutral conditions, column type: Kromasil Eternity XT 250 * 80 mm * 10 μm; mobile phase: [water (0.1% ammonium bicarbonate)-acetonitrile]; gradient: 25%-55%, 20 minutes) to give Compound **5-10.**

Step H: Under nitrogen protection, in tetrahydrofuran (500 mL), Compound **5-10** (50 g, 159.06 mmol), Compound **5-11** (32.25 g, 174.96 mmol), and potassium hydroxide (35.70 g, 636.23 mmol) were added. The system was heated to 50 °C for the reaction for 16 hours. The reaction was detected to be complete. The reaction mixture was diluted with water (500 mL) and extracted with methyl tert-butyl ether (500 mL * 2). The combined organic phases were washed with saturated brine (200 mL * 2), dried over anhydrous sodium sulfate, filtered, and concentrated under reduced pressure to obtain a crude product. The crude product was purified by silica gel column chromatography (eluted with ethyl acetate/petroleum ether from 1/0 to 10/1, with 0.1% triethylamine added to the eluent) to give Compound **5-12.**

Step I: Under nitrogen protection, Compound **5-12** (28 g, 85.77 mmol) and 37% concentrated hydrochloric acid (6 mL) were added to methanol (300 mL) and water (60 mL), and the reaction was stirred at 25 °C for 2 hours. The reaction was detected to be complete. After concentration under reduced pressure, water and methanol were removed through azeotropic distillation with methanol followed by tetrahydrofuran to give Compound **5-13.**

Step J: Under nitrogen protection, Compound **5-13** (25.94 g, 85.77 mmol), 2,2-dimethoxyacetone (10.72 g, 102.82 mmol) and p-toluenesulfonyl chloride monohydrate (1.63 g, 8.58 mmol) were added to N,N-dimethylformamide (250 mL), and the reaction was stirred at 25 °C for 16 hours. The reaction was detected to be complete. The reaction mixture was quenched by adding saturated sodium bicarbonate (10 mL), diluted with water (10 mL), and extracted with ethyl acetate (50 mL * 2). The combined organic phases were washed with saturated brine (30 mL * 2), dried over anhydrous sodium sulfate, filtered, and concentrated under reduced pressure to obtain a crude product. The crude product was purified by silica gel column chromatography (eluted with ethyl acetate/petroleum ether from 1/0 to 1/1, with 0.1% triethylamine added to the eluent) to give Compound **5-14.**

Step K: Under nitrogen protection, Compound **5-14** (9.8 g, 28.61 mmol) and Dess-Martin periodinane (14.56 g, 34.33 mmol) were added to dichloromethane (98 mL), and the reaction was stirred at 25 °C for 16 hours. The reaction was detected to be complete. The reaction mixture was diluted with saturated sodium bicarbonate (20 mL) and extracted with dichloromethane (50 mL * 2). The combined organic phases were washed with saturated brine (30 mL), dried over anhydrous sodium sulfate, filtered, and concentrated under reduced pressure to obtain a crude product. The crude product was purified by silica gel column chromatography (eluted with ethyl acetate/petroleum ether from 1/0 to 1/5) to give

Compound **5-15.**

Step L: Under nitrogen protection, Compound **5-15** (6.8 g, 19.97 mmol), disodium hydrogen phosphate (4.19 g, 34.95 mmol), 2-methyl-2-butene (9.8 g, 139.80 mmol) and sodium chlorite (8.31 g, 91.87 mmol) were added to tert butyl alcohol (40 mL) and water (10 mL). The reaction was stirred at 25 °C for 16 hours. The reaction was detected to be complete. The reaction mixture was diluted with water (100 mL), extracted with dichloromethane (100 mL * 2), dried over anhydrous sodium sulfate, filtered, and concentrated under reduced pressure to give Compound **5-16.**

Step M: Under nitrogen protection, Compound **5-16** (7.12 g, 19.97 mmol) and 37% concentrated hydrochloric acid (0.7 mL) were added to methanol (35 mL) and water (7 mL), and the reaction was stirred at 25 °C for 16 hours. The reaction was detected to be complete. Methanolwas removed by concentration under reduced pressure, and water and methanol were removed through azeotropic distillation with methanol followed by tetrahydrofuran to give Compound **5-17.**

Step N: Under nitrogen protection, Compound **5-17** (6.32 g, 19.97 mmol), Compound **5-18** (3.19 g, 21.97 mmol), triethylamine (3.03 g, 29.96 mmol) and O-(7-azabenzotriazol-1-yl)-N,N,N,N-tetramethylurea hexafluorophosphate (9.11 g, 23.97 mmol) were added to dichloromethane (60 mL), and the reaction was stirred at 25 °C for 16 hours. The reaction was detected to be complete. The reaction mixture was diluted with water (20 mL) and extracted with dichloromethane (20 mL * 2). The combined organic phases were washed with saturated brine (20 mL * 2), dried over anhydrous sodium sulfate, filtered, and concentrated under reduced pressure to obtain a crude product. The crude product was purified by silica gel column chromatography (eluted with ethyl acetate/petroleum ether from 1/0 to 1/1) to give Compound **5-19.**

Step O: Under nitrogen protection, Compound **5-19** (3 g, 6.76 mmol), tert-butyl acrylate (2.60 g, 20.29 mmol) and potassium hydroxide (37.95 mg, 676.26 μmol) were added to dimethyl sulfoxide (6 mL) and water (0.6 mL), and the reaction was stirred at 25 °C for 16 hours. The reaction was detected to be complete. The reaction mixture was diluted with water (20 mL) and extracted with ethyl acetate (50 mL). The combined organic phases were washed with saturated brine (30 mL), dried over anhydrous sodium sulfate, filtered, and concentrated under reduced pressure to obtain a crude product. The crude product was purified by silica gel column chromatography (eluted with ethyl acetate/petroleum ether from 1/0 to 4/1) to give Compound **5-20.**

Step P: Under nitrogen protection, Compound **5-20** (1.9 g, 2.71 mmol) was added to dioxane hydrochloride (2 M, 20 mL), and the reaction was stirred at 25 °C for 16 hours. The reaction was detected to be complete. The reaction mixture was concentrated under reduced pressure to dryness to give Compound **5-21.**

Step Q: Under nitrogen protection, Compound **5-21** (1 g, 1.88 mmol), Compound **5-22** (1.23 g, 7.05 mmol), triethylamine (1.14 g, 11.29 mmol), 1-(3-dimethylaminopropyl)-3-ethylcarbodiimide hydrochloride (1.35 g, 7.05 mmol) and 1-hydroxy-7-azabenzotriazole (960.09 mg, 7.05 mmol) were added to 2-methyltetrahydrofuran (10 mL), and the reaction was stirred at 25 °C for 16 hours. The reaction was detected to be complete. The reaction mixture was diluted with water (50 mL) and extracted with 2-methyltetrahydrofuran (50 mL * 2). The combined organic phases were washed with 10% citric acid (30 mL * 2), saturated brine (30 mL) and 10% sodium bicarbonate (30 mL) and saturated brine (50 mL * 2), dried over anhydrous sodium sulfate, filtered, and concentrated under reduced pressure to give Compound **5-23.**

Step R: Under nitrogen protection, Compound **5-23** (1.88 g, 1.88 mmol) and p-toluenesulfonic acid monohydrate (2.14 g, 11.28 mmol) were added to 1,2-dichloroethane (9 mL), and the reaction was stirred at 50 °C for 1.5 hours. The reaction was detected to be complete. Compound **5-24** was obtained and used directly for the next reaction.

Step S: Under nitrogen protection, Compound **5-24** (the reaction mixture from the previous step), triethylamine (2.86 g, 28.29 mmol), Compound **5-4** (2.78 g, 6.22 mmol), 1-hydroxy-7-azabenzotriazole (898.38 mg, 6.6 mmol) and 1-(3-dimethylaminopropyl)-3-ethylcarbodiimide hydrochloride (1.27 g, 6.60 mmol) were added to 1,2-dichloroethane (9 mL), and the reaction was stirred at 25 °C for 2 hours. The reaction was detected to be complete. Dichloromethane (50 mL) was added to the reaction mixture, which was then washed with 10% citric acid (50 mL * 2), 10% sodium bicarbonate (50 mL * 2) and saturated brine (50 mL), dried over anhydrous sodium sulfate, filtered, and concentrated under reduced pressure to give Compound **5-25.**

Step T: To a solution of dioxane (10 mL), water (10 mL), Compound **5-25** (3 g, 1.51 mmol) and ruthenium trichloride trihydrate (19.73 mg, 75.44 μmol) was added sodium periodate (1.78 g, 8.30 mmol) slowly at 25 °C, with stirring at 25 °C for 2 hours. The reaction was detected to be complete. The reaction mixture was filtered, rinsed with dichloromethane (50 mL), diluted with water (30 mL), adjusted to pH = 8-9 with saturated sodium bicarbonate, and extracted with dichloromethane (50 mL * 2). The resulting aqueous phase was retained, adjusted to pH = 3-4 with 10% citric acid, and extracted with dichloromethane/isopropanol = 3/1 (50 mL * 3). The combined organic phases were washed with 10% sodium bisulfite (20 mL) and saturated brine (30 mL), dried over anhydrous sodium sulfate, filtered, and concentrated under reduced pressure to give Compound **5-26.**

Step U: Compound **5-26** (1.6 g, 797.53 μmol), Compound **3-3** (351.30 mg, 837.41 μmol), N,N-diisopropylethylamine (309.23 mg, 2.39 mmol), 1-hydroxy-7-azabenzotriazole (54.28 mg, 398.77 μmol) and O-(7-azabenzotriazol-1-yl)-N,N,N,N-tetramethylurea hexafluorophosphate (363.90 mg, 957.04 μmol) were added to dichloromethane (15 mL). The reaction was stirred for 16 hours under nitrogen protection. The reaction was detected to be complete. The reaction mixture was diluted with dichloromethane (50 mL), washed with 10% saturated sodium hydroxide (50 mL * 2) and saturated brine (50 mL), dried over anhydrous sodium sulfate, filtered, and concentrated under reduced pressure to give

Compound **5-27.**

Step V: Under nitrogen protection, Compound **5-27** (1 g, 415.34 μmol), triethylamine (210.14 mg, 2.08 μmol), 4-dimethylpyridine (5.07 mg, 41.53 μmol) and butanedioic anhydride (166.26 mg, 1.66 mmol) were added to dichloromethane (5 mL), and the reaction was stirred at 25 °C for 2 hours. The reaction was detected to be complete. The reaction mixture was concentrated under reduced pressure to give a crude product, which was purified by reversephase column chromatography (neutral conditions; column type: Waters Xbridge C18 150 * 50 mm * 10 μm; mobile phase: [water (0.1% ammonium bicarbonate)-acetonitrile]; gradient: 22%-52%, 10 minutes) to give Compound **D18-M.** $^1$H NMR (400 MHz, DMSO-$d_6$) δ = 12.45 - 12.01 (m, 1H), 7.88 - 7.79 (m, 6H), 7.74 (br t, J = 5.4 Hz, 3H), 7.51 (br t, J = 5.0 Hz, 1H), 7.37 - 7.26 (m, 4H), 7.26 - 7.16 (m, 5H), 6.95 - 6.78 (m, 4H), 5.22 (d, J = 3.4 Hz, 3H), 4.97 (dd, J = 3.4, 11.2 Hz, 3H), 4.49 (d, J = 8.6 Hz, 3H), 4.08 - 3.98 (m, 9H), 3.88 (td, J = 9.0, 10.9 Hz, 3H), 3.74 (s, 6H), 3.72 - 3.67 (m, 3H), 3.53 (br t, J = 6.2 Hz, 4H), 3.47 - 3.44 (m, 4H), 3.43 - 3.39 (m, 4H), 3.27 - 3.19 (m, 3H), 3.03 (br d, J = 5.5 Hz, 13H), 2.49 - 2.44 (m, 3H), 2.32 - 2.18 (m, 9H), 2.10 (s, 9H), 2.08 (s, 7H), 2.04 (br t, J = 7.1 Hz, 6H), 2.00 (s, 9H), 1.89 (s, 9H), 1.78 (s, 9H), 1.56 - 1.38 (m, 23H), 1.23 (br s, 13H); LCMS (ESI) m/z: 2507.76.

## Example 7: Synthesis of mDVPU-M

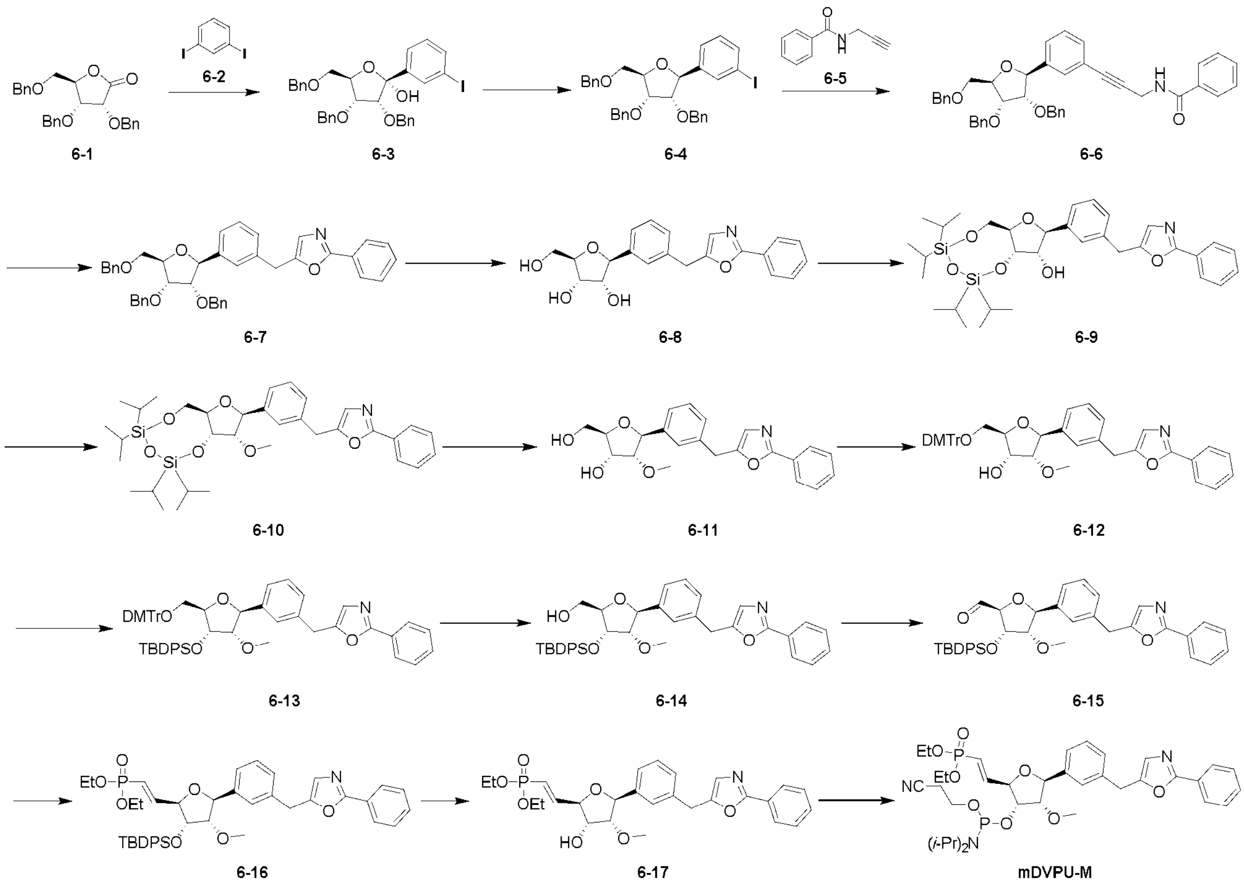


Step A: Under nitrogen protection, n-butyllithium (2.5 M, in tetrahydrofuran, 94.57 mL) was added to a solution of Compound **6-2** (78 g, 236.43 mmol) in tetrahydrofuran (1 L) at -78°C over 10 minutes. After the addition, the mixture was stirred at this temperature for 20 minutes, and a solution of Compound **6-1** (98.94 g, 236.43 mmol) in tetrahydrofuran (500 mL) was added at -78 °C. The reaction was stirred at -78 °C for 1.5 hours, and quenched at 0 °C by adding saturated ammonium chloride (500 mL). The reaction mixture was extracted with ethyl acetate (1 L), washed with saturated brine (500 mL * 2), dried over anhydrous sodium sulfate, filtered, and concentrated under reduced pressure to obtain a crude product. The crude product was purified by silica gel column chromatography (petroleum ether/ethyl acetate = 1/0 to 3/1) to give Compound **6-3.**

Step B: Under nitrogen protection, boron trifluoride ether (217.33 mL) and triethylsilyl hydrogen (282.24 mL) were added to a solution of Compound **6-3** (110 g, 176.71 mmol) in dichloromethane (1.1 L) at -78 °C. The reaction was stirred at -78 °C for 2 hours, and was quenched by 5% sodium carbonate (500 mL) at 0 °C. The reaction mixture was extracted with

dichloromethane (300 mL * 2), and the combined organic phases were washed with saturated sodium chloride (200 mL * 2), dried over anhydrous sodium sulfate, filtered, and concentrated under reduced pressure to give a crude product. The crude product was purified by silica gel column chromatography (petroleum ether/ethyl acetate = 1/0 to 5/1) to give Compound **6-4.**

Step C: Under nitrogen protection, Compound **6-4** (73 g, 120.36 mmol), Compound 6-5 (21.08 g, 132.40 mmol), cuprous iodide (2.29 g, 12.04 mmol), N,N,-diisopropylethylamine (601.82 mmol, 104.83 mL) and 1,1-bis (diphenylphosphine) ferrocene palladium chloride (8.81 g, 12.04 mmol) were added to acetonitrile (400 mL), with stirring at 25°C for the reaction for 2 hours. The reaction mixture was diluted with water (300 mL) and extracted with ethyl acetate (500 mL * 2). The combined organic phases were washed with saturated sodium chloride (300 mL * 2), dried over anhydrous sodium sulfate, filtered, and concentrated to give a crude product. The crude product was purified by silica gel column chromatography (petroleum ether/ethyl acetate = 1/0 to 3/1) to give Compound **6-6.**

Step D: Under nitrogen protection, Compound **6-6** (70 g, 109.76 mmol) and cesium carbonate (71.52 g, 219.52 mmol) were added to acetonitrile (700 mL), with stirring at 80 °C for the reaction for 16 hours. The reaction mixture was concentrated under reduced pressure to remove acetonitrile, diluted with water (100 mL), and extracted with ethyl acetate (100 mL * 2). The combined organic phases were washed with saturated sodium chloride (50 mL * 2), dried over anhydrous sodium sulfate, filtered, and concentrated under reduced pressure to obtain a crude product. The crude product was purified by silica gel column chromatography (petroleum ether/ethyl acetate = 1/0 to 3/1) to give Compound **6-7.**

Step E: Under nitrogen protection, boron trichloride (1M, in ether, 627.19 mL) was added to a solution of Compound **6-7** (50 g, 78.40 mmol) in dichloromethane (500 mL) at -78 °C. The reaction was stirred at -78°C for 2 hours. The reaction was detected to be complete. The reaction mixture was quenched by slowly adding methanol (100 mL) dropwise at -78 °C and then concentrated under reduced pressure to obtain a crude product. The crude product was purified by silica gel column chromatography (petroleum ether/ethyl acetate = 1/0 to 1/1, and then ethyl acetate/ethanol = 10/1 to 5/1) to give compound **6-8.**

Step F: Under nitrogen protection, Compound **6-8** (28.8 g, 78.39 mmol), 1,3-dichloro-1,1,3,3-tetraisopropyldisilyl ether (29.67 g, 94.07 mmol) and imidazole (21.35 g, 313.56 mmol) were added to dichloromethane (280 mL), and the reaction was stirred at 25 °C for 16 hours. The reaction mixture was diluted with water (200 mL) and extracted with dichloromethane (200 mL * 2). The combined organic phases were washed with saturated sodium chloride (200 mL), dried over anhydrous sodium sulfate, filtered, and concentrated under reduced pressure to obtain a crude product. The crude product was purified by silica gel column chromatography (petroleum ether/ethyl acetate = 1/0 to 4/1) to give Compound **6-9.**

Step G: Under nitrogen protection, sodium hydride (2.20 g, 55.09 mmol, 60% purity) was slowly added to a solution of Compound **6-9** (28 g, 45.91 mmol) in N,N-dimethylformamide (140 mL) at 0 °C. After the addition, the reaction was stirred at 0 °C for 0.5 hours, and then methyl iodide (137.73 mmol, 8.57 mL) was added with stirring at 25 °C for the reaction for 1.5 hours. The reaction mixture was quenched with saturated ammonium chloride (100 mL) at 10-25 °C, diluted with water (200 mL), and extracted with ethyl acetate (200 mL * 2). The combined organic phases were washed with saturated sodium chloride (30 mL * 2), dried over anhydrous sodium sulfate, filtered, and concentrated under reduced pressure to give a crude product. The crude product was purified by silica gel column chromatography (petroleum ether/ethyl acetate = 1/0 to 4/1) to give Compound **6-10.**

Step H: Under nitrogen protection, Compound **6-10** (22.3 g, 35.74 mmol) and triethylamine hydrogen fluoride (9.53 g, 78.63 mmol) were added to tetrahydrofuran (160 mL). The reaction was stirred at 25 °C for 16 hours. The reaction mixture was concentrated under reduced pressure to obtain a crude product. The crude product was purified by silica gel column chromatography (petroleum ether/ethyl acetate = 1/0 to 0/1) to give Compound **6-11.**

Step I: Under nitrogen protection, Compound **6-11** (10.6 g, 27.79 mmol), 4,4-dimethoxytrityl chloride (11.30 g, 33.35 mmol) and triethylenediamine (4.68 g, 41.69 mmol) were mixed in dichloromethane (60 mL), and the reaction was stirred at 25 °C for 16 hours. The reaction mixture was diluted with water (30 mL) and extracted with dichloromethane (50 mL * 2). The combined organic phases were washed with saturated sodium chloride (20 mL), dried over anhydrous sodium sulfate, filtered, and concentrated under reduced pressure to obtain a crude product. The crude product was purified by silica gel column chromatography (petroleum ether/ethyl acetate = 1/0 to 1/1, with 0.1% triethylamine) to give Compound **6-12.**

Step J: Under nitrogen protection, Compound **6-12** (17 g, 24.86 mmol), imidazole (3.39 g, 49.72 mmol) and tert-butyldiphenylchlorosilane (8.20 g, 29.83 mmol) were added to dichloromethane (170 mL), and the reaction was stirred at 25 °C for 16 hours. The reaction mixture was diluted with water (20 mL) and extracted with dichloromethane (30 mL * 2). The combined organic phases were washed with saturated sodium chloride (30 mL), dried over anhydrous sodium sulfate, filtered, and concentrated under reduced pressure to obtain a crude product. The crude product was purified by silica gel column chromatography (petroleum ether/ethyl acetate = 1/0 to 2/1) to give Compound **6-13.**

Step K: Under nitrogen protection, Compound **6-13** (21 g, 22.77 mmol) and p-toluenesulfonyl chloride monohydrate (2.17 g, 11.39 mmol) were added to dichloromethane (200 mL) and methanol (60 mL), and the reaction was stirred at 5 °C for 1 hour. The reaction mixture was quenched with saturated sodium bicarbonate (20 mL) and extracted with

dichloromethane (50 mL * 2). The combined organic phases were washed with saturated sodium chloride (30 mL * 2), dried over anhydrous sodium sulfate, filtered, and concentrated under reduced pressure to obtain a crude product. The crude product was purified by silica gel column chromatography (petroleum ether/ethyl acetate = 1/0 to 2/1) to give Compound **6-14.**

Step L: Under nitrogen protection, compound **6-14** (13.7 g, 22.10 mmol) and Dess-Martin periodinane (11.25 g, 26.52 mmol) were added to dichloromethane (100 mL), and the reaction was stirred at 25 °C for 1 hour. The reaction mixture was quenched with saturated sodium bicarbonate (20 mL) and extracted with dichloromethane (50 mL * 2). The organic resulting phases were combined, washed with saturated sodium chloride (30 mL * 2), dried over anhydrous sodium sulfate, filtered, and concentrated under reduced pressure to obtain a crude product. The crude product was purified by silica gel column chromatography (petroleum ether/ethyl acetate = 1/0 to 2/1) to give Compound **6-15.**

Step M: Under nitrogen protection, Compound **6-15** (2 g, 3.24 mmol), tetraethyl methylenediphosphonate (1.12 g, 3.88 mmol), lithium chloride (274.48 mg, 6.47 mmol) and 1.8-diazabicyclo[5.4.0]undec-7-ene (1.48 g, 9.71 mmol) were added to dichloromethane (20 mL), the reaction was stirred at 25 °C for 3 hours. The reaction mixture was quenched with water (10 mL) at 25 °C and extracted with dichloromethane (30 mL * 2). The combined organic phases were washed with saturated sodium chloride (30 mL), dried over anhydrous sodium sulfate, filtered, and concentrated under reduced pressure to obtain a crude product. The crude product was purified by silica gel column chromatography (petroleum ether/ethyl acetate = 1/0 to 1/1) to give Compound **6-16.**

Step N: Under nitrogen protection, Compound **6-16** (2.1 g, 2.79 mmol) and triethylamine hydrogen fluoride (676.97 mg, 5.59 mmol) were added to tetrahydrofuran (10 mL). The reaction mixture was stirred at 40 °C for 16 hours, and then was concentrated under reduced pressure to give a crude product. The crude product was purified by silica gel column chromatography (petroleum ether/ethyl acetate = 1/0 to 0/1, and then ethyl acetate/ethanol = 1/0 to 5/1) to obtain Compound **6-17.**

Step O: Under nitrogen protection, Compound **6-17** (1.3 g, 2.53 mmol), (2-cyanoethoxy) bis(diisopropylamino) phosphine (1.07 g, 3.54 mmol) and 4,5-dicyanoimidazole (149.48 mg, 1.27 mmol) were added to dichloromethane (15 mL), and the reaction was allowed to proceed at 25 °C for 1 hour. The reaction mixture was diluted with water (10 mL) and extracted with dichloromethane (50 mL * 2). The combined organic phases were washed with saturated sodium chloride (30 mL * 2), dried over anhydrous sodium sulfate, filtered, and concentrated under reduced pressure to obtain a crude product. The crude product was purified by reverse phase column chromatography (neutral conditions; column: Waters Xbridge C18 150 * 50 mm * 10 μm; mobile phase: [water (0.1% ammonium bicarbonate)-acetonitrile]; gradient: % acetonitrile = 57%-87%) to give Compound **mDVPU-M.** MS=774.5 (M+$HCO_3^-$), $^1$H NMR (400 MHz, DMSO-$d_6$) δ = 8.00 - 7.82 (m, 2H), 7.58 - 7.44 (m, 3H), 7.43 - 7.23 (m, 4H), 7.04 (s, 1H), 6.90 - 6.65 (m, 1H), 6.10 (br dd, *J* = 18.0, 19.4 Hz, 1H), 4.89 (dd, *J* = 5.9, 15.8 Hz, 1H), 4.69 - 4.53 (m, 1H), 4.33 - 4.19 (m, 1H), 4.15 (s, 2H), 4.04 - 3.92 (m, 4H), 3.86 - 3.53 (m, 5H), 3.34 - 3.29 (m, 3H), 2.80 (td, *J* = 5.4, 11.0 Hz, 2H), 1.30 - 1.06 (m, 18H).

**Example 8: Synthesis of D21-M**

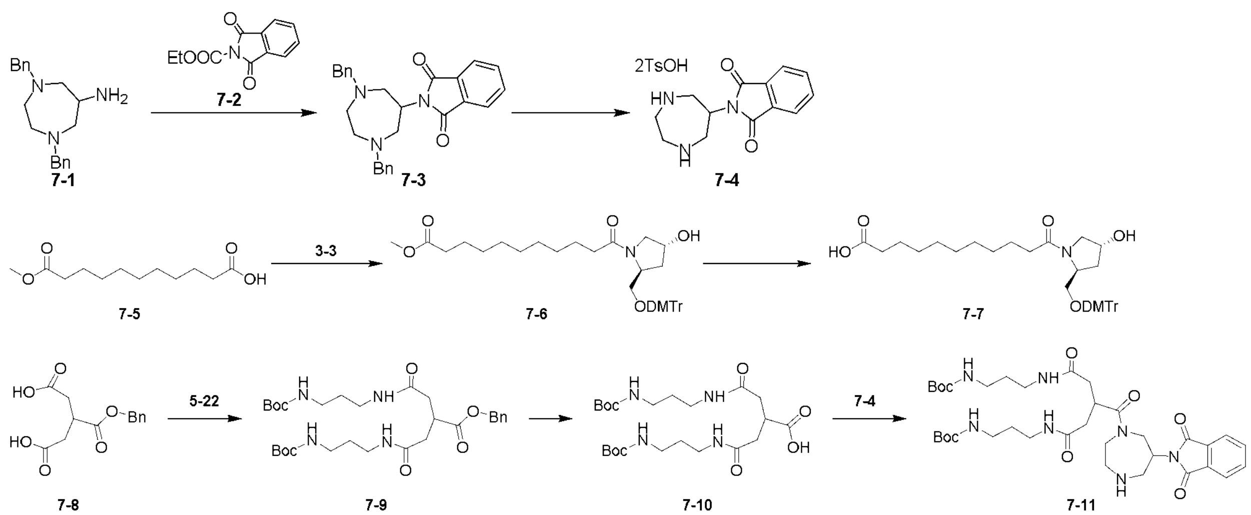

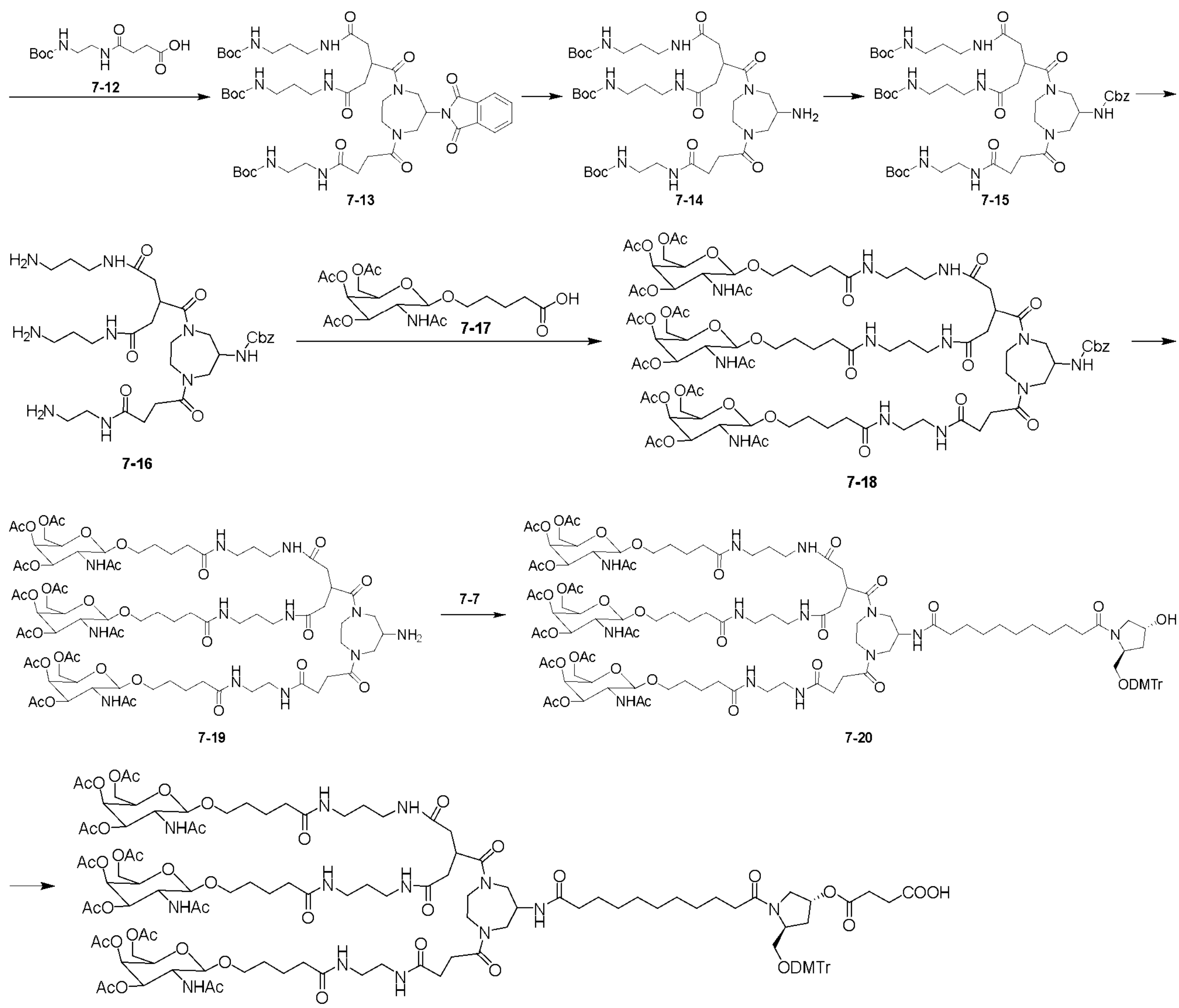


7-13 7-14 7-15 7-16 7-18 7-19 7-20

D21-M

Step A: Sodium carbonate (14.91 g, 140.65 mmol) was added to a solution of Compound **7-1** (27.7 g, 93.76 mmol) and Compound **7-2** (21.58 g, 98.45 mmol) in tetrahydrofuran (300 mL), and the reaction was stirred at 25 °C for 12 hours. After the reaction was completed, the reaction mixture was diluted with ethyl acetate (800 mL), and washed with saturated brine (500 mL * 3). The organic phase was dried over anhydrous sodium sulfate, filtered, and concentrated under reduced pressure to give a crude product. The crude product was purified by silica gel column chromatography (eluted with ethyl acetate/petroleum ether = 0/1 to 1/10) to give Compound **7-3.**

Step B: Under nitrogen protection, palladium on carbon (14 g, 10% palladium content) and palladium hydroxide on carbon (14 g, 20% purity) were added to a solution of Compound **7-3** (27.9 g, 65.57 mmol) and p-toluenesulfonic acid monohydrate (27.44 g, 144.25 mmol) in tetrahydrofuran (1 L). The reaction system was subjected to hydrogen displacement three times and reacted under a hydrogen atmosphere of 50 psi at 50 °C for 12 hours. After completion of the reaction, the reaction mixture was filtered through Celite, and the resulting filter cake was washed with a mixed solution of methylene chloride/methanol (400 mL/400 mL), and the filtrate was concentrated under reduced pressure to obtain a crude product. The crude product was triturated with ethyl acetate (600 mL) at 25 °C for 12 hours, filtered, and the resulting filter cake was concentrated under reduced pressure to give Compound **7-4.**

Step C: To a solution of Compound **7-5** (9 g, 39.08 mmol) in dichloromethane (150 mL) were added N,N-diisopropylethylamine (7.58 g, 58.62 mmol), 1-hydroxy-7-azabenzotriazole (7.98 g, 58.62 mmol), 1-(3-dimethylaminopropyl)-3-ethylcarbodiimide hydrochloride (11.24 g, 58.62 mmol) and Compound 3-3 (18.03 g, 42.99 mmol) successively, with stirring at 25 °C for 12 hours. After completion of the reaction, the reaction mixture was diluted with water (250 mL) and extracted with dichloromethane (200 mL). The resulting organic phase was dried over anhydrous sodium sulfate, filtered, and concentrated under reduced pressure to give a crude product. The crude product was then purified by reverse phase column chromatography (neutral conditions, column type: Phenomenex luna c18 250 mm * 100 mm * 10 μm; mobile phase: [water (10 mM ammonium bicarbonate)-acetonitrile]; gradient: % acetonitrile = 60%-80%, 30 minutes) to give

Compound **7-6.**

Step D: To a solution of Compound **7-6** (20.7 g, 32.76 mmol) in a mixture of methanol (65 mL), tetrahydrofuran (65 mL) and water (65 mL) was added lithium hydroxide (941.56 mg, 39.32 mmol). The mixture was stirred at 25 °C for 12 hours, followed by additional lithium hydroxide (392.32 mg, 16.38 mmol), and stirred at 25 °C for an additional 8 hours. After completion of the reaction, the reaction mixture was concentrated under reduced pressure to give a crude product, which was purified by reverse phase column chromatography (alkaline conditions, column type: Kromasil Eternity XT 250 * 80 mm * 10 μm; mobile phase: [water (0.05% ammonium hydroxide)-acetonitrile]; gradient: %acetonitrile = 15%-45%, 20 minutes) to give Compound **7-7.**

Step E: To a solution of Compound **7-8** (7 g, 26.29 mmol) and triethylamine (13.3 g, 131.46 mmol) in N,N-dimethylformamide (70 mL) was added O-(7-azabenzotriazol-1-yl)-N,N,N,N-tetramethylurea hexafluorophosphate (23.99 g, 63.1 mmol). The mixture was stirred at 25 °C for 0.5 h, followed by the addition of Compound **5-22** (10.99 g, 63.1 mmol), and stirred at 25 °C for an additional 12 hours. After completion of the reaction, the reaction was diluted with ethyl acetate (400 mL), washed successively with hydrochloric acid (0.5 M, 300 mL * 2), saturated brine (300 mL * 2), sodium bicarbonate solution (300 mL * 2), and saturated brine (300 mL * 2), dried over anhydrous sodium sulfate, filtered, and concentrated under reduced pressure to obtain a crude product. The crude product was purified by silica gel column chromatography (eluted with ethyl acetate/petroleum ether = 0/1 to 1/0) to give Compound **7-9.**

Step F: To a solution of Compound **7-9** (18 g, 31.1 mmol) in a mixture of methanol (60 mL) and water (30 mL) was added sodium hydroxide (1.74 g, 43.55 mmol). The mixture was stirred at 30 °C for 3 hours, and then concentrated under reduced pressure to remove methanol. Then the reaction system was diluted with water (40 mL) and extracted with dichloromethane (60 mL * 3), and the organic phase was discarded. The aqueous phase was adjusted to pH 2 to 3 with hydrochloric acid (1M), then extracted with a mixture of dichloromethane/isopropanol (volume ratio: 4/1, 50 mL*4). The combined organic phases were dried over anhydrous sodium sulfate, filtered, and concentrated under reduced pressure to give Compound **7-10.**

Step G: To a solution of Compound **7-10** (7.42 g) and triethylamine (9.22 g, 91.13 mmol) in N,N-dimethylformamide (100 mL) was added benzotriazole-N,N,N,N-tetramethylurea hexafluorophosphate (6.91 g, 18.23 mmol). The mixture was stirred at 25 °C for 0.5 hours, followed by the addition of Compound **7-4** (17.91 g), and stirred at 25 °C for an additional 12 hours. After completion of the reaction, the reaction mixture was diluted with ethyl acetate (300 mL) and 2-methyltetrahydrofuran (300 mL) and adjusted to pH 10, which was then washed with saturated brine (300 mL * 3), dried over anhydrous sodium sulfate, filtered, and concentrated under reduced pressure to obtain a crude product. The crude product was purified by silica gel column chromatography (eluted with methanol/dichloromethane = 0/1 to 1/10) to give Compound **7-11.**

Step H: To a solution of Compound **7-11** (3.62 g, 13.91 mmol) and triethylamine (3.52 g, 34.78 mmol) in N,N-dimethylformamide (80 mL) was added O-(7-azabenzotriazol-1-yl)-N,N,N,N-tetramethylurea hexafluorophosphine salt (6.17 g, 16.23 mmol). The mixture was stirred at 25 °C for 0.5 hours, followed by the addition of Compound **7-12** (8.3 g, 11.59 mmol), and stirred at 25 °C for an additional 12 hours. After completion of the reaction, the reaction was diluted with 2-methyltetrahydrofuran (400 mL), which was then washed successively with citric acid aqueous solution (10%, 300 mL * 2), saturated brine (300 mL * 2), sodium bicarbonate aqueous solution (300 mL * 2), and saturated brine (300 mL * 2), dried over anhydrous sodium sulfate, filtered, and concentrated under reduced pressure to obtain a crude product. The crude product was purified by silica gel column chromatography (eluted with methanol/dichloromethane = 0/1 to 12/100) to give Compound **7-13.**

Step I: To a solution of Compound **7-13** (8.52 g, 8.89 mmol) in a mixture of tetrahydrofuran (50 mL) and ethanol (50 mL) was added hydrazine hydrate (7.66 g, 149.96 mmol, 98% purity), with stirring at 45 °C for 6 hours. After completion of the reaction, the reaction mixture was concentrated under reduced pressure to obtain a crude product, which was triturated with a mixed solution of acetonitrile (50 mL) and dichloromethane (50 mL) at 25 °C for 0.5 hours, filtered, and the filtrate was concentrated under reduced pressure to give Compound **7-14.**

Step J: To a solution of Compound **7-14** (7.9 g, 9.54 mmol) and N,N-diisopropylethylamine (2.47 g, 19.08 mmol) in tetrahydrofuran (70 mL) was added benzoxycarbonylsuccinimide (2.85 g, 11.45 mmol), with stirring at 25 °C for 12 hours. After completion of the reaction, the reaction mixture was diluted with 2-methyltetrahydrofuran (400 mL), and then washed successively with citric acid aqueous solution (10%, 200 mL * 2), saturated brine (200 mL * 2), sodium bicarbonate aqueous solution (200 mL * 2), and saturated brine (200 mL * 2) for liquid separation. The resulting organic phase was dried over anhydrous sodium sulfate, filtered, and concentrated under reduced pressure to obtain a crude product. The crude product was purified by silica gel column chromatography (eluted with methanol/dichloromethane = 0/1 to 12/100) to give Compound **7-15.**

Step K: A solution of Compound **7-15** (8 g, 8.31 mmol) in formic acid (80 mL, 98% purity) was stirred at 45 °C for 2 hours and, after completion of the reaction, was concentrated under reduced pressure to give the formate salt of crude Compound **7-16.**

Step L: To a mixed solution of a formate salt of Compound **7-16** (6.65 g), Compound 7-17 (20.82 g, 38.24 mmol), tetrahydrofuran (170 mL) and water (170 mL) was added N,N-diisopropylethylamine (16.12 g, 124.71 mmol), with stirring

at 30 °C for 12 hours. After completion of the reaction, the reaction was extracted with a mixed solution of dichloromethane/isopropanol (volume ratio: 5/1, 100 mL * 3). The combined organic phases were dried over anhydrous sodium sulfate, filtered, and concentrated under reduced pressure to obtain a crude product. The crude product was then dissolved in acetic anhydride (27.18 g, 266.19 mmol, 25 mL) and pyridine (100 mL) with stirring at 25 °C for 12 hours, which was then concentrated under reduced pressure to obtain a crude product. The crude product was purified by reverse phase column chromatography (acidic conditions, column type: Phenomenex luna c18 250mm * 80mm * 10μm; mobile phase: [water (0.225% formic acid)-acetonitrile]; gradient: % acetonitrile = 25%-45%, 20 minutes) to give Compound **7-18.**

Step M: Under nitrogen protection, to a solution of Compound **7-18** (2.5 g, 1.28 mmol) and p-toluenesulfonic acid monohydrate (243.86 mg, 1.28 mmol) in tetrahydrofuran (50 mL) was added palladium on carbon (1 g, 10% palladium content). The reaction system was subjected to hydrogen displacement three times and reacted under a hydrogen atmosphere of 20 psi at 25°C for 12 hours. After the reaction was completed, the reaction mixture was filtered through Celite, and the filtrate was concentrated under reduced pressure to obtain the p-toluenesulfonate salt of crude Compound **7-19.**

Step N: A solution of Compound **7-7** (950.84 mg, 1.54 mmol), 1-hydroxy-7-azabenzotriazole (226.95 mg, 1.67 mmol), 1-(3-dimethylaminopropyl)-3-ethylcarbodiimide hydrochloride (319.64 mg, 1.67 mmol) and N,N-diisopropylethylamine (994.62 mg, 7.7 mmol) in N,N-dimethylformamide (25 mL) was subjected to nitrogen displacement three times and stirred at 25 °C for 0.5 hours. Then the p-toluenesulfonate salt of Compound **7-19** was added to the reaction system, with stirring at 25 °C for 0.5 hours. After completion of the reaction, the reaction mixture was directly purified by reverse phase column chromatography (neutral condition, column type: Waters Xbridge C18 150 * 50 mm * 10 μm; mobile phase: [water (10 mM ammonium bicarbonate)-acetonitrile]; gradient: % acetonitrile = 28%-58%, 10 minutes) to give compound **7-20.**

Step O: A solution of Compound **7-20** (1.86 g, 769.97 μmol), butanedioic anhydride (385.27 mg, 3.85 mmol) and triethylamine (623.3 mg, 6.16 mmol) in dichloromethane (5 mL) was subjected to nitrogen displacement three times and stirred at 30 °C for 2 hours under nitrogen protection. After completion of the reaction, the reaction mixture was concentrated under reduced pressure to give a crude product, which was purified by reverse phase column chromatography (neutral conditions; column type: Waters Xbridge C18 150 * 50 mm * 10 μm; mobile phase: [water (10 mM ammonium bicarbonate)-acetonitrile]; gradient: % acetonitrile = 20%-50%, 10 minutes) to give Compound **D21-M.**
$^1$H NMR (400 MHz, DMSO-d6) δ = 8.04 - 7.63 (m, 9H), 7.34 - 7.13 (m, 9H), 6.93 - 6.80 (m, 4H), 5.42 - 5.31 (m, 1H), 5.21 (d, J = 3.2 Hz, 3H), 4.97 (dd, J = 3.2, 11.2 Hz, 3H), 4.49 (d, J = 8.4 Hz, 3H), 4.25 - 4.15(m, 1H), 4.07 - 3.95 (m, 9H), 3.93 - 3.82 (m, 3H), 3.79 - 3.64 (m, 12H), 3.62 - 3.50 (m, 4H), 3.48 - 3.17 (m, 14H), 3.11 - 2.91 (m, 14H), 2.48 - 2.28 (m, 8H), 2.26 - 2.15 (m, 4H), 2.10 (s, 9H), 2.07 - 2.01 (m, 8H), 1.99 (s, 10H), 1.89 (s, 9H), 1.77 (s, 9H), 1.46 (s, 19H), 1.31 - 1.09 (m, 10H).

### Example 9: Synthesis of B29-M

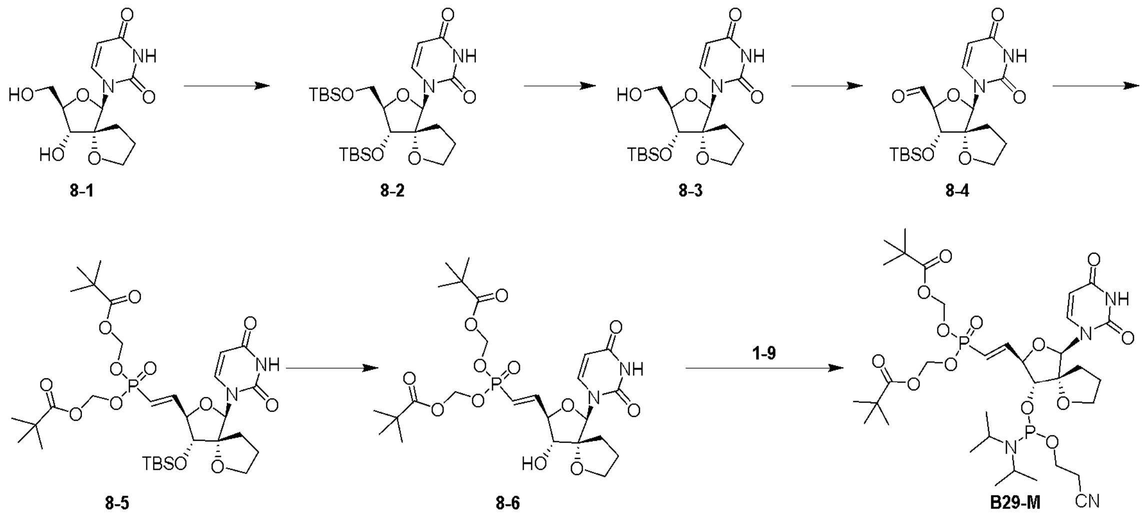


8-1 8-2 8-3 8-4

8-5 8-6 B29-M

Step A: Under nitrogen protection, Compound **8-1** (1.83 g, 6.44 mmol), imidazole (2.63 g, 38.63 mmol), tert-butyldimethylchlorosilane (3.88 g, 25.75 mmol) and 4-dimethylaminopyridine (235.94 mg, 1.93 mmol) were mixed in a N,N-dimethylformamide (30 mL) solution. The reaction mixture was heated to 60 °C for 16 hours. The reaction mixture was diluted with water (100 mL), and extracted with ethyl acetate (150 mL × 2). The combined organic phases were washed with 5% aqueous citric acid solution (50 mL × 2) and aqueous sodium chloride solution (100 mL × 2), dried over anhydrous sodium sulfate, filtered, and concentrated under reduced pressure to give Compound **8-2.**

Step B: Under nitrogen protection, Compound **8-2** (3.3 g, 6.44 mmol) and trifluoroacetic acid (23.03 g, 201.93 mmol) were mixed in a solution of tetrahydrofuran (60 mL) and water (15 mL), and the reaction was stirred at 25 °C for 2 hours. The reaction mixture was diluted with water (30 mL), and extracted with ethyl acetate (50 mL × 2). The combined organic phases were washed with saturated sodium bicarbonate (50 mL × 2) and saturated sodium chloride (50 mL × 2), dried over anhydrous sodium sulfate, filtered, and concentrated under reduced pressure to obtain a crude product. The crude product was purified by silica gel column chromatography (petroleum ether/ethyl acetate = 1/0 to 1/1) to give Compound **8-3.**

Step C: Under nitrogen protection, Compound **8-3** (1.29 g, 3.24 mmol) and 2-iodoacylbenzoic acid (1.81 g, 6.47 mmol) were mixed in a solution of acetonitrile (20 mL), and the reaction mixture was stirred at 50 °C for 12 hours. The reaction mixture was filtered, and the filtrate was concentrated under reduced pressure to dryness to give Compound **8-4.**

Step D: Under nitrogen protection, to a solution of tetrahydrofuran (5 mL) in sodium hydrogen (363.13 mg, 9.08 mmol, 60% purity) at -78 °C was added tetrakis[(pivalyloxy)methyl] methylenediphosphonate (4.79 g, 7.57 mmol) at -78 °C. The system was reacted at -78 °C for 0.5 hours, followed by addition of a solution of Compound **8-4** (1.2 g, 3.03 mmol) in tetrahydrofuran (5 mL) at -78 °C. The reaction was warmed to 25 °C with stirring for 2.5 hours. The reaction mixture was quenched at 10-25 °C by adding saturated ammonium chloride (30 mL), diluted with water (10 mL), and extracted with ethyl acetate (100 mL × 2). The combined organic phases were washed with saturated sodium chloride (50 mL × 2), dried over anhydrous sodium sulfate, filtered and concentrated under reduced pressure to obtain a crude product. The crude product was purified by silica gel column chromatography (petroleum ether/ethyl acetate = 1/0 to 1/1) to give Compound **8-5.**

Step E: Under nitrogen protection, Compound **8-5** (1.1 g, 1.57 mmol) and formic acid (10 mL) were mixed in water (10 mL), and the reaction mixture was reacted at 40 °C for 12 hours. The reaction mixture was diluted with water (50 mL), and extracted with ethyl acetate (100 mL × 2). The combined organic phases were washed with saturated sodium bicarbonate (50 mL × 2) and saturated sodium chloride (50 mL × 2), dried over anhydrous sodium sulfate, filtered and concentrated under reduced pressure to obtain a crude product, which was purified by silica gel column chromatography (petroleum ether/ethyl acetate = 1/0 to 0/1) to obtain compound **8-6.**

Step F: Under nitrogen protection, compound **8-6** (430 mg, 730.62 μmol), compound **1-9** (330.32 mg, 1.1 mmol) and 4,5-dicyanoimidazole (430 mg, 730.62 μmol) were mixed in dichloromethane (5 mL). The mixture was reacted at 25 °C for 2 hours. The reaction mixture was concentrated under reduced pressure to give a crude product, which was purified by reverse phase column chromatography (column: Waters Xbridge C18 150×50 mm×10 μm; mobile phase: [10 mmol/L aqueous ammonium bicarbonate solution-acetonitrile]; gradient: % acetonitrile = 56%-76%, 10 minutes) to give Compound **B29-M.** LCMS (ESI) m/z: 789.5 $[M+H]^+$. $^1$H NMR (400 MHz, DMSO-d6) δ = 11.48 (s, 1H), 7.70 - 7.42 (m, 1H), 7.11 - 6.76 (m, 1H), 6.30 - 6.07 (m, 1H), 5.70 - 5.54 (m, 5H), 4.53 - 4.16 (m, 2H), 3.99 - 3.74 (m, 3H), 3.72 - 3.50 (m, 3H), 2.92 - 2.73 (m, 2H), 2.12 - 1.99 (m, 1H), 1.96 - 1.58 (m, 3H), 1.25 - 1.06 (m, 30H).

**Example 10: Synthesis of B30-M**

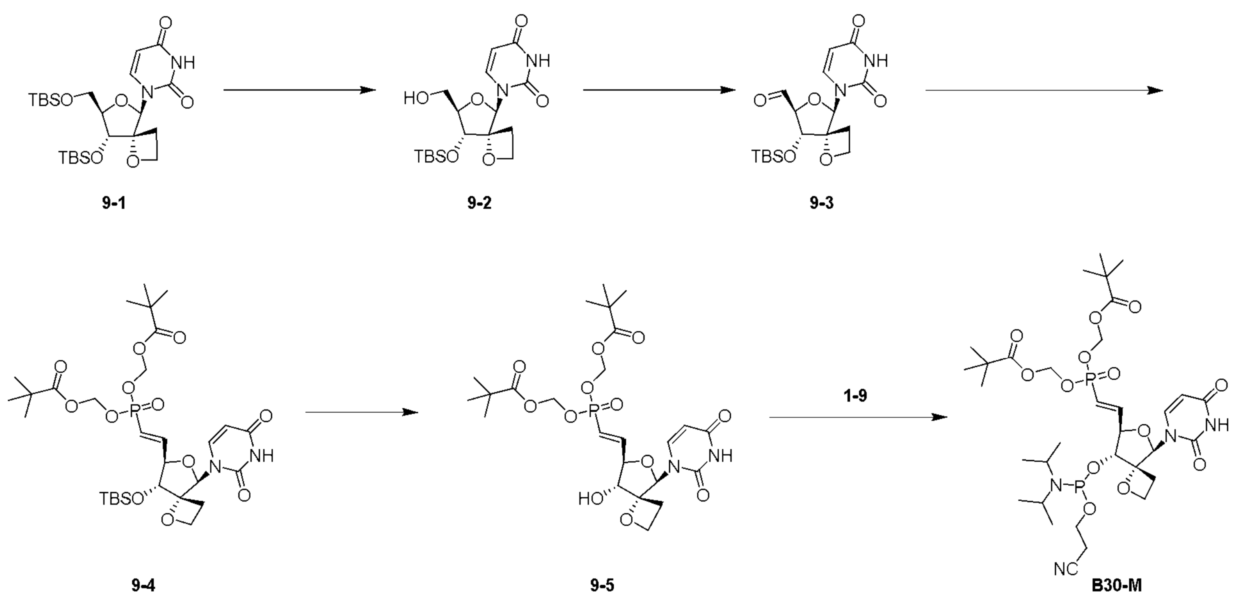


9-1 9-2 9-3

9-4 9-5 B30-M

Step A: Compound **9-1** (5 g, 10.02 mmol) was dissolved in tetrahydrofuran (40 mL), trifluoroacetic acid (10 mL) and water (10 mL), and the reaction mixture was stirred at 25 °C for 2 hours. The reaction mixture was diluted with 40 mL of water and extracted twice with ethyl acetate (40 mL × 2). The combined organic phases were washed with a mixture of saturated brine/saturated aqueous sodium bicarbonate solution (50 mL/10 mL × 4), dried over anhydrous sodium sulfate, filtered, and concentrated to give a crude product. The crude product was purified by silica gel column chromatography (ethyl acetate/petroleum ether = 0/1-3/5) to give Compound **9-2.** LCMS (ESI) m/z: 385.1 $[M+H]^+$.

Step B: Compound **9-2** (3.52 g, 9.15 mmol) was dissolved in acetonitrile (40 mL), to which 2-iodoacylbenzoic acid (5.40 g, 18.31 mmol, 95% purity) was added. The reaction mixture was stirred at 50 °C for 12 hours. The reaction mixture was cooled to room temperature, filtered, and the filtrate was concentrated in vacuo to obtain Compound **9-3.** LCMS (ESI) m/z: 383.2 $[M+H]^+$.

Step C: Sodium hydride (1.10 g, 27.45 mmol, 60% purity) was added to tetrahydrofuran (30 mL) and the reaction mixture was cooled to -70 °C and protected under a nitrogen atmosphere. Tetrakis[(pivalyloxy)methyl] methylenediphosphonate (14.47 g, 22.88 mmol) was added to the reaction mixture, and the reaction mixture was stirred at -70 ° C for 0.5 hours. Compound **9-3** (3.5 g, 9.15 mmol) was dissolved in tetrahydrofuran (30 mL) at -70 °C, which was then added dropwise to the above reaction mixture. The reaction mixture was stirred at 30 °C for 12 hours. The reaction mixture was slowly poured into a saturated aqueous ammonium chloride solution (200 mL) and extracted with ethyl acetate (300 mL). The resulting organic phase was dried over anhydrous sodium sulfate, filtered, and concentrated in vacuo to obtain a crude product. The crude product was purified by silica gel column chromatography (ethyl acetate/petroleum ether = 0/1 to 1/2) to give Compound **9-4.** LCMS (ESI) m/z: 689.2 $[M+H]^+$.

Step D: Compound **9-4** (3 g, 4.36 mmol) was dissolved in formic acid (15 mL) and water (15 mL), and the reaction mixture was stirred at 45 °C for 12 hours. The reaction mixture was cooled to room temperature, 40 mL of water was added, and the mixture was extracted with ethyl acetate (40 mL × 2). The combined organic phases were washed with a mixture of saturated brine/saturated aqueous sodium bicarbonate solution (50 mL/10 mL x 5), dried over anhydrous sodium sulfate, filtered, and concentrated in vacuo to obtain a crude product. The crude product was purified by silica gel column chromatography (ethyl acetate/petroleum ether = 0/1 to 17/20) to give Compound **9-5.** LCMS (ESI) m/z: 575.2 $[M+H]^+$.

Step 5: Compound **9-5** (1.2 g, 2.09 mmol) was dissolved in dichloromethane (10 mL), to which Compound **1-9** (944.33 mg, 3.13 mmol) and 4,5-dicyanoimidazole (123.34 mg, 1.04 mmol) were added. The reaction mixture was stirred at 25 °C for 3 hours. The reaction mixture was concentrated in vacuo to obtain a crude product. The crude product was purified by reversephase column chromatography (column: Waters Xbridge C18 150 × 50 mm × 10 μm; mobile phase: [aqueous ammonium bicarbonate (10 mmol/L)-acetonitrile]; gradient: 48%-68% acetonitrile, 10 minutes) to obtain **B30-M.**

$^1H$ NMR (400 MHz, DMSO-d6) δ = 11.48 (s, 1H), 7.78 - 7.55 (m, 1H), 7.01 - 6.64 (m, 1H), 6.22 - 6.04 (m, 1H), 5.91 (s, 1H), 5.71 - 5.53 (m, 5H), 4.58 - 4.15 (m, 4H), 3.95 - 3.70 (m, 2H), 2.97 - 2.52 (m, 4H), 1.26 - 1.10 (m, 32H).

$^{31}P$ NMR (162 MHz, DMSO-d6) δ = 153.98 - 144.66 (m, 1P), 17.24 - 16.44 (m, 1P).

**Example 11: Synthesis of B33-M**

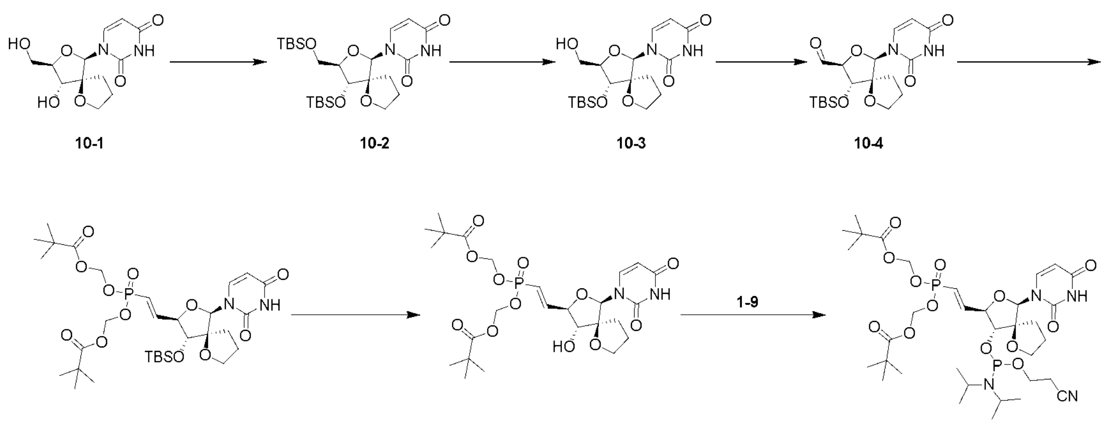


10-1 10-2 10-3 10-4 10-5 10-6 B33-M

Step A: Compound **10-1** (1.9 g, 6.68 mmol) was dissolved in *N,N*-dimethylformamide (30 mL), to which imidazole (2.73 g, 40.10 mmol), 4-dimethylaminopyridine (244.97 mg, 2.01 mmol), and tert-butyldimethylchlorosilane (4.03 g, 26.74 mmol) were added. The mixture was stirred at 65 °C for 16 hours. The reaction mixture was diluted with ethyl acetate (100 mL) and washed with saturated brine (100 mL × 2). The resulting organic phase was dried over anhydrous sodium sulfate, filtered, and concentrated under reduced pressure to obtain a crude product. The crude product was purified by silica gel

column chromatography (petroleum ether/ethyl acetate = 1/1 to 1/2) to give Compound **10-2.** LCMS (ESI) m/z: 513 $[M+H]^+$.

Step B: Compound **10-2** (2.5 g, 4.88 mmol) was dissolved in tetrahydrofuran (24 mL), to which aqueous trifluoroacetic acid solution (24 mL, V/V = 1/1) was added. The mixture was stirred at 25 °C for 2 hours. The reaction mixture was diluted with ethyl acetate (120 mL) and washed with saturated aqueous sodium bicarbonate solution (120 mL) and water (120 mL). The resulting organic phase was dried over anhydrous sodium sulfate, filtered, and concentrated under reduced pressure to obtain a crude product. The crude product was purified by silica gel column chromatography (petroleum ether/ethyl acetate = 2/1 to 1/1) to give Compound **10-3.** LCMS (ESI) m/z: 399 $[M+H]^+$.

Step C: Compound **10-3** (1.8 g, 4.52 mmol) was dissolved in acetonitrile (30 mL), and 2-iodoacylbenzoic acid (2.53 g, 9.03 mmol) was added. The mixture was stirred at 50 °C for 16 hours. The reaction mixture was filtered, and the filtrate was concentrated to obtain crude Compound **10-4.** LCMS (ESI) m/z: 397 $[M+H]^+$.

Step D: Under nitrogen protection, sodium hydride (411.55 mg, 10.29 mmol, 60% purity) and compound tetrakis [(pivalyloxy)methyl] methylenediphosphonate (5.42 g, 8.57 mmol) were added in tetrahydrofuran (15 mL) at -78 °C. The reaction was stirred at -78 °C for 0.5 hours, followed by the addition of a solution of Compound **10-4** (1.7 g, 4.29 mmol) in tetrahydrofuran (15 mL), and the mixture was stirred at 25 °C for 1 hour. The reaction mixture was quenched by adding saturated aqueous ammonium chloride solution (30 mL) and extracted with ethyl acetate (30 mL). The resulting organic phase was dried over anhydrous sodium sulfate, filtered, and concentrated under reduced pressure to obtain a crude product. The crude product was purified by silica gel column chromatography (petroleum ether/ethyl acetate = 2/1 to 1/1) to obtain Compound **10-5.**

Step E: Compound **10-5** (1.3 g, 1.85 mmol) was added to formic acid (13 mL) and water (13 mL). The mixture was stirred at 65 °C for 12 hours. The reaction mixture was diluted with ethyl acetate (120 mL) and washed with saturated aqueous sodium bicarbonate solution (120 mL) and water (120 mL). The resulting organic phase was dried over anhydrous sodium sulfate, filtered, and concentrated under reduced pressure to obtain a crude product. The crude product was purified by silica gel column chromatography (petroleum ether/ethyl acetate = 2/1 to 1/1) to give Compound **10-6.**

Step F: Under nitrogen protection, Compound **10-6** (520 mg, 883.54 μmol) was dissolved in dichloromethane (10 mL), and 4,5-dicyanoimidazole (520 mg, 883.54 μmol) and Compound **1-9** (399.46 mg, 1.33 mmol) were added. The mixture was stirred at 25 °C for 16 hours. The reaction mixture was concentrated under reduced pressure to obtain a crude product. The crude product was purified by high-performance liquid chromatography (column: Waters Xbridge C18 150 × 50 mm × 10 μm; mobile phase: [10 mmol/L aqueous ammonium bicarbonate solution-acetonitrile]; acetonitrile gradient: 54%-84%, 10 minutes) to give Compound **B33-M.** $^1H$ NMR (400 MHz, DMSO-$d^6$) $\delta$ = 11.45 (s, 1H), 7.62 - 7.58 (m, 1H), 6.99 - 6.75 (m, 1H), 6.22 - 6.10 (m, 1H), 6.04 (s, 1H), 5.66 - 5.59 (m, 5H), 4.58 - 4.51 (m, 1H), 4.17 - 4.01 (m, 1H), 3.84 - 3.68 (m, 3H), 3.65 - 3.51 (m, 3H), 2.84 - 2.78 (m, 2H), 2.39 - 2.16 (m, 1H), 1.94 - 1.80 (m, 2H), 1.77 - 1.64 (m, 1H), 1.17 (s, 30H).

**Example 12: Synthesis of B34-M**

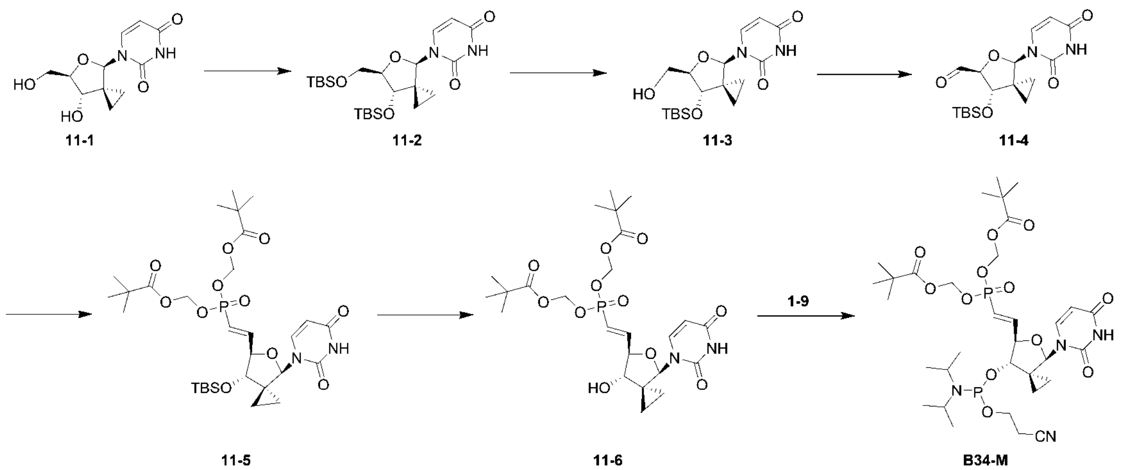


Step A: Compound **11-1** (2.5 g, 9.83 mmol) was dissolved in N,N-dimethylformamide (70 mL), to which imidazole (4.02 g, 59.00 mmol), 4-dimethylaminopyridine (360.39 mg, 2.95 mmol) and tert-butyldimethylchlorosilane (5.93 g, 39.33 mmol) were added. The mixture was stirred at 65 °C for 16 hours. The reaction mixture was diluted with ethyl acetate (200 mL) and washed with saturated brine (200 mL * 2). The resulting organic phase was dried over anhydrous sodium sulfate,

filtered, and concentrated under reduced pressure to give crude Compound **11-2.**

Step B: Compound **11-2** (4.7 g, 9.74 mmol) was dissolved in tetrahydrofuran (50 mL), and aqueous trifluoroacetic acid solution (50 mL, V/V = 1/1) was added. The mixture was stirred at 25 °C for 2 hours. The reaction mixture was diluted with ethyl acetate (200 mL) and washed with saturated aqueous sodium bicarbonate solution (200 mL) and water (200 mL). The resulting organic phase was dried over anhydrous sodium sulfate, filtered, and concentrated under reduced pressure to obtain a crude product. The crude product was purified by silica gel column chromatography (petroleum ether/ethyl acetate = 2/1 to 1/1) to give Compound **11-3.**

Step C: Compound **11-3** (1.8 g, 4.88 mmol) was dissolved in acetonitrile (40 mL), and 2-iodoacylbenzoic acid (3.42 g, 12.21 mmol) was added. The mixture was stirred at 55°C for 16 hours. The reaction mixture was filtered, and the filtrate was concentrated to give crude Compound **11-4.**

Step D: Under nitrogen protection, sodium hydride (458.41 mg, 11.46 mmol) and tetrakis[(pivalyloxy)methyl] methylenediphosphonate (6.04 g, 9.55 mmol) were added in tetrahydrofuran (20 mL) at -78 °C. The reaction was stirred at -78 °C for 0.5 hours, followed by the addition of a solution of Compound **11-4** (1.75 g, 4.78 mmol) in tetrahydrofuran (20 mL), and the mixture was stirred at 25 °C for 4 hours. The reaction mixture was quenched by adding saturated aqueous ammonium chloride solution (100 mL) and extracted with ethyl acetate (100 mL). The resulting organic phase was dried over anhydrous sodium sulfate, filtered, and concentrated under reduced pressure to obtain a crude product. The crude product was purified by silica gel column chromatography (petroleum ether/ethyl acetate = 2/1 to 1/1) to give Compound **11-5.**

Step E: Compound **11-5** (2.2 g, 3.27 mmol) was added to formic acid (20 mL) and water (20 mL), and the mixture was stirred at 65 °C for 12 hours. The reaction mixture was diluted with ethyl acetate (120 mL) and washed with saturated aqueous sodium bicarbonate solution (120 mL) and water (120 mL). The resulting organic phase was dried over anhydrous sodium sulfate, filtered, and concentrated under reduced pressure to obtain a crude product. The crude product was purified by silica gel column chromatography (petroleum ether/ethyl acetate = 2/1 to 1/1) to give Compound **11-6.**

Step F: Under nitrogen protection, Compound **11-6** (600 mg, 1.07 mmol) was dissolved in dichloromethane (12 mL), and 4,5-dicyanoimidazole (63.44 mg, 537.14 μmol) and Compound **1-9** (485.70 mg, 1.61 mmol) were added. The mixture was stirred at 25 °C for 16 hours. The reaction mixture was concentrated under reduced pressure to obtain a crude product. The crude product was purified by high-performance liquid chromatography (neutral conditions; column: Waters Xbridge C18 150 * 50 mm * 10 micron; mobile phase: [water (ammonium bicarbonate -10 mmol/L)-acetonitrile]; acetonitrile gradient: 53%-83%, 10 minutes) to give Compound **B34-M.**

$^1$H NMR (400 MHz, DMSO-$d_6$) δ = 11.34 (s, 1H), 7.67 - 7.62 (m, 1H), 7.02 - 6.77 (m, 1H), 6.18 - 6.07 (m, 1H), 5.92 (d, *J* = 9.2 Hz, 1H), 5.64 - 5.59 (m, 5H), 4.53 - 4.38 (m, 2H), 3.79 - 3.53 (m, 4H), 2.83 (t, *J* = 8.4 Hz, 1H), 2.78 (t, *J* = 8.4 Hz, 1H), 1.31 - 1.12 (m, 31H), 0.92 - 0.56 (m, 3H).

## Example 13: Synthesis of B36-M

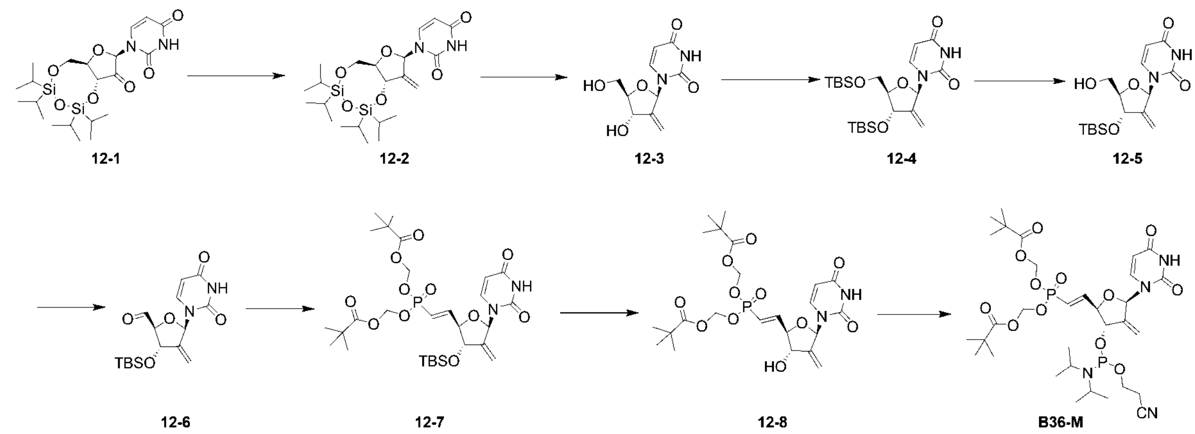

12-1 12-2 12-3 12-4 12-5

12-6 12-7 12-8 B36-M

Step A: Under nitrogen protection, potassium tert-butoxide (61.90 mL, 1 M) was added to a solution of Compound **12-1** (22.11 g, 61.90 mmoL) in tetrahydrofuran (60 mL) at 25 °C. After the addition was completed, the system was stirred at 25 °C for 0.5 hours, and then a solution of methyltriphenylphosphorus bromide (15 g, 30.95 mmoL) in tetrahydrofuran (40 mL) was added at 25 °C. The mixture was stirred at 35 °C for reaction for 15.5 hours. The reaction mixture was quenched by adding 5% citric acid (100 mL) and extracted with ethyl acetate (200 mL * 2). The resulting organic phase was washed with

saturated sodium chloride (100 mL * 2), dried over anhydrous sodium sulfate, filtered, and concentrated under reduced pressure to give a crude product. The crude product was purified by silica gel column chromatography (petroleum ether/ethyl acetate = 1/0 to 1/1) to give Compound **12-2.**

Step B: Under nitrogen protection, Compound **12-2** (12 g, 24.86 mmoL) and triethylamine trihydrofluoride (12.02 g, 74.58 mmoL) were mixed in tetrahydrofuran (100 mL). The mixture was reacted at 35 °C for 3 hours. The reaction mixture was concentrated under reduced pressure. The crude product was purified by silica gel column chromatography (petroleum ether/ethyl acetate = 1/0 to 0/1, ethanol/ethyl acetate = 0/1 to 1/4,) to give Compound **12-3.**

Step C: Under nitrogen protection, Compound **12-3** (3.9 g, 16.24 mmoL), 4-dimethylaminopyridine (595.05 mg, 4.87 mmoL), imidazole (6.63 g, 97.41 mmoL) and tert-butyldimethylchlorosilane (9.79 g, 64.94 mmoL) were mixed in N,N-dimethylformamide (40 mL). The mixture was reacted at 60 °C for 5 hours. The reaction mixture was diluted with water (50 mL) and extracted with ethyl acetate (100 mL * 2). The combined organic phases were washed with saturated sodium bicarbonate (100 mL * 2) and saturated sodium chloride (100 mL * 2), dried over anhydrous sodium sulfate, filtered, and concentrated under reduced pressure to give Compound **12-4.**

Step D: Under nitrogen protection, Compound **12-4** (6.61 g, 14.10 mmoL) and trifluoroacetic acid (10.15 g, 88.99 mmoL) were mixed in a solution of tetrahydrofuran (66 mL) and water (13 mL). The mixture was reacted at 15 °C for 6 hours. The reaction mixture was diluted with water (50 mL) and extracted with ethyl acetate (100 mL * 2). The combined organic phases were washed with saturated sodium bicarbonate (100 mL * 2), followed by saturated sodium chloride (100 mL * 2), dried over anhydrous sodium sulfate, filtered, and concentrated under reduced pressure to obtain a crude product. The crude product was purified by silica gel column chromatography (petroleum ether/ethyl acetate = 1/0 to 1/1) to give Compound **12-5.**

Step E: Under nitrogen protection, Compound **12-5** (3.3 g, 9.31 mmoL) and 2-iodoacylbenzoic acid (5.21 g, 18.62 mmoL) were mixed in acetonitrile (40 mL), and the mixture was reacted at 50 °C for 16 hours. The reaction mixture was filtered, and the filtrate was concentrated under reduced pressure to give Compound **12-6.**

Step F: Under nitrogen protection, tetrakis[(pivalyloxy)methyl] methylenediphosphonate (7.36 g, 11.63 mmoL) was added to a solution of tetrahydrofuran (30 mL) in sodium hydride (558.31 mg, 13.96 mmoL, 60% purity) at -78 °C. After the addition was completed, the mixture was reacted at -78 °C for 0.5 hours, and then a solution of Compound 12-6 (1.64 g, 4.65 mmoL) in tetrahydrofuran (10 mL) was added at -78 °C. The mixture was reacted at 25 °C for 2.5 hours. The reaction mixture was quenched by adding saturated ammonium chloride (50 mL) at 10-25 °C, diluted with water (50 mL), and extracted with ethyl acetate (100 mL * 2). The combined organic phases were washed with saturated sodium chloride (50 mL * 2), dried over anhydrous sodium sulfate, filtered, and concentrated under reduced pressure to obtain a crude product. The crude product was purified by silica gel column chromatography (petroleum ether/ethyl acetate = 1/0 to 1/1) to give Compound **12-7.**

Step G: Under nitrogen protection, Compound **12-7** (2 g, 3.04 mmoL) was mixed in formic acid (20 mL) and water (20 mL), then the mixture was stirred for 4 hours at 40 °C. The reaction mixture was diluted with water (100 mL) and extracted with ethyl acetate (100 mL * 2). The combined organic phases were washed with saturated sodium bicarbonate (50 mL * 2) and brine (50 mL * 2), dried over anhydrous sodium sulfate, filtered, and concentrated under reduced pressure to give a crude product. The crude product was purified by silica gel column chromatography (petroleum ether/ethyl acetate = 1/0 to 0/1) to give Compound **12-8.**

Step H: Under nitrogen protection, Compound **12-8** (1 g, 1.84 mmoL), (2-cyanoethoxy) bis(diisopropylamino) phosphine (830.34 mg, 2.75 mmoL) and 4,5-dicyanoimidazole (108.45 mg, 918.29 mmoL) were mixed in dichloromethane (20 mL). The mixture was reacted at 25 °C for 2 hours. The reaction mixture was concentrated under reduced pressure to obtain a crude product. The crude product was purified by reverse phase preparative chromatography (neutral conditions, column: Waters xbridge 150 * 25 mm * 10 micron; mobile phase: [water (ammonium bicarbonate)-acetonitrile]; gradient: % acetonitrile = 62%-82%, 8 minutes) to give Compound **B36-M.**

$^1$H NMR (400 MHz, DMSO-d6) $\delta$ = 11.42 (br s, 1H), 7.56 (d, J = 8.1 Hz, 1H), 7.04 - 6.74 (m, 1H), 6.48 (dd, J = 1.5, 8.9 Hz, 1H), 6.23 - 6.05 (m, 1H), 5.73 - 5.53 (m, 6H), 5.46 - 5.41 (m, 1H), 4.98 - 4.81 (m, 1H), 4.47 - 4.30 (m, 1H), 3.92 - 3.54 (m, 4H), 2.88 - 2.76 (m, 2H), 1.19 - 1.14 (m, 30H).

**Example 14: Synthesis of dR-M**

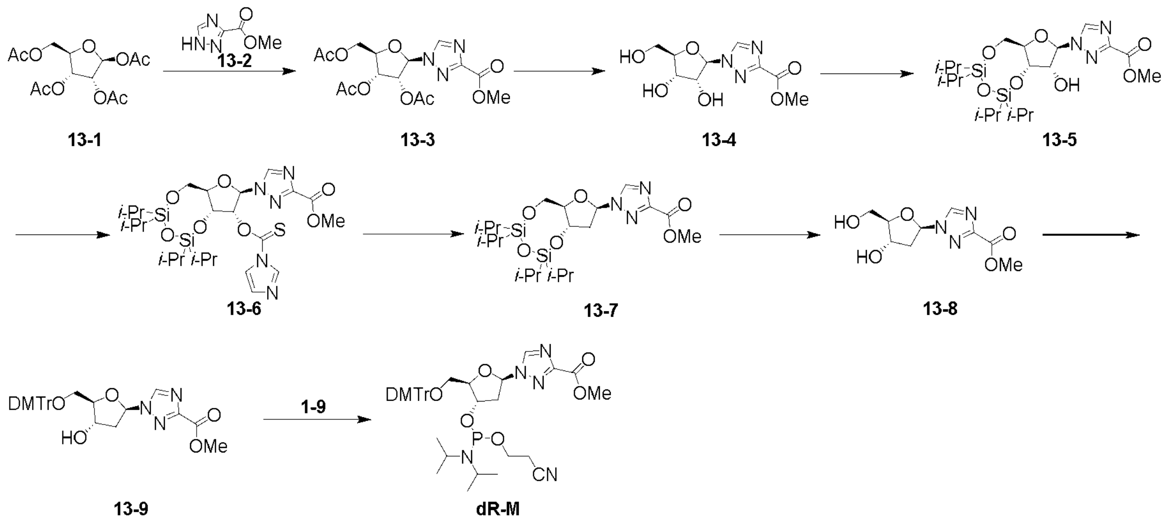


13-1 13-3 13-4 13-5 13-6 13-7 13-8 13-9 dR-M

Step A: Compound **13-1** (250.00 g, 785.48 mmol), Compound **13-2** (119.8 g, 942.58 mmol), and trifluoromethanesulfonic acid (1.18 g, 7.85 mmol) were mixed in a flask. The mixture was stirred at 125 °C for 3 hours under nitrogen protection. After completion of the reaction, ethyl acetate (2.5 L) was added, then the system was washed by adding water (2.5 L), 5% aqueous sodium bicarbonate solution (2.5 L × 2) and saturated brine (2.5 L) successively. The resulting organic phase was dried over anhydrous sodium sulfate and concentrated under reduced pressure to obtain a crude product. The crude product of the compound was recrystallized from ethanol (1 L) and n-heptane (1.75 L) at 70 °C, filtered, and the collected filter cake was dried in vacuo to give Compound **13-3.**

Step B: Triethylamine (26.26 g, 259.52 mmol) was added to a solution of Compound 13-3 (100.00 g, 259.52 mmol) in methanol (700 mL). The mixture was stirred at 65 °C for 10 hours under nitrogen protection. After completion of the reaction, 2 molar hydrogen chloride in methanol solution (130 mL) was added to the reaction mixture. The mixture was concentrated under reduced pressure to obtain Compound **13-4.**

Step C: Imidazole (88.33 g, 1.3 mol) and 1,3-dichloro-1,1,3,3-tetraisopropyldisiloxane (90.04 g, 285.47 mmol) were added dropwise to a solution of Compound **13-4** (67.27 g, 259.51 mmol) in dichloromethane (670 mL) at 0 °C. After the addition, the reaction mixture was naturally warmed to 25 °C and stirred continuously for 16 hours. After completion of the reaction, the organic phase was washed with water (1 L), 10% aqueous citric acid solution (1 L), and saturated brine (1 L) successively. The resulting organic phase was dried over anhydrous sodium sulfate and concentrated under reduced pressure to obtain a crude product. The crude product of the compound was recrystallized from n-heptane (350 mL) and tertiary methyl ether (350 mL) at 80 °C, filtered, and the collected filter cake was dried in vacuo to give Compound **13-5.**

Step D: Compound **13-5** (2 g, 3.99 mmol) and thiocarbonyldiimidazole (1.07 g, 5.98 mmol) were dissolved in dichloromethane (20 mL), and 4-dimethylaminopyridine (97.4 mg, 0.797 mmol) was added. The mixed solution was stirred at 25 °C for 16 hours. After the reaction was completed, the reaction mixture was concentrated under reduced pressure to afford a crude product of Compound **13-6.**

Step E: The crude product of Compound **13-6** (2.44 g), azobisisobutyronitrile (163.71 mg, 996.97 μ mol), tri-n-butyltin hydrogen (7.55 g, 25.94 mmol) were dissolved in anhydrous toluene (30 mL), and the system was subjected to nitrogen displacement 3 times. The reaction mixture was stirred at 100 °C for 6 hours. After completion of the reaction, the reaction mixture was cooled to 25 °C, quenched with water (100 mL), and extracted with ethyl acetate (100 mL × 2). The resulting organic phase was washed with saturated aqueous sodium chloride solution (100 mL), dried over anhydrous sodium sulfate, and filtered. The filtrate was concentrated under reduced pressure to obtain a crude product. The crude product was purified by silica gel column chromatography (eluent: petroleum ether/ethyl acetate = 100/1 to 1/1) to give Compound **13-7.**

Step F: Compound **13-7** (1.4 g, 2.88 mmol) was dissolved in anhydrous tetrahydrofuran (20 mL), and triethylamine trihydrofluoride (1.02 g, 6.34 mmol, 1.03 mL) was added. The mixture was stirred at 25 °C for 16 hours. The reaction mixture was concentrated under reduced pressure to give a crude product, which was purified by silica gel column chromatography (eluent: dichloromethane/methanol = 100/1 to 10/1) to give Compound **13-8.**

Step G: Compound **13-8** (0.53 g, 2.18 mmol), 4,4-dimethoxytrityl chloride (886.02 mg, 2.61 mmol), and triethylenediamine (366.66 mg, 3.27 mmol) were dissolved in a dichloromethane solution (10 mL), and the system was subjected to nitrogen displacement three times. The mixed solution was stirred at 25 °C under nitrogen protection for 4 hours. The reaction was quenched with methanol (2 mL) and concentrated under reduced pressure to give a crude product, which was purified by silica gel column chromatography (eluent: petroleum ether/ethyl acetate = 100/1 to 1/1) to give Compound **13-9.**

Step H: Compound **13-9** (0.6 g, 1.1 mmol), Compound **1-9** (497.2 mg, 1.65 mmol) and 4,5-dicyanoimidazole (64.94 mg, 549.87 μmol) were dissolved in dichloromethane solution (2 mL), and the system was subjected to nitrogen displacement three times. The mixed solution was stirred at 25 °C under nitrogen protection for 3 hours. The reaction mixture was quenched by adding 2 mL of saturated sodium bicarbonate solution, diluted with 20 mL of water, and extracted with dichloromethane (30 mL × 2). The combined organic phases were washed with saturated sodium chloride (30 mL × 2), dried over anhydrous sodium sulfate, filtered, and concentrated under reduced pressure to obtain a crude product. The crude product was purified by silica gel column chromatography (eluent: petroleum ether/ethyl acetate = 100/1 to 1/1) to give Compound **dR-M.**

LCMS(ESI) m/z: 746.5 $[M+H]^+$.
$^1$H NMR (400 MHz, DMSO-$d_6$): δ = 8.92 -8.91 (d, *J* = 4.0 Hz, 1H), 7.31 - 7.27 (m, 2H), 7.26 - 7.13 (m, 7H), 6.89 - 6.73 (m, 4H), 6.48 - 6.35 (m, 1H), 4.80 - 4.55 (m, 1H), 4.18 -4.08 (m, 1H), 3.82 (s, 3H), 3.76 - 3.68 (m, 7H), 3.64 - 3.59 (m, 1H), 3.56 - 3.42 (m, 2H), 3.21 - 3.07 (m, 2H), 2.81 - 2.72 (m, 2H), 2.66 - 2.63 (m, 1H), 2.61 -2.52 (m, 1H), 1.14 - 1.09 (m, 9H), 1.03 -0.95 (d, *J* = 6.8 Hz, 3H).

## BIOLOGICAL DETECTION

### Experimental Example 1: Evaluation of Inhibitory Activity of siRNA against LPA mRNA Using psiCHECK-2 Dual-Luciferase Reporter Vector

#### 1. Experimental materials

#### 1.1. Test Compounds

A stock solution was prepared by dissolving in PBS and stored at -20 °C.

#### 1.2. psiCHECK-2 reporter construct

The human LPA gene (Genbank number: NM_005577.4) was cloned into the psiCHECK™-2 vector.

#### 1.3. Cell culture

Huh7 cells were cultured in DMEM (Gibco-11995-065, 500 mL) medium, supplemented with FBS (ExCellBio-FSP500, 57 mL), Penicillin-Streptomycin solution (Hyclone-SV30010, 5.7 mL), GlutaMAX™ (Gibco-35050-061, 5.7 mL) and Non-Essential Amino Acids Solution (Gibco-11140050, 5.7 mL).

#### 1.4. Main reagents

Lipofectamine RNAiMAX (Invitrogen-13778-150); DMEM medium (Gibco-11995-065); Fetal bovine serum (ExCellBio-FSP500); FuGENE-HD (Promega-E2311); Opti-MEM medium (Gibco-31985070); MEM NEAA (100X) (Gibco-11140050); Penicillin-Streptomycin solution (PS) (Hyclone-SV30010); Dual-Glo® Luciferase Assay System (Promega-E2980).

#### 1.5. Main equipment

Multimode Plate Reader (Perkin Elmer-Envision® 2105)

#### 2. Experimental procedure

#### 2.1. Transfection with LPA_psiCHECK (TM)-2 plasmid

On Day 1, a method for transfection of the LPA_PSICHECK (TM)-2 plasmid is briefly described as follows: Huh7 cells were washed with DPBS, followed by digestion with 0.05% trypsin, gently pipetted into single cells in DMEM containing 10% FBS, and counted. Plasmid DNA was transferred into Huh7 cells using Fugene HD. The transfected cells were seeded into 96-well plates at a density of 10,000 cells per well, with 100 μL culture medium per well. The cells were incubated overnight in a 5% $CO_2$ incubator at 37 °C.

### 2.2. Compound transfection and dosing

On Day 2, the test compounds were diluted serially with PBS to 3 concentrations.

RNAiMAX transfection reagent was prepared by blending homogeneously with Opti-MEM at a ratio of RNAiMAX: Opti-MEM=3:47 in a 15 mL centrifuge tube as needed. The compounds were mixed with the RNAiMAX/Opti-MEM (1:1) and incubated for 15 min. The compound-containing RNAiMAX/Opti-MEM mixture was diluted 10-fold with fresh medium and blended homogeneously.

The medium in each well was aspirated, and then 100 μL of the fresh compound-containing medium was added, and then incubated in a 5% $CO_2$, 37 °C incubator.

### 2.3. Detection with Dual-Glo® Dual Luciferase Assay System

Experimental procedures refer to DUAL-GLO® Dual Luciferase Assay System instructions, briefly described as follows: Taking out the cells from the incubator, aspirating and discarding the supernatant, adding 75μL fresh culture medium and 75μL detection reagent, shaking for 10 minutes in the dark, and after the cells had been fully lysed, transfering100μL sample to an opaque whiteboard for detecting the Firefly luminescence signal; adding 50 μL Dual-Glo®Stop & Glo® test reagent to each well and detecting the Renilla luciferase signal after shaking for 10 minutes in the dark. The ratio of the reporter signal to the internal control reporter signal was calculated for each well.

### 2.4. Analysis

Measurement ratio = (Renilla luminescence - Background Renilla luminescence) / (Firefly luminescence - Background Firefly luminescence)

$$\% \text{ Inhibition} = (1 - \text{Sample ratio} / \text{RNAiMAX control ratio}) \times 100\%$$

### 3. The experimental results shown in Table 4

**Table 4: Inhibitory Activity against LPA mRNA in the psiCHECK-2 Dual-Luciferase Reporter Vector**

| Double-stranded siRNA conjugates | $EC_{50}$ (nM) | Double-stranded siRNA conjugates | $EC_{50}$ (nM) |
|---|---|---|---|
| Z1 | 0.12 | Z2 | 0.48 |
| Z6 | 0.42 | Z7 | 2.41 |
| Z8 | 0.81 | Z9 | 1.77 |
| Z10 | 2.11 | Z11 | 0.56 |
| Z12 | 0.32 | Z13 | 0.42 |
| Z14 | 1.59 | Z15 | 0.23 |
| Z17 | 1.50 | Z18 | 0.25 |
| Z20 | 0.51 | Z21 | 0.44 |
| Z22 | 0.28 | Z23 | 0.12 |
| Z25 | 0.68 | Z26 | 2.83 |
| Z27 | 0.96 | Z32 | 0.21 |
| Z34 | 3.05 | Z35 | 0.14 |
| Z36 | 0.23 | Z37 | 0.23 |
| Z40 | 0.07 | Z41 | 0.09 |

(continued)

| Double-stranded siRNA conjugates | $EC_{50}$ (nM) | Double-stranded siRNA conjugates | $EC_{50}$ (nM) |
|---|---|---|---|
| Z42 | 0.31 | Z43 | 0.09 |
| Z46 | 0.31 | Z47 | 0.20 |
| Z48 | 0.09 | Z49 | 0.63 |
| Z50 | 0.41 | Z51 | 0.31 |
| Z52 | 0.14 | | |

**4. Experimental conclusions**

The double-stranded siRNA conjugates of the present disclosure can effectively inhibit human LPA mRNA.

**Experimental Example 2: Evaluation of *In Vivo* Efficacy of Compounds Using an LPA-HDI Mouse Model**

**1. Experimental Principles**

The knockdown effect of the test double-stranded siRNA conjugate on the target gene LPA upon entry into the mouse is evaluated by rapid high-pressure injection of the physiological saline containing recombinant plasmid pcDNA-LPA carrying the target gene via the tail vein.

**2. Experimental materials**

pcDNA-LPA plasmid, BALB/c mouse, PBS (phosphate buffer), and conjugates of the present invention.

**3. Experimental method**

Six-week-old female BALB/c mice were ordered and acclimated/quarantined for three days upon arrival in the animal room.

On Day 0, the mice were randomly grouped based on body weight data. After grouping, all mice were given a single administration via subcutaneous injection, with a dose volume of 10 mL/kg. Mice in Group 1 received PBS; mice in the other groups received the conjugates (dissolved in PBS).

On Day 13 post-administration, all mice were injected with normal saline containing the pcDNA-LPA plasmid via the tail vein within 5 seconds. The injection volume (mL) = mouse body weight (g) × 8%. The amount of plasmid injected per mouse was 10 $\mu$g.

On Day 14 post-administration, all groups of mice were euthanized by $CO_2$ inhalation, and 2 liver samples were collected from each mouse. Liver samples were treated overnight with RNAlater at 4 °C, then RNAlater was removed, and stored at -80 °C for detection of LPA gene expression levels.

**4. The experimental results are shown in Table 5**

Table 5 Experiment Results of *In Vivo* Efficacy of Conjugates of the Present Disclosure (4 mice per group)

| Double-stranded siRNA conjugates No. | Dose administered (mg/kg) | % Downregulation of LPA mRNA | Standard deviation (SD) |
|---|---|---|---|
| Z27 | 3 | 92.2 | 0.01 |
| Z32 | 3 | 86.4 | 0.02 |
| Z33 | 3 | 79.2 | 0.02 |
| Z34 | 3 | 78.1 | 0.05 |
| Z35 | 3 | 92.3 | 0.02 |
| Z40 | 3 | 59.0 | 0.05 |

(continued)

| Double-stranded siRNA conjugates No. | Dose administered (mg/kg) | % Downregulation of LPA mRNA | Standard deviation (SD) |
|---|---|---|---|
| Z42 | 3 | 78.0 | 0.07 |
| Z43 | 3 | 84.5 | 0.01 |
| Z46 | 3 | 89.0 | 0.02 |
| Z47 | 3 | 64.0 | 0.03 |
| Z48 | 3 | 64.0 | 0.05 |
| Note: the "% Downregulation of LPA mRNA" refers to the percentage downregulation in LPA mRNA in the liver of mice in the treatment group relative to the PBS blank group. | | | |

**5. Experimental conclusions**

The double-stranded siRNA conjugates of the present disclosure can effectively inhibit LPA mRNA in the LPA-HDI mouse model.

**Experimental Example 3: Stability of the Double-Stranded siRNAs or Conjugates Thereof**

**1. Experimental method**

The Tm value was determined by recording the change in UV absorption at 260 nm of DNA/RNA in phosphate buffer solution as the temperature increased.

**2. Experimental parameters**

The instrument parameters used in the experiment are shown in Table 6.

Table 6 Melting temperature (Tm) test instrument parameters

| Instrument | Cary300 |
|---|---|
| Wavelength (nm) | 260 |
| Data acquisition interval (min) | 0.5 |
| Heating Rate (°C/min) | 0.5 |
| Starting Temperature (°C) | 20.0 |
| Starting Temperature (°C) | 95.0 |
| Hold time (min) | 1.0 |
| Starting Temperature (°C) | 20.0 |

**3. Experimental results**

The melting temperature (Tm) was calculated from the first derivative of the smoothed heating curve, and the reported value was the average of at least two independent measurements. Results are shown in Table 7.

Table 7 Melting temperatures (Tm) of conjugates of the present disclosure

| Double-stranded siRNA conjugates No. | Melting temperature Tm (°C) |
|---|---|
| Z42 | 67.75 |
| Z46 | 76.75 |
| Z54 | 69.00 |
| Z56 | 77.25 |

## 4. Experimental conclusions

The double-stranded siRNA conjugates of the present disclosure have high melting temperatures and good stability.

## Experimental Example 4: Off-Target Assessment of Double-Stranded siRNA Conjugates

### 1 Experimental material

1.1 Double-stranded siRNA conjugates
1.2 PHH cells, purchased from Research Institute for Liver Diseases(Shanghai)Co. Ltd.
1.3 Main reagents: InvitroGRO CP Medium (Cat. No.: BIOIVT-S03316), Fetal Bovine Serum (Cat. No.: ExCellBio-FSP500), Penicillin-Streptomycin (Cat. No.: Hyclone-SV30010), TRLzol Reagent (Cat. No.: Ambion-15596018).

### 2 Experimental method

2.1 Cell resuscitation and compound treatment

The cryopreserved PHH was taken from the liquid nitrogen and thawed in a water bath at 37 °C. The fully thawed PHH was transferred to a 10% FBS InvitrogRO CP medium and blended well, and counted using a counter. The cell density was adjusted to $6 \times 10^5$ cells/mL.

siRNAs were diluted with RNase-free PBS to 10 times the final concentration (for example, diluted to 1000 nM if the test concentration is 100 nM). The diluted siRNAs were then added to a collagen-coated 12-well plate at 100 μL/well, with 1 concentration point, 3 replicated wells, and blank PBS as the negative control group. PHH cells at a density of $6 \times 10^5$ cells/mL were added to the collagen-coated 12-well plate at 900 μL/well. The final concentrations of the test compounds were 20 nM, 100 nM, and 500 nM. The cell plates were incubated in a 5% $CO_2$, 37 ° C incubator for 48 hours. After 48 hours, the medium was removed, 600μL of Trizol was added per well to lyse the cells, and sent to Beijing Novogene Co., Ltd. for RNA-sequencing.

2.2 Library construction and quality inspection

NEB Standard Library Preparation: Fragmented mRNAs were used as a template and random oligonucleotides as primers. The first strand of cDNA was synthesized in an M-MuLV reverse transcriptase system. The RNA strand was then degraded with RNaseH, and the second strand of cDNA was synthesized using a DNA polymerase I system with dNTPs as raw materials. The purified double-stranded cDNA underwent end repair, A-tailing, and adapter ligation for sequencing. The cDNA of approximately 250-300 bp was screened out by AMPure XP beads, PCR amplified, and the PCR product was purified again by AMPure XP beads to finally obtain the library. The kit used for library construction was NEBNext® Ultra ™ RNA Library Prep Kit for Illumina.

After the library construction, quantification was primarily performed using a Qubit2.0 Fluorometer, and the library was diluted to 1.5 ng/μL. The insert size of the library was then detected using an Agilent 2100 bioanalyzer. If the insert size meets expectations, the effective concentration of the library was accurately quantified by qRT-PCR (an effective concentration of the library higher than 2nM) to ensure the quality of the library.

2.3 Performing sequencing on a sequencer

After passing quality control of the library, different libraries were pooled according to the effective concentration and target output data volume for Illumina sequencing. The basic principle of sequencing is Sequencing by Synthesis. Four fluorescently labeled dNTPs, DNA polymerase, and adapter primers were added to the sequencing flow cell for amplification. As each sequencing cluster extends the complementary strand, the incorporation of each fluorescently labeled dNTP can release the corresponding fluorescence. The sequencer captures the fluorescence signal and converts the optical signals into sequencing peaks through computer software, thereby obtaining the sequence information of the test fragments.

3 Sequencing Data Analysis

Experimental results were obtained through software analysis

4 Experimental results:

Differential gene analysis takes DESeq2 padj ≤ 0.05 |$log_2$FoldChange| ≥ 1.0 as the threshold. The statistical results are shown in Table 8.

Table 8. Differential gene statistics

| Compared Group Name | Total differential genes | Upregulated differential genes | Maximum up-regulation folds | Downregulated differential genes | Maximum downregulation folds |
|---|---|---|---|---|---|
| Z42 vs PBS | 4 | 4 | 1.9 | 0 | NA |
| Z46 vs PBS | 0 | 0 | NA | 0 | NA |

5 Experimental conclusions

The double-stranded siRNA conjugates of the present disclosure have a low risk of off-target effects.

**Experimental Example 5: Cytotoxicity Experiment of Double-Stranded siRNA Conjugates**

**1 Experimental materials**

1.1 Double-stranded siRNA conjugates
1.2 PHH cells, purchased from Research Institute for Liver Diseases (Shanghai)Co. Ltd. HEK293 cells, purchased from ATCC (Cat. No.: CRL-1573)
1.3 Main reagents: InvitroGRO CP Medium (Cat. No.: BIOIVT-S03316), EMEM Medium (Cat. No.: ATCC-30-2003), Fetal Bovine Serum (Cat. No.: ExCellBio-FSP500), Penicillin-Streptomycin (Cat. No.: Hyclone-SV30010), TRLzol Reagent (Cat. No.: Ambion-15596018), CellTriter-Glo ® Luminescent Cell Viability Assay (Cat. No.: Promega-G7573).

**2 Experimental method**

2.1 Compound treatment

PHH: The test siRNA was diluted with RNase-free PBS to 10 times the final concentration (diluted to 1000 nM if a final concentration of the test is 100 nM). The diluted siRNAs were added to a collagen-coated 96-well cell plate at 10 μL/well, with 9 concentration points in total, 2 replicated wells, and RNase-free PBS was added as a control.

HEK293 cells: siRNA was transfected using the reverse transfection, that is, the transfection reagent and siRNA mixture were first added to a culture plate and co-incubated before adding cells for culture. The 20 μM stock solution was diluted 5-fold serially with RNase-free PBS to 9 concentrations, so that the final concentrations after being added to the cell culture system were 500 nM, 100 nM, 20 nM, 4 nM, 0.8 nM, 0.16 nM, 0.032 nM, 0.064 nM, and 0.00128 nM. At the same time, the PBS well was set as a control. A mixture of the RNAiMAX transfection reagent and Opti-MEM (RNAiMAX transfection reagent: Opti-MEM = 1.5: 48.5) was prepared as needed, and incubated at room temperature for 15 minutes; then 10 μL of the diluted compounds were added to 10 μL of RNAiMAX: Opti-MEM culture, blended homogeneously, and incubated for 15 minutes. 10 μL of mixtures of Opti-MEM: RNAiMax and compounds or PBS were added to the cell culture plate.

2.2 Cell treatment

PHH cells: The cryopreserved cells were thawed in a water bath at 37 °C, then transferred to 10% FBS InvitroGRO CP medium and blended homogeneously. The cells were counted using a counter, and the cell density was adjusted to $6 \times 10^5$ cells/mL. The densityadjusted cells were added to the corresponding 96-well cell plates described above, respectively, at 90 μL/well, with a final volume of 100 μL per well. The cell plates were incubated in a 5% $CO_2$, 37 ° C incubator for 48 hours.

HEK293 cells: HEK293 cells with 80% confluence were digested with trypsin, centrifuged, resuspended, and counted. HEK293 cells were formulated as a suspension of 75000 cells/mL using EMEM complete medium. The

formulated cell suspension was added to a 96-well plate at 90 μL per well with a final volume of 100 μL.

2.3 Luminescence signal detection

The 96-well cell culture plate was taken and allowed to stand at room temperature for 5 minutes to equilibrate. 50 μL of Cell Titer Glo reagent per well was added to a 96-well plate, shaken at 500 rpm for 5 min, and equilibrated at room temperature for 5 min. Readings were performed on a microplate reader using the * USLum-US LUM 96 (cps) program.

2.4 Data processing

1)

Cell Viability: Viability % = (Sample Value - Mean of Blank wells) / (Mean of Control wells - Mean of Blank wells) × 100%

2) The Viability % and the corresponding siRNA concentration were substituted into the curve fit by the GraphPad Prism 8.3 statistical software to give the $CC_{50}$ value, using the calculation formula: Y = Bottom + (Top-Bottom) / (1 +10 ^ ((Log$IC_{50}$-X) * HillSlope)), where the $IC_{50}$ is equivalent to $CC_{50}$ in this experiment, and the absolute $CC_{50}$ refers to the siRNA concentration corresponding to when the cell Viability rate is 50%.
3 Experimental results:

Table 9. Cytotoxicity of compounds of the present disclosure on PHH and HEK293 cells

| Cells / Compounds | PHH cells (Max concentration: 10000 nM) | | HEK293 cells (Max concentration: 500 nM) | |
|---|---|---|---|---|
| | $CC_{50}$ (nM) | Maximum inhibition rate | $CC_{50}$ (nM) | Maximum inhibition rate |
| Z27 | | | > 500 | 11.9% |
| Z42 | >10000 | 0.4% | > 500 | 7.7% |
| Z43 | | | > 500 | 2.0% |
| Z46 | >10000 | 11.8% | > 500 | 2.3% |

4 Experimental conclusions

The double-stranded siRNA conjugates of the present disclosure have a low risk of cytotoxicity.

**Experimental Example 6: Immunogenicity Test of Compounds of the Present Disclosure in hPBMCs**

1 Experimental material

1.1 Double-stranded siRNA conjugates
1.2 hPBMCs, purchased from Shanghai Sailybio Medical Co., LTD.
1.3 Main reagents: Penicillin-Streptomycin (Cat. No.: 15140122), RNase-free water (Cat. No.: AM9938), IL-6 ELISA kit (Cat. No.: 555220), TNF-α ELISA kit (Cat. No.: 555212), LPS (Cat. No.: L2630), etc.

2 Experimental method

2.1 Compound Preparation: Compounds were prepared as stock solutions with enzyme-free water. Compound weight = powder weight * purity. The concentration of prepared solutions was 900 μg/mL, followed by three-fold serial

dilution with enzyme-free water. Each concentration was diluted 10-fold with 1640 medium, resulting in a final enzyme-free water content of 5.0%.

2.2 Experimental procedure:

2.2.1 PBMC cells were resuscitated one day in advance and incubated overnight in 1640 complete medium.

2.2.2 On the day of the experiment, PBMC cells were centrifuged at 300 × g for 8 minutes, and the cell density was adjusted to $1.6 \times 10^6$ mL with 1640 complete medium. The cells were then added to a 96-well plate, 125 μL per well, and incubated in a 37 °C, 5% $CO_2$ incubator for 15 minutes.

2.2.3 The diluted test compounds were added to the 96-well plate according to the plate map, all in duplicate, 125 μL for each well; the positive control group and the blank group were added with an equal volume of 1640 complete culture medium containing the same amount of enzyme-free water.

2.2.4 After gently shaking, the well plates were incubated in an incubator at 37 °C for 18-20 h. The supernatant was collected by centrifugation, and secretions of TNF-α and IL-6 were detected by ELISA.

2.2.5 Data were analyzed using GraphPad Prism 8 software.

3 Experimental results

Table 10. IL-6 and TNF-α levels in the cell supernatant of donor 1 hPBMCs treated with different doses of test compounds

| siRNA conjugates | IL-6 (pg/mL) | | | TNF alpha (pg/mL) | | |
|---|---|---|---|---|---|---|
| | 45 μg/mL | 15 μg/mL | 5 μg/mL | 45 μg/mL | 15 μg/mL | 5 μg/mL |
| Enzyme-free water | 1268.6 | | | 32.3 | | |
| LPS (5 μg/mL) | 7289.2 | | | 471.3 | | |
| LPS (1.67 μg/mL) | 7317.3 | | | 474.4 | | |
| Z38 | 1268.3 | 1257.1 | 1281.4 | 34.4 | 34.8 | 33.5 |
| Z39 | 1297.0 | 1304.8 | 1333.6 | 33.3 | 32.6 | 31.4 |
| Z40 | 1343.4 | 1266.6 | 1333.8 | 32.7 | 32.3 | 35.9 |
| Z41 | 1537.6 | 2424.8 | 1362.7 | 44.7 | 94.0 | 37.3 |
| Z42 | 1457.0 | 1406.8 | 1298.3 | 39.4 | 34.8 | 35.8 |
| Z43 | 1314.4 | 1339.1 | 1270.9 | 32.2 | 36.9 | 35.4 |
| Z44 | 1354.7 | 1331.0 | 1326.7 | 35.8 | 35.2 | 34.6 |
| Z45 | 1321.4 | 1321.5 | 1327.5 | 34.9 | 35.1 | 35.3 |

Table 11. IL-6 and TNF-α levels in the cell supernatant of donor 2 hPBMCs treated with different doses of test compounds

| siRNA conjugates | IL-6 (pg/mL) | | | TNF alpha (pg/mL) | | |
|---|---|---|---|---|---|---|
| | 45 μg/mL | 15 μg/mL | 5 μg/mL | 45 μg/mL | 15 μg/mL | 5 μg/mL |
| Enzyme-free water | 0.0 | | | 2.7 | | |
| LPS (5 μg/mL) | 8510.6 | | | 2110.7 | | |
| LPS (1.67 μg/mL) | 7313.0 | | | 2065.5 | | |
| Z46 | 9.3 | 88.8 | 15.0 | 10.2 | 22.8 | 8.1 |
| Z47 | 16.7 | 0.0 | 49.9 | 9.5 | 3.4 | 15.6 |
| Z48 | 1.1 | 49.5 | 32.0 | 5.9 | 20.4 | 15.3 |

4 Experimental conclusions

The compounds of the present disclosure have a low risk of immunogenicity.

**Experimental Example 7: Assessment of in vivo activity of compounds of the disclosure using cynomolgus macaques**

1 Experimental material

1.1 Double-stranded siRNA conjugates of the present disclosure
1.2 Cynomolgus macaques
1.3 Experimental reagents

PBS as vehicle, Lp (a) ELISA Kit, Cat. No.: Mercodia-10-1106-01

2 Experimental method

2.1 Cynomolgus macaques (body weight: 3.5 to 5 kg) were recruited, with 2 animals per group. Each animal was injected subcutaneously with 2 mg/kg test article (double-stranded siRNA conjugates dissolved in PBS). After overnight fasting, blood collection was performed on Days -15 (pre-dose), -7 (pre-dose), 0 (pre-dose), and Days 7, 14, 21, and 28 post-dose. The collected blood samples were allowed to clot at room temperature for at least 30 minutes and then centrifuged at 3500 rpm for 10 minutes at 4 °C. The collected serum (approximately 0.5 mL) was used for the ELISA assay.

3 Experimental results

Table 12. Effect of compounds of the present disclosure on Lp(a) reduction in cynomolgus macaques

| Compounds | Animal No. | % Lp(a) remaining | | | | |
|---|---|---|---|---|---|---|
| | | Day 0 | Day 7 | Day 14 | Day 21 | Day 28 |
| Z42 | 1 | 100% | 21.3% | 21.0% | 17.5% | 15.9% |
| | 2 | 100% | 18.6% | 11.4% | 11.0% | 7.3% |
| Z46 | 3 | 100% | 15.6% | 11.4% | 7.3% | 6.5% |
| | 4 | 100% | 23.4% | 19.0% | 21.7% | 15.7% |

4 Experimental conclusions

The compounds of the present disclosure have a significant and long-lasting effect on reducing the Lp(a) level in cynomolgus macaques.

## Claims

1. A double-stranded siRNA that inhibits expression of LPA or a pharmaceutically acceptable salt thereof, comprising a sense strand and an antisense strand capable of forming a double-stranded region; wherein the sense strand comprises any one of the sequences set forth in SEQ ID NOs: 1-18, and the antisense strand comprises any one of the sequences set forth in SEQ ID NOs: 19-26, wherein each nucleotide on the sense strand and the antisense strand is optionally a modified nucleotide.

2. The double-stranded siRNA or the pharmaceutically acceptable salt thereof according to claim 1, wherein at least one nucleotide on the sense strand and the antisense strand is a modified nucleotide.

3. The double-stranded siRNA or the pharmaceutically acceptable salt thereof according to claim 1, wherein 0, 1, 2, 3 or 4 nucleotides on the sense strand and the antisense strand are unmodified nucleotides and remaining nucleotides are modified nucleotides.

4. The double-stranded siRNA or the pharmaceutically acceptable salt thereof according to claim 1, wherein the

modified nucleotide is selected from the group consisting of a 2'-OMe-modified nucleotide, a 2'-F-modified nucleotide, and a 2'-deoxynucleotide.

5. The double-stranded siRNA or the pharmaceutically acceptable salt thereof according to claim 1, wherein the 5' end of the antisense strand optionally comprises one nucleotide selected from the group consisting of mDVPU, B29, B30, B33, B34 and B36, wherein,

the mDVPU is

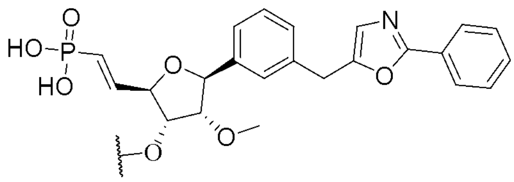

;

the B29 is

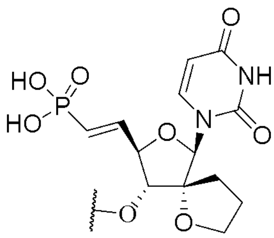

;

the B30 is

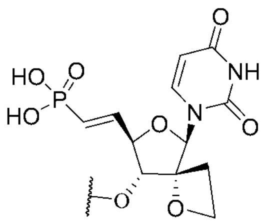

;

the B33 is

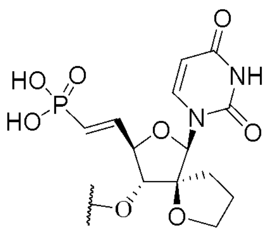

;

the B34 is

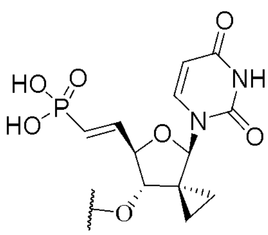

;

and

the B36 is

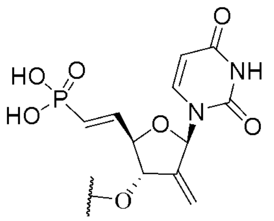

.

6. The double-stranded siRNA or the pharmaceutically acceptable salt thereof according to claim 1, wherein the 5' end of the sense strand optionally comprises one nucleotide selected from invAb, wherein the invAb is

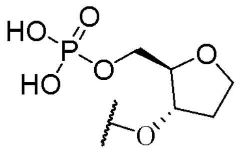

.

7. The double-stranded siRNA or the pharmaceutically acceptable salt thereof according to claim 1, comprising any one of the duplexes shown in S1-S37, S39-S44, or S46-S64.

8. A double-stranded siRNA conjugate that inhibits expression of LPA or a pharmaceutically acceptable salt thereof, wherein the double-stranded siRNA conjugate is formed by conjugating the double-stranded siRNA or the pharmaceutically acceptable salt thereof of any one of claims 1-7 to a ligand.

9. The double-stranded siRNA conjugate or the pharmaceutically acceptable salt thereof according to claim 8, wherein the ligand is one or more GalNAc derivatives attached by using bivalent, trivalent, or tetravalent branchedlinkages.

10. The double-stranded siRNA conjugate or the pharmaceutically acceptable salt thereof according to claim 9, wherein the ligand is selected from the group consisting of D01, D08, D10, D11, D18, D21, and L96, which respectively have the following structural formulas:

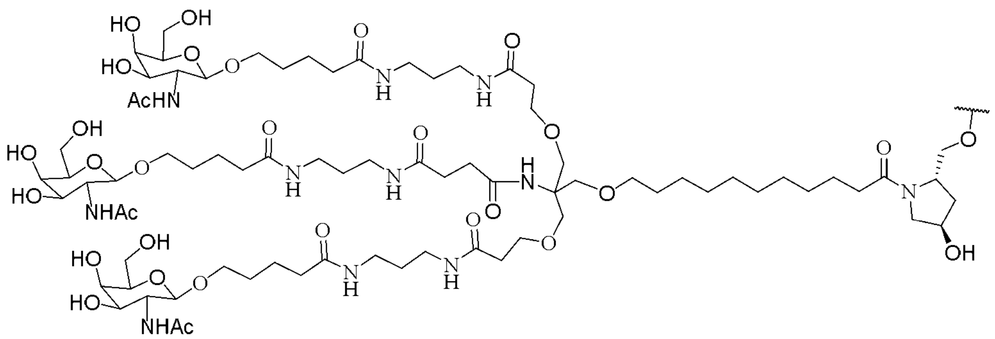

D01

,

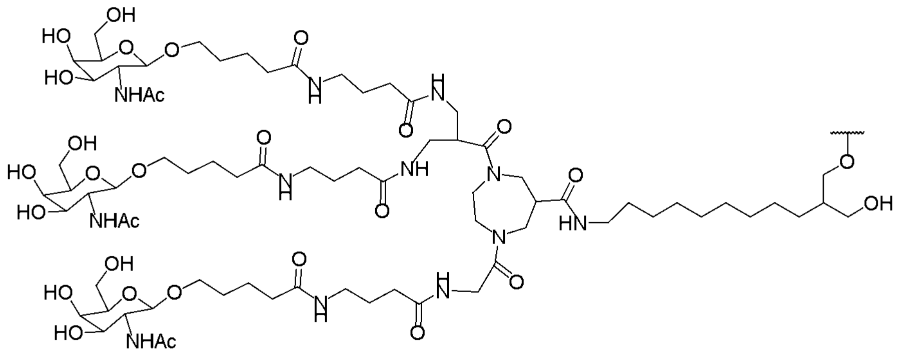

D08

,

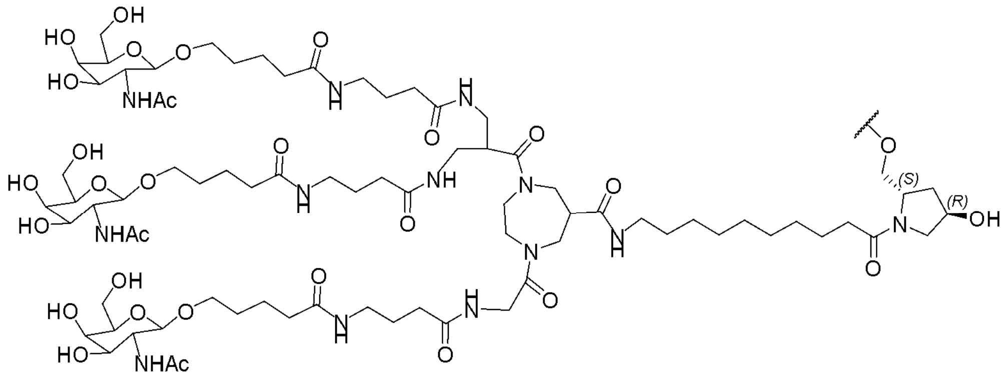

D10

,

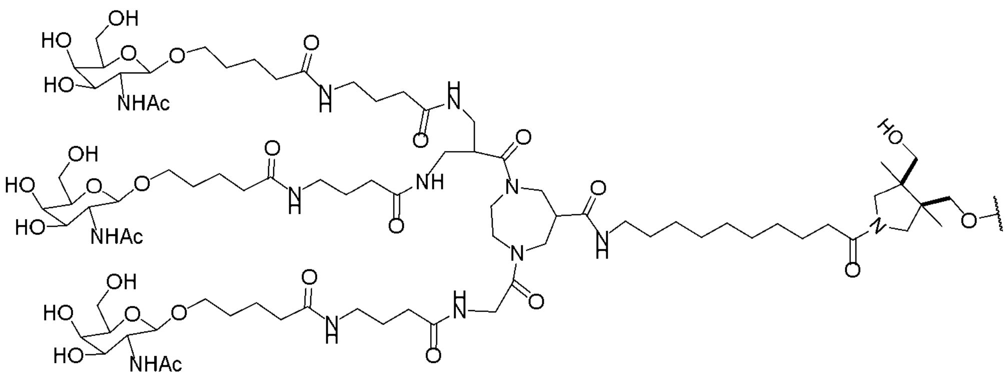

D11

,

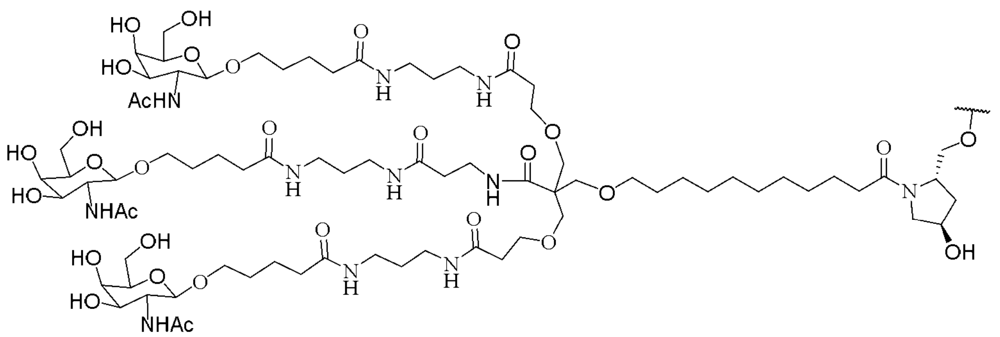

D18

,

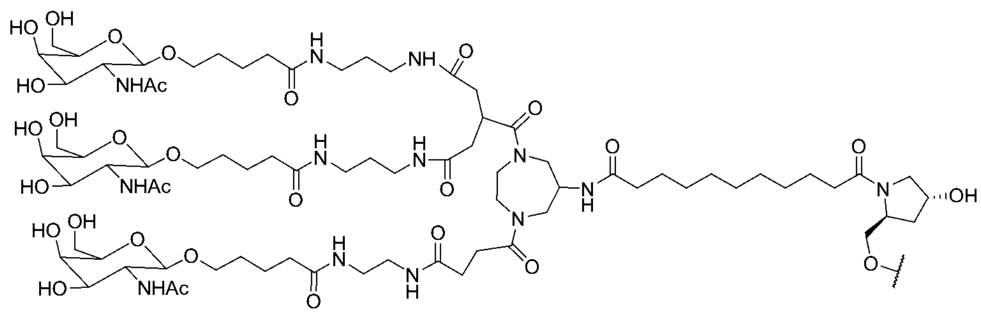

D21

,

and

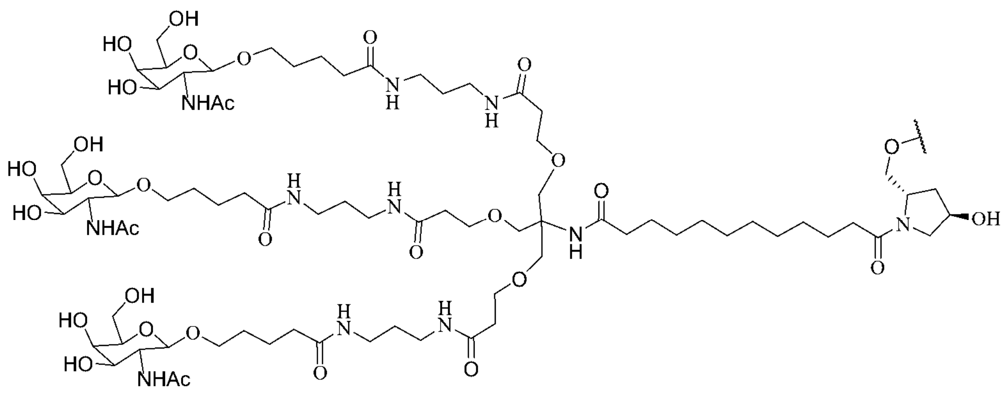

L96

.

11. The double-stranded siRNA conjugate or the pharmaceutically acceptable salt thereof according to claim 8, wherein the ligand is conjugated to the 3' end of the sense strand.

12. The double-stranded siRNA conjugate or the pharmaceutically acceptable salt thereof according to any one of claims 8-10, wherein the ligand is conjugated to the double-stranded siRNA via a phosphodiester linkage or a phosphorothioate linkage.

13. The double-stranded siRNA conjugate or the pharmaceutically acceptable salt thereof according to claim 8, wherein the double-stranded siRNA conjugate is selected from any one of the conjugates shown in Z1-Z65.

14. The double-stranded siRNA conjugate or the pharmaceutically acceptable salt thereof according to claim 8, wherein the double-stranded siRNA conjugate is shown in Formula I:

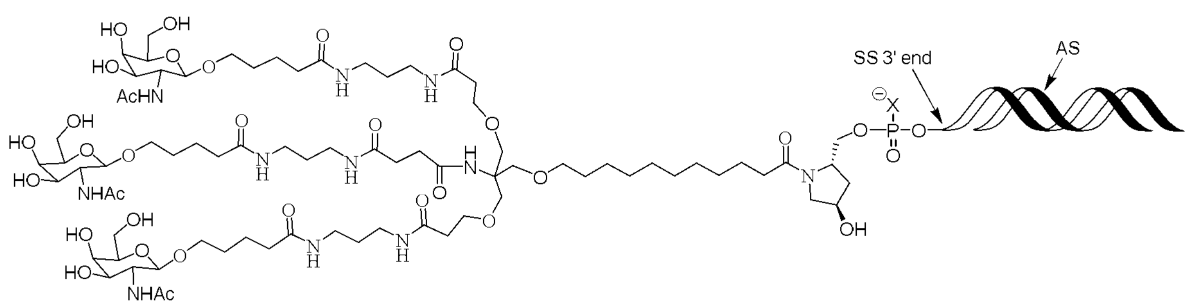


(formula I),

wherein X is O; SS is the sense strand and AS is the antisense strand; the sense strand and the antisense strand are selected from any one of the combinations shown in (1) to (13):

(1) the sense strand set forth in SEQ ID NO: 49 and the antisense strand set forth in SEQ ID NO: 80;
(2) the sense strand set forth in SEQ ID NO: 49 and the antisense strand set forth in SEQ ID NO: 81;
(3) the sense strand set forth in SEQ ID NO: 43 and the antisense strand set forth in SEQ ID NO: 66;
(4) the sense strand set forth in SEQ ID NO: 51 and the antisense strand set forth in SEQ ID NO: 86;
(5) the sense strand set forth in SEQ ID NO: 53 and the antisense strand set forth in SEQ ID NO: 88;
(6) the sense strand set forth in SEQ ID NO: 43 and the antisense strand set forth in SEQ ID NO: 92;
(7) the sense strand set forth in SEQ ID NO: 43 and the antisense strand set forth in SEQ ID NO: 94;
(8) the sense strand set forth in SEQ ID NO: 43 and the antisense strand set forth in SEQ ID NO: 95;
(9) the sense strand set forth in SEQ ID NO: 43 and the antisense strand set forth in SEQ ID NO: 96;
(10) the sense strand set forth in SEQ ID NO: 49 and the antisense strand set forth in SEQ ID NO: 94;
(11) the sense strand set forth in SEQ ID NO: 49 and the antisense strand set forth in SEQ ID NO: 95;
(12) the sense strand set forth in SEQ ID NO: 49 and the antisense strand set forth in SEQ ID NO: 96;
(13) the sense strand set forth in SEQ ID NO: 55 and the antisense strand set forth in SEQ ID NO: 95.

**15.** Use of the double-stranded siRNA or the pharmaceutically acceptable salt thereof according to any one of claims 1 to 7, or the double-stranded siRNA conjugate or the pharmaceutically acceptable salt thereof according to any one of claims 8 to 14 in the manufacture of a medicament for treating a cardiovascular disease.

**16.** The use according to claim 15, wherein the cardiovascular disease is selected from the group consisting of: Burger's disease, peripheral artery disease, coronary artery disease, metabolic syndrome, aortic valve stenosis, aortic valve regurgitation, aortic dissection, retinal artery occlusion, mesenteric ischemia, superior mesenteric artery occlusion, renal artery stenosis, stable/unstable angina pectoris, acute coronary syndrome, heterozygous or homozygous familial hypercholesterolemia, hyperapolipoprotein beta lipoproteinemia, cerebrovascular atherosclerosis, and venous thrombosis.

**INTERNATIONAL SEARCH REPORT**

International application No.
**PCT/CN2024/094986**

**A. CLASSIFICATION OF SUBJECT MATTER**

C12N15/113(2010.01)i； A61K31/713(2006.01)i； A61P3/06(2006.01)i； A61P9/00(2006.01)i； A61P9/10(2006.01)i

According to International Patent Classification (IPC) or to both national classification and IPC

**B. FIELDS SEARCHED**

Minimum documentation searched (classification system followed by classification symbols)

IPC： C12N A61K A61P

Documentation searched other than minimum documentation to the extent that such documents are included in the fields searched

Electronic data base consulted during the international search (name of data base and, where practicable, search terms used)

CNTXT; VCN; VEN; ENTXT; ENTXTC; OETXT; CNKI; 万方, WANFANG; 读秀学术, Duxiu Scholar; 中国专利生物序列检索系统, China Patent Biological Sequence Search System; NCBI; STNEXT; ISI Web of Science; Springer: 明德新药, MEDSHINE, 双链, siRNA, LPA, 核苷酸, 正义链, 反义链, 修饰, 改性, 改造, 缀合物, 配体, 基于序列1, 19的检索, search based on sequences 1 and 19, double stranded, nucleotide, sense strand, antisense strand, modification, conjugate, ligand

**C. DOCUMENTS CONSIDERED TO BE RELEVANT**

| Category* | Citation of document, with indication, where appropriate, of the relevant passages | Relevant to claim No. |
|---|---|---|
| A | WO 2022098841 A1 (AMGEN INC.) 12 May 2022 (2022-05-12)<br>description, paragraphs [0003], [0103] and [0106] | 1-16 (in part) |
| A | CN 114703184 A (KYLONOVA (XIAMEN) BIOPHARMA CO., LTD.) 05 July 2022 (2022-07-05)<br>claims 1-10 | 1-16 (in part) |
| A | CN 115851723 A (KYLONOVA (XIAMEN) BIOPHARMA CO., LTD.) 28 March 2023 (2023-03-28)<br>claims 1-12 | 1-16 (in part) |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 August 2024** | **21 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/094986**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/094986**

**Box No. III Observations where unity of invention is lacking (Continuation of item 3 of first sheet)**

This International Searching Authority found multiple inventions in this international application, as follows:

Inventions 1-5: the solutions of claims 1-16 (in part) involving sense strands respectively containing SEQ ID NOs: 1, 2, 3, 4 and 5, and antisense strands containing SEQ ID NO: 19.

Inventions 6-8: the solutions of claims 1-16 (in part) involving sense strands containing SEQ ID NO: 1, and antisense strands respectively containing SEQ ID NOs: 20, 21 and 21.

Inventions 9 and 10: the solutions of claims 1-16 (in part) involving sense strands respectively containing SEQ ID NOs: 6 and 7, and antisense strands containing SEQ ID NO: 19.

Inventions 11-16: the solutions of claims 1-16 (in part) involving sense strands containing SEQ ID NOs: 7, 8, 9, 10, 11 and 12, and antisense strands respectively containing SEQ ID NOs: 21, 21, 21, 22, 22 and 22.

Inventions 17-22: the solutions of claims 1-16 (in part) involving sense strands respectively containing SEQ ID NOs: 13, 14, 15, 16, 17 and 18, and antisense strands respectively containing SEQ ID NOs: 22, 23, 24, 25, 25 and 26.

This International Authority considers that the claims comprise at least 22 inventions. The above-mentioned inventions do not have a same or corresponding special technical feature that defines a contribution which the inventions make over the prior art, do not have a technical relationship therebetween, do not belong to a single general inventive concept, and thus do not comply with the requirement of unity of invention, and do not comply with PCT Rule 13.1.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-16 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/094986**

| Patent document cited in search report | Publication date (day/month/year) | Patent family member(s) | Publication date (day/month/year) |
|---|---|---|---|
| WO 2022098841 A1 | 12 May 2022 | AU 2021373780 A1 | 08 June 2023 |
| | | JP 2023549115 A | 22 November 2023 |
| | | CA 3199678 A1 | 12 May 2022 |
| | | US 2023405040 A1 | 21 December 2023 |
| | | KR 20230104200 A | 07 July 2023 |
| | | EP 4240855 A1 | 13 September 2023 |
| | | MX 2023005198 A | 16 May 2023 |
| | | IL 302330 A | 01 June 2023 |
| | | TW 202233837 A | 01 September 2022 |
| | | AU 2021373780 A1 | 08 June 2023 |
| | | JP 2023549115 A | 22 November 2023 |
| | | CA 3199678 A1 | 12 May 2022 |
| | | US 2023405040 A1 | 21 December 2023 |
| | | KR 20230104200 A | 07 July 2023 |
| | | EP 4240855 A1 | 13 September 2023 |
| | | MX 2023005198 A | 16 May 2023 |
| | | IL 302330 A | 01 June 2023 |
| | | TW 202233837 A | 01 September 2022 |
| CN 114703184 A | 05 July 2022 | HK 40075830 A0 | 03 February 2023 |
| | | WO WO2023169548 A1 | 03 February 2023 |
| | | CN 114703184 B | 03 February 2023 |
| | | HK 40075830 A0 | 03 February 2023 |
| | | WO 2023169548 A1 | 14 September 2023 |
| | | CN 114703184 B | 18 June 2024 |
| CN 115851723 A | 28 March 2023 | HK 40081882 A0 | 02 June 2023 |
| | | CN 115851723 B | 03 October 2023 |
| | | HK 40081882 A1 | 24 November 2023 |
| | | WO 2024088190 A1 | 02 May 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310598735
- CN 202311140396
- CN 202311601195
- CN 202410112975
- CN 202410505495
- CN 202410621293


### Non-patent literature cited in the description


- **KONRAD SCHMIDT et al.** *J. Lipid Res*, 2016, vol. 57, 1339-1359
- **HOPEWELL et al.** *J. Intern. Med*, 2013, vol. 273 (1), 260-8
- **WILSON et al.** *J Clin Lipidol*, 2019, vol. 13 (3), 374-92